# EUROPEAN PATENT SPECIFICATION

(11) **EP 2 787 008 B1**
(45) Date of publication and mention of the grant of the patent: **04.09.2019**
(21) Application number: 14162847.9
(22) Date of filing: 31.03.2014
(51) Int. Cl.: C07K 16/28, C07K 16/42

(54) **Anti-idiotype antibody against anti-C-met antibody**
Anti-Idiotyp-Antikörper gegen Anti-C-Met-Antikörper
Anticorps anti-idiotype contre des anticorps anti-C-met

(30) Priority: 02.04.2013 KR 20130036053
(43) Date of publication of application: 08.10.2014
(73) Proprietor: Samsung Electronics Co., Ltd., Suwon-si Gyeonggi-do 443-742 (KR)
(72) Inventor: Jung, Soo Yeon, Gyeonggi-do (KR); Cho, Mi Young, Seoul (KR); Song, Yun Jeong, Gyeonggi-do (KR); Choi, Han Na, Seoul (KR)
(74) Representative: Grünecker Patent- und Rechtsanwälte PartG mbB

(56) References cited:
- EP-A1- 2 316 484
- WO-A1-2012/030982
- WO-A2-2011/110642
- YOUNG MI OH ET AL: "A new anti-c-met antibody selected by a mechanism-based dual-screening method: Therapeutic potential in cancer", MOLECULES AND CELLS, vol. 34, no. 6, 1 December 2012 (2012-12-01), pages 523-529, XP055128965, ISSN: 1016-8478, DOI: 10.1007/s10059-012-0194-z
- GIOVANNI PACCHIANA ET AL: "Monovalency Unleashes the Full Therapeutic Potential of the DN-30 Anti-Met Antibody", JOURNAL OF BIOLOGICAL CHEMISTRY, AMERICAN SOCIETY FOR BIOCHEMISTRY AND MOLECULAR BIOLOGY, US, vol. 285, no. 46, 12 November 2010 (2010-11-12), pages 36149-36157, XP002621766, ISSN: 0021-9258, DOI: 10.1074/JBC.M110.134031 [retrieved on 2010-09-10]

### Sequence Listing

<110> Samsung Electronics Co. Ltd
<120> Anti-idiotype antibody against anti-c-Met antibody
<130> EP93180FZSEpau
<140> not yet assigned
   <141> herewith
<150> KR 10-2013-0036053
   <151> 2013-04-02
<160> 275
<170> KopatentIn 1.71
<210> 1
   <211> 5
   <212> PRT
   <213> Artificial Sequence
<220>
   <223> heavy chain CDR1 of AbF46
<400> 1
   Asp Tyr Tyr Met Ser
   1 5
<210> 2
   <211> 19
   <212> PRT
   <213> Artificial Sequence
<220>
   <223> heavy chain CDR2 of AbF46
<400> 2
   Phe Ile Arg Asn Lys Ala Asn Gly Tyr Thr Thr Glu Tyr Ser Ala Ser
   1 5 10 15
   Val Lys Gly
<210> 3
   <211> 6
   <212> PRT
   <213> Artificial Sequence
<220>
   <223> heavy chain CDR3 of AbF46
<400> 3
   Asp Asn Trp Phe Ala Tyr
   1 5
<210> 4
   <211> 6
   <212> PRT
   <213> Artificial Sequence
<220>
   <223> heavy chain CDR1 of c-Met antibody
<220>
   <221> MOD_RES
   <222> (1)
   <223> X is Pro or Ser or absent
<220>
   <221> MOD_RES
   <222> (2)
   <223> X is Glu or Asp
<400> 4
   Xaa Xaa Tyr Tyr Met Ser
   1 5
<210> 5
   <211> 8
   <212> PRT
   <213> Artificial Sequence
<220>
   <223> heavy chain CDR2 of c-Met antibody
<220>
   <221> MOD_RES
   <222> (3)
   <223> X is Asn or Lys
<220>
   <221> MOD_RES
   <222> (4)
   <223> X is Ala or Val
<220>
   <221> MOD_RES
   <222> (7)
   <223> X is Asn or Thr
<400> 5
   Arg Asn Xaa Xaa Asn Gly Xaa Thr
   1               5
<210> 6
   <211> 6
   <212> PRT
   <213> Artificial Sequence
<220>
   <223> heavy chain CDR3 of c-Met antibody
<220>
   <221> MOD_RES
   <222> (5)
   <223> X is Ser or Thr
<400> 6
   Asp Asn Trp Leu Xaa Tyr
   1               5
<210> 7
   <211> 17
   <212> PRT
   <213> Artificial Sequence
<220>
   <223> light chain CDR1 of c-Met antibody
<220>
   <221> MOD_RES
   <222> (4)
   <223> X is His, Arg, Gln or Lys
<220>
   <221> MOD_RES
   <222> (12)
   <223> X is His or Gln
<220>
   <221> MOD_RES
   <222> (13)
   <223> X is Lys or Asn
<220>
   <221> MOD_RES
   <222> (9)
   <223> X is Ser or Trp
<400> 7
   Lys Ser Ser Xaa Ser Leu Leu Ala Xaa Gly Asn Xaa Xaa Asn Tyr Leu
   1 5 10 15
   Ala
<210> 8
   <211> 7
   <212> PRT
   <213> Artificial Sequence
<220>
   <223> light chain CDR2 of c-Met antibody
<220>
   <221> MOD_RES
   <222> (2)
   <223> X is Ala or Gly
<220>
   <221> MOD_RES
   <222> (4)
   <223> X is Thr or Lys
<220>
   <221> MOD_RES
   <222> (7)
   <223> X is Ser or Pro
<400> 8
   Trp Xaa Ser Xaa Arg Val Xaa
   1 5
<210> 9
   <211> 9
   <212> PRT
   <213> Artificial Sequence
<220>
   <223> light chain CDR3 of c-Met antibody
<220>
   <221> MOD_RES
   <222> (1)
   <223> X is Gly, Ala or Gln
<220>
   <221> MOD_RES
   <222> (6)
   <223> X is Arg, His, Ser, Ala, Gly or Lys
<220>
   <221> MOD_RES
   <222> (8)
   <223> X is Leu, Tyr, Phe or Met
<400> 9
   Xaa Gln Ser Tyr Ser Xaa Pro Xaa Thr
    1               5
<210> 10
   <211> 17
   <212> PRT
   <213> Artificial Sequence
<220>
   <223> light chain CDR1 of AbF46
<400> 10
   Lys Ser Ser Gln Ser Leu Leu Ala Ser Gly Asn Gln Asn Asn Tyr Leu
    1               5                  10                  15
   Ala
<210> 11
   <211> 7
   <212> PRT
   <213> Artificial Sequence
<220>
   <223> light chain CDR2 of AbF46
<400> 11
   Trp Ala Ser Thr Arg Val Ser
    1               5
<210> 12
   <211> 9
   <212> PRT
   <213> Artificial Sequence
<220>
   <223> light chain CDR3 of AbF46
<400> 12
   Gln Gln Ser Tyr Ser Ala Pro Leu Thr
   1 5
<210> 13
   <211> 9
   <212> PRT
   <213> Artificial Sequence
<220>
   <223> CDR-L3 derived from L3-1 clone
<400> 13
   Gln Gln Ser Tyr Ser Arg Pro Tyr Thr
   1 5
<210> 14
   <211> 9
   <212> PRT
   <213> Artificial Sequence
<220>
   <223> CDR-L3 derived from L3-2 clone
<400> 14
   Gly Gln Ser Tyr Ser Arg Pro Leu Thr
   1 5
<210> 15
   <211> 9
   <212> PRT
   <213> Artificial Sequence
<220>
   <223> CDR-L3 derived from L3-3 clone
<400> 15
   Ala Gln Ser Tyr Ser His Pro Phe Ser
   1 5
<210> 16
   <211> 9
   <212> PRT
   <213> Artificial Sequence
<220>
   <223> CDR-L3 derived from L3-5 clone
<400> 16
   Gln Gln Ser Tyr Ser Arg Pro Phe Thr
   1 5
<210> 17
   <211> 117
   <212> PRT
   <213> Artificial Sequence
<220>
   <223> heavy chain variable region of anti c-Met humanized antibody(huAbF46-H4)
<400> 17
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Thr Asp Tyr
               20                  25                  30
   Tyr Met Ser Trp Val Arg Gln Ala Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser
           115
<210> 18
   <211> 114
   <212> PRT
   <213> Artificial Sequence
<220>
   <223> light chain variable region of anti c-Met humanized antibody(huAbF46-H4)
<400> 18
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
   65                  70                  75                  80
   Ile Ser Ser Leu Gln Pro Glu Asp Phe Ala Thr Tyr Tyr Cys Gln Gln
                   85                  90                  95
   Ser Tyr Ser Arg Pro Tyr Thr Phe Gly Gln Gly Thr Lys Val Glu Ile
               100                 105                 110
   Lys Arg
<210> 19
   <211> 114
   <212> PRT
   <213> Artificial Sequence
<220>
   <223> light chain variable region of anti c-Met humanized antibody(huAbF46-H4)
<400> 19
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
   65                  70                  75                  80
   Ile Ser Ser Leu Gln Pro Glu Asp Phe Ala Thr Tyr Tyr Cys Gly Gln
                   85                  90                  95
   Ser Tyr Ser Arg Pro Leu Thr Phe Gly Gln Gly Thr Lys Val Glu Ile
               100                 105                 110
   Lys Arg
<210> 20
   <211> 114
   <212> PRT
   <213> Artificial Sequence
<220>
   <223> light chain variable region of anti c-Met humanized antibody(huAbF46-H4)
<400> 20
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
   65                  70                  75                  80
   Ile Ser Ser Leu Gln Pro Glu Asp Phe Ala Thr Tyr Tyr Cys Ala Gln
                   85                  90                  95
   Ser Tyr Ser His Pro Phe Ser Phe Gly Gln Gly Thr Lys Val Glu Ile
               100                 105                 110
   Lys Arg
<210> 21
   <211> 114
   <212> PRT
   <213> Artificial Sequence
<220>
   <223> light chain variable region of anti c-Met humanized antibody(huAbF46-H4)
<400> 21
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
   20 25 30
   Gly Asn Gln Asn Asn Tyr Leu Ala Trp His Gln Gln Lys Pro Gly Lys
   35 40 45
   Ala Pro Lys Met Leu Ile Ile Trp Ala Ser Thr Arg Val Ser Gly Val
   50 55 60
   Pro Ser Arg Phe Ser Gly Ser Gly Ser Gly Thr Asp Phe Thr Leu Thr
   65 70 75 80
   Ile Ser Ser Leu Gln Pro Glu Asp Phe Ala Thr Tyr Tyr Cys Gln Gln
   85 90 95
   Ser Tyr Ser Arg Pro Phe Thr Phe Gly Gln Gly Thr Lys Val Glu Ile
   100 105 110
   Lys Arg
<210> 22
   <211> 6
   <212> PRT
   <213> Artificial Sequence
<220>
   <223> CDR-H1 derived from H11-4 clone
<400> 22
   Pro Glu Tyr Tyr Met Ser
   1 5
<210> 23
   <211> 6
   <212> PRT
   <213> Artificial Sequence
<220>
   <223> CDR-H1 derived from YC151 clone
<400> 23
   Pro Asp Tyr Tyr Met Ser
   1 5
<210> 24
   <211> 6
   <212> PRT
   <213> Artificial Sequence
<220>
   <223> CDR-H1 derived from YC193 clone
<400> 24
   Ser Asp Tyr Tyr Met Ser
   1 5
<210> 25
   <211> 8
   <212> PRT
   <213> Artificial Sequence
<220>
   <223> CDR-H2 derived from YC244 clone
<400> 25
   Arg Asn Asn Ala Asn Gly Asn Thr
   1               5
<210> 26
   <211> 8
   <212> PRT
   <213> Artificial Sequence
<220>
   <223> CDR-H2 derived from YC321 clone
<400> 26
   Arg Asn Lys Val Asn Gly Tyr Thr
   1               5
<210> 27
   <211> 6
   <212> PRT
   <213> Artificial Sequence
<220>
   <223> CDR-H3 derived from YC354 clone
<400> 27
   Asp Asn Trp Leu Ser Tyr
   1               5
<210> 28
   <211> 6
   <212> PRT
   <213> Artificial Sequence
<220>
   <223> CDR-H3 derived from YC374 clone
<400> 28
   Asp Asn Trp Leu Thr Tyr
   1               5
<210> 29
   <211> 17
   <212> PRT
   <213> Artificial Sequence
<220>
   <223> CDR-L1 derived from L1-1 clone
<400> 29
   Lys Ser Ser His Ser Leu Leu Ala Ser Gly Asn Gln Asn Asn Tyr Leu
   1 5 10 15
   Ala
<210> 30
   <211> 17
   <212> PRT
   <213> Artificial Sequence
<220>
   <223> CDR-L1 derived from L1-3 clone
<400> 30
   Lys Ser Ser Arg Ser Leu Leu Ser Ser Gly Asn His Lys Asn Tyr Leu
   1 5 10 15
   Ala
<210> 31
   <211> 17
   <212> PRT
   <213> Artificial Sequence
<220>
   <223> CDR-L1 derived from L1-4 clone
<400> 31
   Lys Ser Ser Lys Ser Leu Leu Ala Ser Gly Asn Gln Asn Asn Tyr Leu
   1 5 10 15
   Ala
<210> 32
   <211> 17
   <212> PRT
   <213> Artificial Sequence
<220>
   <223> CDR-L1 derived from L1-12 clone
<400> 32
   Lys Ser Ser Arg Ser Leu Leu Ala Ser Gly Asn Gln Asn Asn Tyr Leu
   1 5 10 15
   Ala
<210> 33
   <211> 17
   <212> PRT
   <213> Artificial Sequence
<220>
   <223> CDR-L1 derived from L1-22 clone
<400> 33
   Lys Ser Ser His Ser Leu Leu Ala Ser Gly Asn Gln Asn Asn Tyr Leu
   1 5 10 15
   Ala
<210> 34
   <211> 7
   <212> PRT
   <213> Artificial Sequence
<220>
   <223> CDR-L2 derived from L2-9 clone
<400> 34
   Trp Ala Ser Lys Arg Val Ser
   1 5
<210> 35
   <211> 7
   <212> PRT
   <213> Artificial Sequence
<220>
   <223> CDR-L2 derived from L2-12 clone
<400> 35
   Trp Gly Ser Thr Arg Val Ser
   1 5
<210> 36
   <211> 7
   <212> PRT
   <213> Artificial Sequence
<220>
   <223> CDR-L2 derived from L2-16 clone
<400> 36
   Trp Gly Ser Thr Arg Val Pro
   1 5
<210> 37
   <211> 9
   <212> PRT
   <213> Artificial Sequence
<220>
   <223> CDR-L3 derived from L3-32 clone
<400> 37
   Gln Gln Ser Tyr Ser Lys Pro Phe Thr
   1 5
<210> 38
   <211> 1416
   <212> DNA
   <213> Artificial Sequence
<220>
   <223> nucleotide sequence of heavy chain of chAbF46
<220>
   <221> misc_feature
   <222> (1)..(6)
   <223> EcoRI restriction site
<220>
   <221> misc_feature
   <222> (7)..(66)
   <223> signal sequence
<220>
   <221> misc_feature
   <222> (67)..(417)
   <223> VH - heavy chain variable region
<220>
   <221> misc_feature
   <222> (418)..(423)
   <223> NdeI restriction site
<220>
   <221> misc_feature
   <222> (418)..(1407)
   <223> CH - heavy chain constant region
<220>
   <221> misc_feature
   <222> (1408)..(1410)
   <223> TGA - stop sodon
<220>
   <221> misc_feature
   <222> (1411)..(1416)
   <223> XhoI restriction site
<400> 38
   gaattcgccg ccaccatgga atggagctgg gtttttctcg taacactttt aaatggtatc 60
   cagtgtgagg tgaagctggt ggagtctgga ggaggcttgg tacagcctgg gggttctctg        120
   agactctcct gtgcaacttc tgggttcacc ttcactgatt actacatgag ctgggtccgc        180
   cagcctccag gaaaggcact tgagtggttg ggttttatta gaaacaaagc taatggttac        240
   acaacagagt acagtgcatc tgtgaagggt cggttcacca tctccagaga taattcccaa        300
   agcatcctct atcttcaaat ggacaccctg agagctgagg acagtgccac ttattactgt        360
   gcaagagata actggtttgc ttactggggc caagggactc tggtcactgt ctctgcagct        420
   agcaccaagg gcccatcggt cttccccctg gcaccctcct ccaagagcac ctctgggggc        480
   acagcggccc tgggctgcct ggtcaaggac tacttccccg aaccggtgac ggtgtcgtgg        540
   aactcaggcg ccctgaccag cggcgtgcac accttcccgg ctgtcctaca gtcctcagga        600
   ctctactccc tcagcagcgt ggtgaccgtg ccctccagca gcttgggcac ccagacctac        660
   atctgcaacg tgaatcacaa gcccagcaac accaaggtgg acaagaaagt tgagcccaaa        720
   tcttgtgaca aaactcacac atgcccaccg tgcccagcac ctgaactcct ggggggaccg        780
   tcagtcttcc tcttcccccc aaaacccaag gacaccctca tgatctcccg gacccctgag        840
   gtcacatgcg tggtggtgga cgtgagccac gaagaccctg aggtcaagtt caactggtac        900
   gtggacggcg tggaggtgca taatgccaag acaaagccgc gggaggagca gtacaacagc        960
   acgtaccgtg tggtcagcgt cctcaccgtc ctgcaccagg actggctgaa tggcaaggag       1020
   tacaagtgca aggtctccaa caaagccctc ccagccccca tcgagaaaac catctccaaa       1080
   gccaaagggc agccccgaga accacaggtg tacaccctgc ccccatcccg ggaggagatg       1140
   accaagaacc aggtcagcct gacctgcctg gtcaaaggct tctatcccag cgacatcgcc       1200
   gtggagtggg agagcaatgg gcagccggag aacaactaca agaccacgcc tcccgtgctg       1260
   gactccgacg gctccttctt cctctacagc aagctcaccg tggacaagag caggtggcag       1320
   caggggaacg tcttctcatg ctccgtgatg catgaggctc tgcacaacca ctacacgcag       1380
   aagagcctct ccctgtctcc gggtaaatga ctcgag                                 1416
<210> 39
   <211> 759
   <212> DNA
   <213> Artificial Sequence
<220>
   <223> nucleotide sequence of light chain of chAbF46
<220>
   <221> misc_difference
   <222> (1)..(6)
   <223> EcoRI restriction site
<220>
   <221> misc_difference
   <222> (7)..(90)
   <223> signal sequence
<220>
   <221> misc_difference
   <222> (91)..(432)
   <223> VL - light chain variable region
<220>
   <221> misc_difference
   <222> (430)..(435)
   <223> BsiWI restriction site
<220>
   <221> misc_difference
   <222> (433)..(750)
   <223> CL - light chain constant region
<220>
   <221> misc_difference
   <222> (751)..(753)
   <223> stop codon
<220>
   <221> misc_difference
   <222> (754)..(759)
   <223> XhoI restriction site
<400> 39
   gaattcacta gtgattaatt cgccgccacc atggattcac aggcccaggt cctcatgttg    60
   ctgctgctat cggtatctgg tacctgtgga gacattttga tgacccagtc tccatcctcc   120
   ctgactgtgt cagcaggaga gaaggtcact atgagctgca agtccagtca gagtctttta   180
   gctagtggca accaaaataa ctacttggcc tggcaccagc agaaaccagg acgatctcct   240
   aaaatgctga taatttgggc atccactagg gtatctggag tccctgatcg cttcataggc   300
   agtggatctg ggacggattt cactctgacc atcaacagtg tgcaggctga agatctggct   360
   gtttattact gtcagcagtc ctacagcgct ccgctcacgt tcggtgctgg gaccaagctg   420
   gagctgaaac gtacggtggc tgcaccatct gtcttcatct tcccgccatc tgatgagcag   480
   ttgaaatctg gaactgcctc tgttgtgtgc ctgctgaata acttctatcc cagagaggcc   540
   aaagtacagt ggaaggtgga taacgccctc caatcgggta actcccagga gagtgtcaca   600
   gagcaggaca gcaaggacag cacctacagc ctcagcagca ccctgacgct gagcaaagca   660
   gactacgaga aacacaaagt ctacgcctgc gaagtcaccc atcagggcct gagctcgccc   720
   gtcacaaaga gcttcaacag gggagagtgt tgactcgag                          759
<210> 40
   <211> 447
   <212> PRT
   <213> Artificial Sequence
<220>
   <223> amino acid sequence of H1-heavy
<400> 40
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Thr Asp Tyr
               20                  25                  30
   Tyr Met Ser Trp Val Arg Gln Ala Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Asn Ser
   65                  70                  75                  80
   Leu Tyr Leu Gln Met Asn Ser Leu Lys Thr Glu Asp Thr Ala Val Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 41
   <211> 447
   <212> PRT
   <213> Artificial Sequence
<220>
   <223> amino acid sequence of H3-heavy
<400> 41
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Thr Asp Tyr
   20 25 30
   Tyr Met Ser Trp Val Arg Gln Ala Pro Gly Lys Gly Leu Glu Trp Leu
   35 40 45
   Gly Phe Ile Arg Asn Lys Ala Asn Gly Tyr Thr Thr Glu Tyr Ser Ala
   50 55 60
   Ser Val Lys Gly Arg Phe Thr Ile Ser Arg Asp Asn Ser Lys Asn Ser
   65 70 75 80
   Leu Tyr Leu Gln Met Asn Ser Leu Arg Ala Glu Asp Thr Ala Val Tyr
   85 90 95
   Tyr Cys Ala Arg Asp Asn Trp Phe Ala Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
   435 440 445
<210> 42
   <211> 447
   <212> PRT
   <213> Artificial Sequence
<220>
   <223> amino acid sequence of H4-heavy
<400> 42
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Thr Asp Tyr
   20 25 30
   Tyr Met Ser Trp Val Arg Gln Ala Pro Gly Lys Gly Leu Glu Trp Leu
   35 40 45
   Gly Phe Ile Arg Asn Lys Ala Asn Gly Tyr Thr Thr Glu Tyr Ser Ala
   50 55 60
   Ser Val Lys Gly Arg Phe Thr Ile Ser Arg Asp Asn Ser Lys Asn Thr
   65 70 75 80
   Leu Tyr Leu Gln Met Asn Ser Leu Arg Ala Glu Asp Thr Ala Val Tyr
   85 90 95
   Tyr Cys Ala Arg Asp Asn Trp Phe Ala Tyr Trp Gly Gln Gly Thr Leu
   100 105 110
   Val Thr Val Ser Ser Ala Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Lys Val Glu Pro Lys Ser Cys Asp Lys Thr His
   210 215 220
   Thr Cys Pro Pro Cys Pro Ala Pro Glu Leu Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 43
   <211> 220
   <212> PRT
   <213> Artificial Sequence
<220>
   <223> amino acid sequence of H1-light
<400> 43
   Asp Ile Val Met Thr Gln Ser Pro Asp Ser Leu Ala Val Ser Leu Gly
   1 5 10 15
   Glu Arg Ala Thr Ile Asn Cys Lys Ser Ser Gln Ser Leu Leu Ala Ser
   20 25 30
   Gly Asn Gln Asn Asn Tyr Leu Ala Trp His Gln Gln Lys Pro Gly Gln
   35 40 45
   Pro Pro Lys Met Leu Ile Ile Trp Ala Ser Thr Arg Val Ser Gly Val
   50 55 60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Ser Tyr Ser Ala Pro Leu Thr Phe Gly Gly Gly Thr Lys Val Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 44
   <211> 220
   <212> PRT
   <213> Artificial Sequence
<220>
   <223> amino acid sequence of H2-light
<400> 44
   Asp Ile Val Met Thr Gln Thr Pro Leu Ser Leu Pro Val Thr Pro Gly
   1               5                   10                  15
   Glu Pro Ala Ser Ile Ser Cys Lys Ser Ser Gln Ser Leu Leu Ala Ser
               20                  25                  30
   Gly Asn Gln Asn Asn Tyr Leu Ala Trp His Leu Gln Lys Pro Gly Gln
           35                  40                  45
   Ser Pro Gln Met Leu Ile Ile Trp Ala Ser Thr Arg Val Ser Gly Val
       50                  55                  60
   Pro Asp Arg Phe Ser Gly Ser Gly Ser Gly Thr Asp Phe Thr Leu Lys
   65                  70                  75                  80
   Ile Ser Arg Val Glu Ala Glu Asp Val Gly Val Tyr Tyr Cys Gln Gln
                   85                  90                  95
   Ser Tyr Ser Ala Pro Leu Thr Phe Gly Gln Gly Thr Lys Leu Glu Leu
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 45
   <211> 220
   <212> PRT
   <213> Artificial Sequence
<220>
   <223> amino acid sequence of H3-light
<400> 45
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Leu Leu Ala Ser
               20                  25                  30
   Gly Asn Gln Asn Asn Tyr Leu Ala Trp Tyr Gln Gln Lys Pro Gly Gln
           35                  40                  45
   Pro Pro Lys Leu Leu Ile Ile Trp Ala Ser Thr Arg Val Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Ser Tyr Ser Ala Pro Leu Thr Phe Gly Gly Gly Thr Lys Val Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 46
   <211> 219
   <212> PRT
   <213> Artificial Sequence
<220>
   <223> amino acid sequence of H4-light
<400> 46
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
   65                  70                  75                  80
   Ile Ser Ser Leu Gln Pro Glu Asp Phe Ala Thr Tyr Tyr Cys Gln Gln
                   85                  90                  95
   Ser Tyr Ser Ala Pro Leu Thr Phe Gly Gln Gly Thr Lys Val Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu
       210                 215
<210> 47
   <211> 1350
   <212> DNA
   <213> Artificial Sequence
<220>
   <223> nucleotide sequence of H1-heavy
<400> 47
   gaggtgcagc tggtggagtc tgggggaggc ttggtccagc ctggagggtc cctgagactc      60
   tcctgtgcag cctctggatt caccttcact gactactaca tgagctgggt ccgccaggct     120
   ccagggaagg ggctggagtg gttgggcttt attagaaaca aagctaacgg ttacaccaca     180
   gaatacagtg cgtctgtgaa aggcagattc accatctcaa gagataattc aaagaactca     240
   ctgtatctgc aaatgaacag cctgaaaacc gaggacacgg ccgtgtatta ctgtgctaga     300
   gataactggt ttgcttactg gggtcaagga accctggtca ccgtctcctc ggctagcacc     360
   aagggcccat cggtcttccc cctggcaccc tcctccaaga gcacctctgg gggcacagcg     420
   gccctgggct gcctggtcaa ggactacttc cccgaaccgg tgacggtgtc gtggaactca     480
   ggcgccctga ccagcggcgt gcacaccttc ccggctgtcc tacagtcctc aggactctac     540
   tccctcagca gcgtggtgac cgtgccctcc agcagcttgg gcacccagac ctacatctgc     600
   aacgtgaatc acaagcccag caacaccaag gtggacaaga aagttgagcc caaatcttgt     660
   gacaaaactc acacatgccc accgtgccca gcacctgaac tcctgggggg accgtcagtc     720
   ttcctcttcc ccccaaaacc caaggacacc ctcatgatct cccggacccc tgaggtcaca     780
   tgcgtggtgg tggacgtgag ccacgaagac cctgaggtca agttcaactg gtacgtggac     840
   ggcgtggagg tgcataatgc caagacaaag ccgcgggagg agcagtacaa cagcacgtac     900
   cgtgtggtca gcgtcctcac cgtcctgcac caggactggc tgaatggcaa ggagtacaag     960
   tgcaaggtct ccaacaaagc cctcccagcc cccatcgaga aaaccatctc caaagccaaa    1020
   gggcagcccc gagaaccaca ggtgtacacc ctgcccccat cccgggagga gatgaccaag    1080
   aaccaggtca gcctgacctg cctggtcaaa ggcttctatc ccagcgacat cgccgtggag    1140
   tgggagagca atgggcagcc ggagaacaac tacaagacca cgcctcccgt gctggactcc    1200
   gacggctcct tcttcctcta cagcaagctc accgtggaca agagcaggtg gcagcagggg    1260
   aacgtcttct catgctccgt gatgcatgag gctctgcaca accactacac gcagaagagc    1320
   ctctccctgt ctccgggtaa atgactcgag                                     1350
<210> 48
   <211> 1350
   <212> DNA
   <213> Artificial Sequence
<220>
   <223> nucleotide sequence of H3-heavy
<400> 48
   gaggtgcagc tggtggagtc tgggggaggc ttggtccagc ctggagggtc cctgagactc 60
   tcctgtgcag cctctggatt caccttcact gactactaca tgagctgggt ccgccaggct 120
   ccagggaagg ggctggagtg gttgggcttt attagaaaca aagctaacgg ttacaccaca 180
   gaatacagtg cgtctgtgaa aggcagattc accatctcaa gagataattc aaagaactca 240
   ctgtatctgc aaatgaacag cctgcgtgct gaggacacgg ccgtgtatta ctgtgctaga 300
   gataactggt ttgcttactg gggtcaagga accctggtca ccgtctcctc ggctagcacc 360
   aagggcccat cggtcttccc cctggcaccc tcctccaaga gcacctctgg gggcacagcg 420
   gccctgggct gcctggtcaa ggactacttc cccgaaccgg tgacggtgtc gtggaactca 480
   ggcgccctga ccagcggcgt gcacaccttc ccggctgtcc tacagtcctc aggactctac 540
   tccctcagca gcgtggtgac cgtgccctcc agcagcttgg gcacccagac ctacatctgc 600
   aacgtgaatc acaagcccag caacaccaag gtggacaaga aagttgagcc caaatcttgt 660
   gacaaaactc acacatgccc accgtgccca gcacctgaac tcctgggggg accgtcagtc 720
   ttcctcttcc ccccaaaacc caaggacacc ctcatgatct cccggacccc tgaggtcaca 780
   tgcgtggtgg tggacgtgag ccacgaagac cctgaggtca agttcaactg gtacgtggac 840
   ggcgtggagg tgcataatgc caagacaaag ccgcgggagg agcagtacaa cagcacgtac 900
   cgtgtggtca gcgtcctcac cgtcctgcac caggactggc tgaatggcaa ggagtacaag 960
   tgcaaggtct ccaacaaagc cctcccagcc cccatcgaga aaaccatctc caaagccaaa 1020
   gggcagcccc gagaaccaca ggtgtacacc ctgcccccat cccgggagga gatgaccaag 1080
   aaccaggtca gcctgacctg cctggtcaaa ggcttctatc ccagcgacat cgccgtggag 1140
   tgggagagca atgggcagcc ggagaacaac tacaagacca cgcctcccgt gctggactcc 1200
   gacggctcct tcttcctcta cagcaagctc accgtggaca agagcaggtg gcagcagggg 1260
   aacgtcttct catgctccgt gatgcatgag gctctgcaca accactacac gcagaagagc 1320
   ctctccctgt ctccgggtaa atgactcgag 1350
<210> 49
   <211> 1350
   <212> DNA
   <213> Artificial Sequence
<220>
   <223> nucleotide sequence of H4-heavy
<400> 49
   gaggttcagc tggtggagtc tggcggtggc ctggtgcagc cagggggctc actccgtttg 60
   tcctgtgcag cttctggctt caccttcact gattactaca tgagctgggt gcgtcaggcc      120
   ccgggtaagg gcctggaatg gttgggtttt attagaaaca aagctaatgg ttacacaaca      180
   gagtacagtg catctgtgaa gggtcgtttc actataagca gagataattc caaaaacaca      240
   ctgtacctgc agatgaacag cctgcgtgct gaggacactg ccgtctatta ttgtgctaga      300
   gataactggt ttgcttactg gggccaaggg actctggtca ccgtctcctc ggctagcacc      360
   aagggcccat cggtcttccc cctggcaccc tcctccaaga gcacctctgg gggcacagcg      420
   gccctgggct gcctggtcaa ggactacttc cccgaaccgg tgacggtgtc gtggaactca      480
   ggcgccctga ccagcggcgt gcacaccttc ccggctgtcc tacagtcctc aggactctac      540
   tccctcagca gcgtggtgac cgtgccctcc agcagcttgg gcacccagac ctacatctgc      600
   aacgtgaatc acaagcccag caacaccaag gtggacaaga aagttgagcc caaatcttgt      660
   gacaaaactc acacatgccc accgtgccca gcacctgaac tcctgggggg accgtcagtc      720
   ttcctcttcc ccccaaaacc caaggacacc ctcatgatct cccggacccc tgaggtcaca      780
   tgcgtggtgg tggacgtgag ccacgaagac cctgaggtca agttcaactg gtacgtggac      840
   ggcgtggagg tgcataatgc caagacaaag ccgcgggagg agcagtacaa cagcacgtac      900
   cgtgtggtca gcgtcctcac cgtcctgcac caggactggc tgaatggcaa ggagtacaag      960
   tgcaaggtct ccaacaaagc cctcccagcc cccatcgaga aaaccatctc caaagccaaa     1020
   gggcagcccc gagaaccaca ggtgtacacc ctgcccccat cccgggagga gatgaccaag     1080
   aaccaggtca gcctgacctg cctggtcaaa ggcttctatc ccagcgacat cgccgtggag     1140
   tgggagagca atgggcagcc ggagaacaac tacaagacca cgcctcccgt gctggactcc     1200
   gacggctcct tcttcctcta cagcaagctc accgtggaca agagcaggtg gcagcagggg     1260
   aacgtcttct catgctccgt gatgcatgag gctctgcaca accactacac gcagaagagc     1320
   ctctccctgt ctccgggtaa atgactcgag                                      1350
<210> 50
   <211> 669
   <212> DNA
   <213> Artificial Sequence
<220>
   <223> nucleotide sequence of H1-light
<400> 50
   gacatcgtga tgacccagtc tccagactcc ctggctgtgt ctctgggcga gagggccacc 60
   atcaactgca agtccagcca gagtctttta gctagcggca accaaaataa ctacttagct 120
   tggcaccagc agaaaccagg acagcctcct aagatgctca ttatttgggc atctacccgg 180
   gtatccgggg tccctgaccg attcagtggc agcgggtctg ggacagattt cactctcacc 240
   atcagcagcc tgcaggctga agatgtggca gtttattact gtcagcaatc ctatagtgct 300
   cctctcacgt tcggaggcgg taccaaggtg gagatcaaac gtacggtggc tgcaccatct 360
   gtcttcatct tcccgccatc tgatgagcag ttgaaatctg gaactgcctc tgttgtgtgc 420
   ctgctgaata acttctatcc cagagaggcc aaagtacagt ggaaggtgga taacgccctc 480
   caatcgggta actcccagga gagtgtcaca gagcaggaca gcaaggacag cacctacagc 540
   ctcagcagca ccctgacgct gagcaaagca gactacgaga aacacaaagt ctacgcctgc 600
   gaagtcaccc atcagggcct gagctcgccc gtcacaaaga gcttcaacag gggagagtgt 660
   tgactcgag 669
<210> 51
   <211> 669
   <212> DNA
   <213> Artificial Sequence
<220>
   <223> nucleotide sequence of H2-light
<400> 51
   gatattgtga tgacccagac tccactctcc ctgcccgtca cccctggaga gccggcctcc 60
   atctcctgca agtccagtca gagtctttta gctagtggca accaaaataa ctacttggcc 120
   tggcacctgc agaagccagg gcagtctcca cagatgctga tcatttgggc atccactagg 180
   gtatctggag tcccagacag gttcagtggc agtgggtcag gcactgattt cacactgaaa 240
   atcagcaggg tggaggctga ggatgttgga gtttattact gccagcagtc ctacagcgct 300
   ccgctcacgt tcggacaggg taccaagctg gagctcaaac gtacggtggc tgcaccatct 360
   gtcttcatct tcccgccatc tgatgagcag ttgaaatctg gaactgcctc tgttgtgtgc 420
   ctgctgaata acttctatcc cagagaggcc aaagtacagt ggaaggtgga taacgccctc 480
   caatcgggta actcccagga gagtgtcaca gagcaggaca gcaaggacag cacctacagc 540
   ctcagcagca ccctgacgct gagcaaagca gactacgaga aacacaaagt ctacgcctgc 600
   gaagtcaccc atcagggcct gagctcgccc gtcacaaaga gcttcaacag gggagagtgt 660
   tgactcgag 669
<210> 52
   <211> 669
   <212> DNA
   <213> Artificial Sequence
<220>
   <223> nucleotide sequence of H3-light
<400> 52
   gacatcgtga tgacccagtc tccagactcc ctggctgtgt ctctgggcga gagggccacc      60
   atcaactgca agtccagcca gagtctttta gctagcggca accaaaataa ctacttagct     120
   tggtaccagc agaaaccagg acagcctcct aagctgctca ttatttgggc atctacccgg     180
   gtatccgggg tccctgaccg attcagtggc agcgggtctg ggacagattt cactctcacc     240
   atcagcagcc tgcaggctga agatgtggca gtttattact gtcagcaatc ctatagtgct     300
   cctctcacgt tcggaggcgg taccaaggtg gagatcaaac gtacggtggc tgcaccatct     360
   gtcttcatct tcccgccatc tgatgagcag ttgaaatctg gaactgcctc tgttgtgtgc     420
   ctgctgaata acttctatcc cagagaggcc aaagtacagt ggaaggtgga taacgccctc     480
   caatcgggta actcccagga gagtgtcaca gagcaggaca gcaaggacag cacctacagc     540
   ctcagcagca ccctgacgct gagcaaagca gactacgaga aacacaaagt ctacgcctgc     600
   gaagtcaccc atcagggcct gagctcgccc gtcacaaaga gcttcaacag gggagagtgt     660
   tgactcgag                                                             669
<210> 53
   <211> 669
   <212> DNA
   <213> Artificial Sequence
<220>
   <223> nucleotide sequence of H4-light
<400> 53
   gatatccaga tgacccagtc cccgagctcc ctgtccgcct ctgtgggcga tagggtcacc          60
   atcacctgca agtccagtca gagtctttta gctagtggca accaaaataa ctacttggcc         120
   tggcaccaac agaaaccagg aaaagctccg aaaatgctga ttatttgggc atccactagg         180
   gtatctggag tcccttctcg cttctctgga tccgggtctg ggacggattt cactctgacc         240
   atcagcagtc tgcagccgga agacttcgca acttattact gtcagcagtc ctacagcgct         300
   ccgctcacgt tcggacaggg taccaaggtg gagatcaaac gtacggtggc tgcaccatct         360
   gtcttcatct tcccgccatc tgatgagcag ttgaaatctg gaactgcctc tgttgtgtgc         420
   ctgctgaata acttctatcc cagagaggcc aaagtacagt ggaaggtgga taacgccctc         480
   caatcgggta actcccagga gagtgtcaca gagcaggaca gcaaggacag cacctacagc         540
   ctcagcagca ccctgacgct gagcaaagca gactacgaga aacacaaagt ctacgcctgc         600
   gaagtcaccc atcagggcct gagctcgccc gtcacaaaga gcttcaacag gggagagtgt         660
   tgactcgag                                                                 669
<210> 54
   <211> 23
   <212> PRT
   <213> Artificial Sequence
<220>
   <223> linker between VH and VL
<400> 54
   Gly Leu Gly Gly Leu Gly Gly Gly Gly Ser Gly Gly Gly Gly Ser Gly
   1               5                   10                  15
   Gly Ser Ser Gly Val Gly Ser
               20
<210> 55
   <211> 1088
   <212> DNA
   <213> Artificial Sequence
<220>
   <223> polynucleotide encoding scFv of huAbF46 antibody
<400> 55
   gctagcgttt tagcagaagt tcaattggtt gaatctggtg gtggtttggt tcaaccaggt      60
   ggttctttga gattgtcttg tgctgcttct ggttttactt tcaccgatta ttacatgtcc     120
   tgggttagac aagctccagg taaaggtttg gaatggttgg gtttcattag aaacaaggct     180
   aacggttaca ctaccgaata ttctgcttct gttaagggta gattcaccat ttctagagac     240
   aactctaaga acaccttgta cttgcaaatg aactccttga gagctgaaga tactgctgtt     300
   tattactgcg ctagagataa ttggtttgct tattggggtc aaggtacttt ggttactgtt     360
   tcttctggcc tcgggggcct cggaggagga ggtagtggcg gaggaggctc cggtggatcc     420
   agcggtgtgg gttccgatat tcaaatgacc caatctccat cttctttgtc tgcttcagtt     480
   ggtgatagag ttaccattac ttgtaagtcc tcccaatctt tgttggcttc tggtaatcag     540
   aacaattact tggcttggca tcaacaaaaa ccaggtaaag ctccaaagat gttgattatt     600
   tgggcttcta ccagagtttc tggtgttcca tctagatttt ctggttctgg ttccggtact     660
   gattttactt tgaccatttc atccttgcaa ccagaagatt tcgctactta ctactgtcaa     720
   caatcttact ctgctccatt gacttttggt caaggtacaa aggtcgaaat caagagagaa     780
   ttcggtaagc ctatccctaa ccctctcctc ggtctcgatt ctacgggtgg tggtggatct     840
   ggtggtggtg gttctggtgg tggtggttct caggaactga caactatatg cgagcaaatc     900
   ccctcaccaa ctttagaatc gacgccgtac tctttgtcaa cgactactat tttggccaac     960
   gggaaggcaa tgcaaggagt ttttgaatat tacaaatcag taacgtttgt cagtaattgc    1020
   ggttctcacc cctcaacaac tagcaaaggc agccccataa acacacagta tgttttttga    1080
   gtttaaac                                                              1088
<210> 56
   <211> 5597
   <212> DNA
   <213> Artificial Sequence
<220>
   <223> expression vector including polynucleotide encoding scFv of huAbF46 antibody
<220>
   <221> misc_difference
   <222> (573)..(578)
   <223> NheI restriction site
<220>
   <221> misc_difference
   <222> (588)..(938)
   <223> huAbF46 VH
<220>
   <221> misc_difference
   <222> (939)..(1007)
   <223> linker
<220>
   <221> misc_difference
   <222> (1008)..(1349)
   <223> huAbF46 VL
<220>
   <221> misc_difference
   <222> (1350)..(1355)
   <223> EcoRI restriction site
<220>
   <221> misc_difference
   <222> (1356)..(1397)
   <223> V5 epitope
<220>
   <221> misc_difference
   <222> (1398)..(1442)
   <223> (G4S)3 linker
<220>
   <221> misc_difference
   <222> (1443)..(1649)
   <223> Aga2
<220>
   <221> misc_difference
   <222> (1650)..(1652)
   <223> TGA(stop codon)
<220>
   <221> misc_difference
   <222> (1653)..(1660)
   <223> PmeI restriction site
<400> 56
   acggattaga agccgccgag cgggtgacag ccctccgaag gaagactctc ctccgtgcgt      60
   cctcgtcttc accggtcgcg ttcctgaaac gcagatgtgc ctcgcgccgc actgctccga     120
   acaataaaga ttctacaata ctagctttta tggttatgaa gaggaaaaat tggcagtaac     180
   ctggccccac aaaccttcaa atgaacgaat caaattaaca accataggat gataatgcga     240
   ttagtttttt agccttattt ctggggtaat taatcagcga agcgatgatt tttgatctat     300
   taacagatat ataaatgcaa aaactgcata accactttaa ctaatacttt caacattttc     360
   ggtttgtatt acttcttatt caaatgtaat aaaagtatca acaaaaaatt gttaatatac     420
   ctctatactt taacgtcaag gagaaaaaac cccggatcgg actactagca gctgtaatac     480
   gactcactat agggaatatt aagctaattc tacttcatac attttcaatt aagatgcagt     540
   tacttcgctg tttttcaata ttttctgtta ttgctagcgt tttagcagaa gttcaattgg     600
   ttgaatctgg tggtggtttg gttcaaccag gtggttcttt gagattgtct tgtgctgctt     660
   ctggttttac tttcaccgat tattacatgt cctgggttag acaagctcca ggtaaaggtt     720
   tggaatggtt gggtttcatt agaaacaagg ctaacggtta cactaccgaa tattctgctt     780
   ctgttaaggg tagattcacc atttctagag acaactctaa gaacaccttg tacttgcaaa     840
   tgaactcctt gagagctgaa gatactgctg tttattactg cgctagagat aattggtttg     900
   cttattgggg tcaaggtact ttggttactg tttcttctgg cctcgggggc ctcggaggag     960
   gaggtagtgg cggaggaggc tccggtggat ccagcggtgt gggttccgat attcaaatga    1020
   cccaatctcc atcttctttg tctgcttcag ttggtgatag agttaccatt acttgtaagt    1080
   cctcccaatc tttgttggct tctggtaatc agaacaatta cttggcttgg catcaacaaa    1140
   aaccaggtaa agctccaaag atgttgatta tttgggcttc taccagagtt tctggtgttc    1200
   catctagatt ttctggttct ggttccggta ctgattttac tttgaccatt tcatccttgc    1260
   aaccagaaga tttcgctact tactactgtc aacaatctta ctctgctcca ttgacttttg    1320
   gtcaaggtac aaaggtcgaa atcaagagag aattcggtaa gcctatccct aaccctctcc    1380
   tcggtctcga ttctacgggt ggtggtggat ctggtggtgg tggttctggt ggtggtggtt    1440
   ctcaggaact gacaactata tgcgagcaaa tcccctcacc aactttagaa tcgacgccgt    1500
   actctttgtc aacgactact attttggcca acgggaaggc aatgcaagga gtttttgaat    1560
   attacaaatc agtaacgttt gtcagtaatt gcggttctca cccctcaaca actagcaaag    1620
   gcagccccat aaacacacag tatgtttttt gagtttaaac ccgctgatct gataacaaca    1680
   gtgtagatgt aacaaaatcg actttgttcc cactgtactt ttagctcgta caaaatacaa 1740
   tatacttttc atttctccgt aaacaacatg ttttcccatg taatatcctt ttctattttt 1800
   cgttccgtta ccaactttac acatacttta tatagctatt cacttctata cactaaaaaa 1860
   ctaagacaat tttaattttg ctgcctgcca tatttcaatt tgttataaat tcctataatt 1920
   tatcctatta gtagctaaaa aaagatgaat gtgaatcgaa tcctaagaga attgggcaag 1980
   tgcacaaaca atacttaaat aaatactact cagtaataac ctatttctta gcatttttga 2040
   cgaaatttgc tattttgtta gagtctttta caccatttgt ctccacacct ccgcttacat 2100
   caacaccaat aacgccattt aatctaagcg catcaccaac attttctggc gtcagtccac 2160
   cagctaacat aaaatgtaag ctctcggggc tctcttgcct tccaacccag tcagaaatcg 2220
   agttccaatc caaaagttca cctgtcccac ctgcttctga atcaaacaag ggaataaacg 2280
   aatgaggttt ctgtgaagct gcactgagta gtatgttgca gtcttttgga aatacgagtc 2340
   ttttaataac tggcaaaccg aggaactctt ggtattcttg ccacgactca tctccgtgca 2400
   gttggacgat atcaatgccg taatcattga ccagagccaa aacatcctcc ttaggttgat 2460
   tacgaaacac gccaaccaag tatttcggag tgcctgaact atttttatat gcttttacaa 2520
   gacttgaaat tttccttgca ataaccgggt caattgttct ctttctattg ggcacacata 2580
   taatacccag caagtcagca tcggaatcta gagcacattc tgcggcctct gtgctctgca 2640
   agccgcaaac tttcaccaat ggaccagaac tacctgtgaa attaataaca gacatactcc 2700
   aagctgcctt tgtgtgctta atcacgtata ctcacgtgct caatagtcac caatgccctc 2760
   cctcttggcc ctctcctttt cttttttcga ccgaatttct tgaagacgaa agggcctcgt 2820
   gatacgccta tttttatagg ttaatgtcat gataataatg gtttcttagg acggatcgct 2880
   tgcctgtaac ttacacgcgc ctcgtatctt ttaatgatgg aataatttgg gaatttactc 2940
   tgtgtttatt tatttttatg ttttgtattt ggattttaga aagtaaataa agaaggtaga 3000
   agagttacgg aatgaagaaa aaaaaataaa caaaggttta aaaaatttca acaaaaagcg 3060
   tactttacat atatatttat tagacaagaa aagcagatta aatagatata cattcgatta 3120
   acgataagta aaatgtaaaa tcacaggatt ttcgtgtgtg gtcttctaca cagacaagat 3180
   gaaacaattc ggcattaata cctgagagca ggaagagcaa gataaaaggt agtatttgtt 3240
   ggcgatcccc ctagagtctt ttacatcttc ggaaaacaaa aactattttt tctttaattt 3300
   ctttttttac tttctatttt taatttatat atttatatta aaaaatttaa attataatta 3360
   tttttatagc acgtgatgaa aaggacccag gtggcacttt tcggggaaat gtgcgcggaa 3420
   cccctatttg tttatttttc taaatacatt caaatatgta tccgctcatg agacaataac 3480
   cctgataaat gcttcaataa tattgaaaaa ggaagagtat gagtattcaa catttccgtg 3540
   tcgcccttat tccctttttt gcggcatttt gccttcctgt ttttgctcac ccagaaacgc 3600
   tggtgaaagt aaaagatgct gaagatcagt tgggtgcacg agtgggttac atcgaactgg 3660
   atctcaacag cggtaagatc cttgagagtt ttcgccccga agaacgtttt ccaatgatga 3720
   gcacttttaa agttctgcta tgtggcgcgg tattatcccg tgttgacgcc gggcaagagc 3780
   aactcggtcg ccgcatacac tattctcaga atgacttggt tgagtactca ccagtcacag 3840
   aaaagcatct tacggatggc atgacagtaa gagaattatg cagtgctgcc ataaccatga 3900
   gtgataacac tgcggccaac ttacttctga caacgatcgg aggaccgaag gagctaaccg 3960
   cttttttgca caacatgggg gatcatgtaa ctcgccttga tcgttgggaa ccggagctga 4020
   atgaagccat accaaacgac gagcgtgaca ccacgatgcc tgtagcaatg gcaacaacgt 4080
   tgcgcaaact attaactggc gaactactta ctctagcttc ccggcaacaa ttaatagact 4140
   ggatggaggc ggataaagtt gcaggaccac ttctgcgctc ggcccttccg gctggctggt 4200
   ttattgctga taaatctgga gccggtgagc gtgggtctcg cggtatcatt gcagcactgg 4260
   ggccagatgg taagccctcc cgtatcgtag ttatctacac gacgggcagt caggcaacta 4320
   tggatgaacg aaatagacag atcgctgaga taggtgcctc actgattaag cattggtaac 4380
   tgtcagacca agtttactca tatatacttt agattgattt aaaacttcat ttttaattta 4440
   aaaggatcta ggtgaagatc ctttttgata atctcatgac caaaatccct taacgtgagt 4500
   tttcgttcca ctgagcgtca gaccccgtag aaaagatcaa aggatcttct tgagatcctt 4560
   tttttctgcg cgtaatctgc tgcttgcaaa caaaaaaacc accgctacca gcggtggttt 4620
   gtttgccgga tcaagagcta ccaactcttt ttccgaaggt aactggcttc agcagagcgc 4680
   agataccaaa tactgtcctt ctagtgtagc cgtagttagg ccaccacttc aagaactctg 4740
   tagcaccgcc tacatacctc gctctgctaa tcctgttacc agtggctgct gccagtggcg 4800
   ataagtcgtg tcttaccggg ttggactcaa gacgatagtt accggataag gcgcagcggt 4860
   cgggctgaac ggggggttcg tgcacacagc ccagcttgga gcgaacgacc tacaccgaac 4920
   tgagatacct acagcgtgag cattgagaaa gcgccacgct tcccgaaggg agaaaggcgg 4980
   acaggtatcc ggtaagcggc agggtcggaa caggagagcg cacgagggag cttccagggg 5040
   ggaacgcctg gtatctttat agtcctgtcg ggtttcgcca cctctgactt gagcgtcgat 5100
   ttttgtgatg ctcgtcaggg gggccgagcc tatggaaaaa cgccagcaac gcggcctttt 5160
   tacggttcct ggccttttgc tggccttttg ctcacatgtt ctttcctgcg ttatcccctg 5220
   attctgtgga taaccgtatt accgcctttg agtgagctga taccgctcgc cgcagccgaa 5280
   cgaccgagcg cagcgagtca gtgagcgagg aagcggaaga gcgcccaata cgcaaaccgc 5340
   ctctccccgc gcgttggccg attcattaat gcagctggca cgacaggttt cccgactgga 5400
   aagcgggcag tgagcgcaac gcaattaatg tgagttacct cactcattag gcaccccagg 5460
   ctttacactt tatgcttccg gctcctatgt tgtgtggaat tgtgagcgga taacaatttc     5520
   acacaggaaa cagctatgac catgattacg ccaagctcgg aattaaccct cactaaaggg     5580
   aacaaaagct ggctagt                                                    5597
<210> 57
   <211> 13
   <212> PRT
   <213> Artificial Sequence
<220>
   <223> U6-HC7 hinge
<400> 57
   Glu Pro Lys Ser Cys Asp Cys His Cys Pro Pro Cys Pro
   1               5                   10
<210> 58
   <211> 435
   <212> DNA
   <213> Artificial Sequence
<220>
   <223> polynucleotide encoding CDR-L3 derived from L3-1 clone
<400> 58
   gaattcacta gtgattaatt cgccgccacc atggattcac aggcccaggt cctcatgttg       60
   ctgctgctat cggtatctgg tacctgtgga gatatccaga tgacccagtc cccgagctcc      120
   ctgtccgcct ctgtgggcga tagggtcacc atcacctgca agtccagtca gagtctttta      180
   gctagtggca accaaaataa ctacttggcc tggcaccaac agaaaccagg aaaagctccg      240
   aaaatgctga ttatttgggc atccactagg gtatctggag tcccttctcg cttctctgga      300
   tccgggtctg ggacggattt cactctgacc atcagcagtc tgcagccgga agacttcgca      360
   acttattact gtcagcagtc ctacagccgc ccgtacacgt tcggacaggg taccaaggtg      420
   gagatcaaac gtacg                                                       435
<210> 59
   <211> 435
   <212> DNA
   <213> Artificial Sequence
<220>
   <223> polynucleotide encoding CDR-L3 derived from L3-2 clone
<400> 59
   gaattcacta gtgattaatt cgccgccacc atggattcac aggcccaggt cctcatgttg 60
   ctgctgctat cggtatctgg tacctgtgga gatatccaga tgacccagtc cccgagctcc 120
   ctgtccgcct ctgtgggcga tagggtcacc atcacctgca agtccagtca gagtctttta 180
   gctagtggca accaaaataa ctacttggcc tggcaccaac agaaaccagg aaaagctccg 240
   aaaatgctga ttatttgggc atccactagg gtatctggag tcccttctcg cttctctgga 300
   tccgggtctg ggacggattt cactctgacc atcagcagtc tgcagccgga agacttcgca 360
   acttattact gtgggcagtc ctacagccgt ccgctcacgt tcggacaggg taccaaggtg 420
   gagatcaaac gtacg 435
<210> 60
   <211> 435
   <212> DNA
   <213> Artificial Sequence
<220>
   <223> polynucleotide encoding CDR-L3 derived from L3-3 clone
<400> 60
   gaattcacta gtgattaatt cgccgccacc atggattcac aggcccaggt cctcatgttg 60
   ctgctgctat cggtatctgg tacctgtgga gatatccaga tgacccagtc cccgagctcc 120
   ctgtccgcct ctgtgggcga tagggtcacc atcacctgca agtccagtca gagtctttta 180
   gctagtggca accaaaataa ctacttggcc tggcaccaac agaaaccagg aaaagctccg 240
   aaaatgctga ttatttgggc atccactagg gtatctggag tcccttctcg cttctctgga 300
   tccgggtctg ggacggattt cactctgacc atcagcagtc tgcagccgga agacttcgca 360
   acttattact gtgcacagtc ctacagccat ccgttctctt tcggacaggg taccaaggtg 420
   gagatcaaac gtacg 435
<210> 61
   <211> 435
   <212> DNA
   <213> Artificial Sequence
<220>
   <223> polynucleotide encoding CDR-L3 derived from L3-5 clone
<400> 61
   gaattcacta gtgattaatt cgccgccacc atggattcac aggcccaggt cctcatgttg        60
   ctgctgctat cggtatctgg tacctgtgga gatatccaga tgacccagtc cccgagctcc       120
   ctgtccgcct ctgtgggcga tagggtcacc atcacctgca agtccagtca gagtctttta       180
   gctagtggca accaaaataa ctacttggcc tggcaccaac agaaaccagg aaaagctccg       240
   aaaatgctga ttatttgggc atccactagg gtatctggag tcccttctcg cttctctgga       300
   tccgggtctg ggacggattt cactctgacc atcagcagtc tgcagccgga agacttcgca       360
   acttattact gtcagcagtc ctacagccgc ccgtttacgt tcggacaggg taccaaggtg       420
   gagatcaaac gtacg                                                        435
<210> 62
   <211> 462
   <212> PRT
   <213> Artificial Sequence
<220>
   <223> polypeptide consisting of heavy chain of huAbF46-H4-A1, U6-HC7 hinge and constant region of human IgG1
<400> 62
   Met Glu Trp Ser Trp Val Phe Leu Val Thr Leu Leu Asn Gly Ile Gln
    1               5                  10                  15
   Cys Glu Val Gln Leu Val Glu Ser Gly Gly Gly Leu Val Gln Pro Gly
               20                  25                  30
   Gly Ser Leu Arg Leu Ser Cys Ala Ala Ser Gly Phe Thr Phe Thr Asp
           35                  40                  45
   Tyr Tyr Met Ser Trp Val Arg Gln Ala Pro Gly Lys Gly Leu Glu Trp
       50                  55                  60
   Leu Gly Phe Ile Arg Asn Lys Ala Asn Gly Tyr Thr Thr Glu Tyr Ser
   65                  70                  75                  80
   Ala Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Asp Asn Trp Phe Ala Tyr Trp Gly Gln Gly Thr
           115                 120                 125
   Leu Val Thr Val Ser Ser Ala Ser Thr Lys Gly Pro Ser Val Phe Pro
       130                 135                 140
   Leu Ala Pro Ser Ser Lys Ser Thr Ser Gly Gly Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Ser Leu Gly Thr Gln Thr Tyr Ile Cys Asn Val Asn His Lys Pro Ser
       210                 215                 220
   Asn Thr Lys Val Asp Lys Lys Val Glu Pro Lys Ser Cys Asp Cys His
   225                 230                 235                 240
   Cys Pro Pro Cys Pro Ala Pro Glu Leu Leu Gly Gly Pro Ser Val Phe
                   245                 250                 255
   Leu Phe Pro Pro Lys Pro Lys Asp Thr Leu Met Ile Ser Arg Thr Pro
               260                 265                 270
   Glu Val Thr Cys Val Val Val Asp Val Ser His Glu Asp Pro Glu Val
           275                 280                 285
   Lys Phe Asn Trp Tyr Val Asp Gly Val Glu Val His Asn Ala Lys Thr
       290                 295                 300
   Lys Pro Arg Glu Glu Gln Tyr Asn Ser Thr Tyr Arg Val Val Ser Val
   305                 310                 315                 320
   Leu Thr Val Leu His Gln Asp Trp Leu Asn Gly Lys Glu Tyr Lys Cys
                   325                 330                 335
   Lys Val Ser Asn Lys Ala Leu Pro Ala Pro Ile Glu Lys Thr Ile Ser
               340                 345                 350
   Lys Ala Lys Gly Gln Pro Arg Glu Pro Gln Val Tyr Thr Leu Pro Pro
           355                 360                 365
   Ser Arg Glu Glu Met Thr Lys Asn Gln Val Ser Leu Thr Cys Leu Val
       370                 375                 380
   Lys Gly Phe Tyr Pro Ser Asp Ile Ala Val Glu Trp Glu Ser Asn Gly
   385                 390                 395                 400
   Gln Pro Glu Asn Asn Tyr Lys Thr Thr Pro Pro Val Leu Asp Ser Asp
                   405                 410                 415
   Gly Ser Phe Phe Leu Tyr Ser Lys Leu Thr Val Asp Lys Ser Arg Trp
               420                 425                 430
   Gln Gln Gly Asn Val Phe Ser Cys Ser Val Met His Glu Ala Leu His
           435                 440                 445
   Asn His Tyr Thr Gln Lys Ser Leu Ser Leu Ser Pro Gly Lys
       450                 455                 460
<210> 63
   <211> 1410
   <212> DNA
   <213> Artificial Sequence
<220>
   <223> polynucleotide encoding polypeptide consisting of heavy chain of huAbF46-H4-A1, U6-HC7 hinge and constant region of human IgG1
<400> 63
   gaattcgccg ccaccatgga atggagctgg gtttttctcg taacactttt aaatggtatc      60
   cagtgtgagg ttcagctggt ggagtctggc ggtggcctgg tgcagccagg gggctcactc     120
   cgtttgtcct gtgcagcttc tggcttcacc ttcactgatt actacatgag ctgggtgcgt     180
   caggccccgg gtaagggcct ggaatggttg ggttttatta gaaacaaagc taatggttac     240
   acaacagagt acagtgcatc tgtgaagggt cgtttcacta taagcagaga taattccaaa     300
   aacacactgt acctgcagat gaacagcctg cgtgctgagg acactgccgt ctattattgt     360
   gctagagata actggtttgc ttactggggc caagggactc tggtcaccgt ctcctcggct 420
   agcaccaagg gcccatcggt cttccccctg gcaccctcct ccaagagcac ctctgggggc 480
   acagcggccc tgggctgcct ggtcaaggac tacttccccg aaccggtgac ggtgtcgtgg 540
   aactcaggcg ccctgaccag cggcgtgcac accttcccgg ctgtcctaca gtcctcagga 600
   ctctactccc tcagcagcgt ggtgaccgtg ccctccagca gcttgggcac ccagacctac 660
   atctgcaacg tgaatcacaa gcccagcaac accaaggtgg acaagaaagt tgagcccaaa 720
   agctgcgatt gccactgtcc tccatgtcca gcacctgaac tcctgggggg accgtcagtc 780
   ttcctcttcc ccccaaaacc caaggacacc ctcatgatct cccggacccc tgaggtcaca 840
   tgcgtggtgg tggacgtgag ccacgaagac cctgaggtca agttcaactg gtacgtggac 900
   ggcgtggagg tgcataatgc caagacaaag ccgcgggagg agcagtacaa cagcacgtac 960
   cgtgtggtca gcgtcctcac cgtcctgcac caggactggc tgaatggcaa ggagtacaag 1020
   tgcaaggtct ccaacaaagc cctcccagcc cccatcgaga aaaccatctc caaagccaaa 1080
   gggcagcccc gagaaccaca ggtgtacacc ctgcccccat cccgggagga gatgaccaag 1140
   aaccaggtca gcctgacctg cctggtcaaa ggcttctatc ccagcgacat cgccgtggag 1200
   tgggagagca atgggcagcc ggagaacaac tacaagacca cgcctcccgt gctggactcc 1260
   gacggctcct tcttcctcta cagcaagctc accgtggaca agagcaggtg gcagcagggg 1320
   aacgtcttct catgctccgt gatgcatgag gctctgcaca accactacac gcagaagagc 1380
   ctctccctgt ctccgggtaa atgactcgag 1410
<210> 64
   <211> 461
   <212> PRT
   <213> Artificial Sequence
<220>
   <223> polypeptide consisting of heavy chain of huAbF46-H4-A1, human IgG2 hinge and constant region of human IgG1
<400> 64
   Met Glu Trp Ser Trp Val Phe Leu Val Thr Leu Leu Asn Gly Ile Gln
    1               5                  10                  15
   Cys Glu Val Gln Leu Val Glu Ser Gly Gly Gly Leu Val Gln Pro Gly
               20                  25                  30
   Gly Ser Leu Arg Leu Ser Cys Ala Ala Ser Gly Phe Thr Phe Thr Asp
           35                  40                  45
   Tyr Tyr Met Ser Trp Val Arg Gln Ala Pro Gly Lys Gly Leu Glu Trp
       50                  55                  60
   Leu Gly Phe Ile Arg Asn Lys Ala Asn Gly Tyr Thr Thr Glu Tyr Ser
   65                  70                  75                  80
   Ala Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Asp Asn Trp Phe Ala Tyr Trp Gly Gln Gly Thr
           115                 120                 125
   Leu Val Thr Val Ser Ser Ala Ser Thr Lys Gly Pro Ser Val Phe Pro
       130                 135                 140
   Leu Ala Pro Ser Ser Lys Ser Thr Ser Gly Gly Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Ser Leu Gly Thr Gln Thr Tyr Ile Cys Asn Val Asn His Lys Pro Ser
       210                 215                 220
   Asn Thr Lys Val Asp Lys Lys Val Glu Arg Lys Cys Cys Val Glu Cys
   225                 230                 235                 240
   Pro Pro Cys Pro Ala Pro Glu Leu Leu Gly Gly Pro Ser Val Phe Leu
                   245                 250                 255
   Phe Pro Pro Lys Pro Lys Asp Thr Leu Met Ile Ser Arg Thr Pro Glu
               260                 265                 270
   Val Thr Cys Val Val Val Asp Val Ser His Glu Asp Pro Glu Val Lys
           275                 280                 285
   Phe Asn Trp Tyr Val Asp Gly Val Glu Val His Asn Ala Lys Thr Lys
       290                 295                 300
   Pro Arg Glu Glu Gln Tyr Asn Ser Thr Tyr Arg Val Val Ser Val Leu
   305                 310                 315                 320
   Thr Val Leu His Gln Asp Trp Leu Asn Gly Lys Glu Tyr Lys Cys Lys
                   325                 330                 335
   Val Ser Asn Lys Ala Leu Pro Ala Pro Ile Glu Lys Thr Ile Ser Lys
               340                 345                 350
   Ala Lys Gly Gln Pro Arg Glu Pro Gln Val Tyr Thr Leu Pro Pro Ser
           355                 360                 365
   Arg Glu Glu Met Thr Lys Asn Gln Val Ser Leu Thr Cys Leu Val Lys
       370                 375                 380
   Gly Phe Tyr Pro Ser Asp Ile Ala Val Glu Trp Glu Ser Asn Gly Gln
   385                 390                 395                 400
   Pro Glu Asn Asn Tyr Lys Thr Thr Pro Pro Val Leu Asp Ser Asp Gly
                   405                 410                 415
   Ser Phe Phe Leu Tyr Ser Lys Leu Thr Val Asp Lys Ser Arg Trp Gln
   420 425 430
   Gln Gly Asn Val Phe Ser Cys Ser Val Met His Glu Ala Leu His Asn
   435 440 445
   His Tyr Thr Gln Lys Ser Leu Ser Leu Ser Pro Gly Lys
   450 455 460
<210> 65
   <211> 1407
   <212> DNA
   <213> Artificial Sequence
<220>
   <223> polynucleotide encoding polypeptide consisting of heavy chain of huAbF46-H4-A1, human IgG2 hinge and constant region of human IgG1
<400> 65
   gaattcgccg ccaccatgga atggagctgg gtttttctcg taacactttt aaatggtatc 60
   cagtgtgagg ttcagctggt ggagtctggc ggtggcctgg tgcagccagg gggctcactc 120
   cgtttgtcct gtgcagcttc tggcttcacc ttcactgatt actacatgag ctgggtgcgt 180
   caggccccgg gtaagggcct ggaatggttg ggttttatta gaaacaaagc taatggttac 240
   acaacagagt acagtgcatc tgtgaagggt cgtttcacta taagcagaga taattccaaa 300
   aacacactgt acctgcagat gaacagcctg cgtgctgagg acactgccgt ctattattgt 360
   gctagagata actggtttgc ttactggggc caagggactc tggtcaccgt ctcctcggct 420
   agcaccaagg gcccatcggt cttccccctg gcaccctcct ccaagagcac ctctgggggc 480
   acagcggccc tgggctgcct ggtcaaggac tacttccccg aaccggtgac ggtgtcgtgg 540
   aactcaggcg ccctgaccag cggcgtgcac accttcccgg ctgtcctaca gtcctcagga 600
   ctctactccc tcagcagcgt ggtgaccgtg ccctccagca gcttgggcac ccagacctac 660
   atctgcaacg tgaatcacaa gcccagcaac accaaggtgg acaagaaagt tgagaggaag 720
   tgctgtgtgg agtgcccccc ctgcccagca cctgaactcc tggggggacc gtcagtcttc 780
   ctcttccccc caaaacccaa ggacaccctc atgatctccc ggacccctga ggtcacatgc 840
   gtggtggtgg acgtgagcca cgaagaccct gaggtcaagt tcaactggta cgtggacggc 900
   gtggaggtgc ataatgccaa gacaaagccg cgggaggagc agtacaacag cacgtaccgt 960
   gtggtcagcg tcctcaccgt cctgcaccag gactggctga atggcaagga gtacaagtgc 1020
   aaggtctcca acaaagccct cccagccccc atcgagaaaa ccatctccaa agccaaaggg 1080
   cagccccgag aaccacaggt gtacaccctg cccccatccc gggaggagat gaccaagaac 1140
   caggtcagcc tgacctgcct ggtcaaaggc ttctatccca gcgacatcgc cgtggagtgg 1200
   gagagcaatg ggcagccgga gaacaactac aagaccacgc ctcccgtgct ggactccgac 1260
   ggctccttct tcctctacag caagctcacc gtggacaaga gcaggtggca gcaggggaac 1320
   gtcttctcat gctccgtgat gcatgaggct ctgcacaacc actacacgca gaagagcctc 1380
   tccctgtctc cgggtaaatg actcgag 1407
<210> 66
   <211> 460
   <212> PRT
   <213> Artificial Sequence
<220>
   <223> polypeptide consisting of heavy chain of huAbF46-H4-A1, human IgG2 hinge and constant region of human IgG2
<400> 66
   Met Glu Trp Ser Trp Val Phe Leu Val Thr Leu Leu Asn Gly Ile Gln
   1               5                   10                  15
   Cys Glu Val Gln Leu Val Glu Ser Gly Gly Gly Leu Val Gln Pro Gly
               20                  25                  30
   Gly Ser Leu Arg Leu Ser Cys Ala Ala Ser Gly Phe Thr Phe Thr Asp
           35                  40                  45
   Tyr Tyr Met Ser Trp Val Arg Gln Ala Pro Gly Lys Gly Leu Glu Trp
       50                  55                  60
   Leu Gly Phe Ile Arg Asn Lys Ala Asn Gly Tyr Thr Thr Glu Tyr Ser
   65                  70                  75                  80
   Ala Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Asp Asn Trp Phe Ala Tyr Trp Gly Gln Gly Thr
           115                 120                 125
   Leu Val Thr Val Ser Ser Ala Ser Thr Lys Gly Pro Ser Val Phe Pro
       130                 135                 140
   Leu Ala Pro Cys Ser Arg Ser Thr Ser Glu Ser Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Asn Phe Gly Thr Gln Thr Tyr Thr Cys Asn Val Asp His Lys Pro Ser
       210                 215                 220
   Asn Thr Lys Val Asp Lys Thr Val Glu Arg Lys Cys Cys Val Glu Cys
   225                 230                 235                 240
   Pro Pro Cys Pro Ala Pro Pro Val Ala Gly Pro Ser Val Phe Leu Phe
                   245                 250                 255
   Pro Pro Lys Pro Lys Asp Thr Leu Met Ile Ser Arg Thr Pro Glu Val
               260                 265                 270
   Thr Cys Val Val Val Asp Val Ser His Glu Asp Pro Glu Val Gln Phe
           275                 280                 285
   Asn Trp Tyr Val Asp Gly Val Glu Val His Asn Ala Lys Thr Lys Pro
       290                 295                 300
   Arg Glu Glu Gln Phe Asn Ser Thr Phe Arg Val Val Ser Val Leu Thr
   305                 310                 315                 320
   Val Val His Gln Asp Trp Leu Asn Gly Lys Glu Tyr Lys Cys Lys Val
                   325                 330                 335
   Ser Asn Lys Gly Leu Pro Ala Pro Ile Glu Lys Thr Ile Ser Lys Thr
               340                 345                 350
   Lys Gly Gln Pro Arg Glu Pro Gln Val Tyr Thr Leu Pro Pro Ser Arg
           355                 360                 365
   Glu Glu Met Thr Lys Asn Gln Val Ser Leu Thr Cys Leu Val Lys Gly
       370                 375                 380
   Phe Tyr Pro Ser Asp Ile Ala Val Glu Trp Glu Ser Asn Gly Gln Pro
   385                 390                 395                 400
   Glu Asn Asn Tyr Lys Thr Thr Pro Pro Met Leu Asp Ser Asp Gly Ser
                   405                 410                 415
   Phe Phe Leu Tyr Ser Lys Leu Thr Val Asp Lys Ser Arg Trp Gln Gln
               420                 425                 430
   Gly Asn Val Phe Ser Cys Ser Val Met His Glu Ala Leu His Asn His
           435                 440                 445
   Tyr Thr Gln Lys Ser Leu Ser Leu Ser Pro Gly Lys
       450                 455                 460
<210> 67
   <211> 1404
   <212> DNA
   <213> Artificial Sequence
<220>
   <223> polynucleotide encoding polypeptide consisting of heavy chain of huAbF46-H4-A1, human IgG2 hinge and constant region of human IgG2
<400> 67
   gaattcgccg ccaccatgga atggagctgg gtttttctcg taacactttt aaatggtatc      60
   cagtgtgagg ttcagctggt ggagtctggc ggtggcctgg tgcagccagg gggctcactc     120
   cgtttgtcct gtgcagcttc tggcttcacc ttcactgatt actacatgag ctgggtgcgt     180
   caggccccgg gtaagggcct ggaatggttg ggttttatta gaaacaaagc taatggttac     240
   acaacagagt acagtgcatc tgtgaagggt cgtttcacta taagcagaga taattccaaa     300
   aacacactgt acctgcagat gaacagcctg cgtgctgagg acactgccgt ctattattgt     360
   gctagagata actggtttgc ttactggggc caagggactc tggtcaccgt ctcctcggct     420
   agcaccaagg gcccatcggt cttccccctg gcgccctgct ccaggagcac ctccgagagc     480
   acagcggccc tgggctgcct ggtcaaggac tacttccccg aaccggtgac ggtgtcgtgg     540
   aactcaggcg ctctgaccag cggcgtgcac accttcccag ctgtcctaca gtcctcagga     600
   ctctactccc tcagcagcgt ggtgaccgtg ccctccagca acttcggcac ccagacctac     660
   acctgcaacg tagatcacaa gcccagcaac accaaggtgg acaagacagt tgagcgcaaa     720
   tgttgtgtcg agtgcccacc gtgcccagca ccacctgtgg caggaccgtc agtcttcctc     780
   ttccccccaa aacccaagga caccctcatg atctcccgga cccctgaggt cacgtgcgtg     840
   gtggtggacg tgagccacga agaccccgag gtccagttca actggtacgt ggacggcgtg     900
   gaggtgcata atgccaagac aaagccacgg gaggagcagt tcaacagcac gttccgtgtg     960
   gtcagcgtcc tcaccgttgt gcaccaggac tggctgaacg gcaaggagta caagtgcaag    1020
   gtctccaaca aaggcctccc agcccccatc gagaaaacca tctccaaaac caaagggcag    1080
   ccccgagaac cacaggtgta caccctgccc ccatcccggg aggagatgac caagaaccag    1140
   gtcagcctga cctgcctggt caaaggcttc taccccagcg acatcgccgt ggagtgggag    1200
   agcaatgggc agccggagaa caactacaag accacgcctc ccatgctgga ctccgacggc    1260
   tccttcttcc tctacagcaa gctcaccgtg gacaagagca ggtggcagca ggggaacgtc    1320
   ttctcatgct ccgtgatgca tgaggctctg cacaaccact acacgcagaa gagcctctcc    1380
   ctgtctccgg gtaaatgact cgag                                           1404
<210> 68
   <211> 240
   <212> PRT
   <213> Artificial Sequence
<220>
   <223> polypeptide consisting of light chain of huAbF46-H4-A1(H36Y) and human kappa constant region
<400> 68
<210> 69
   <211> 758
   <212> DNA
   <213> Artificial Sequence
<220>
   <223> polynucleotide encoding polypeptide consisting of light chain of huAbF46-H4-A1(H36Y) and human kappa constant region
<400> 69
   aattcactag tgattaattc gccgccacca tggattcaca ggcccaggtc ctcatgttgc 60
   tgctgctatc ggtatctggt acctgtggag atatccagat gacccagtcc ccgagctccc 120
   tgtccgcctc tgtgggcgat agggtcacca tcacctgcaa gtccagtcag agtcttttag 180
   ctagtggcaa ccaaaataac tacttggcct ggtaccaaca gaaaccagga aaagctccga 240
   aaatgctgat tatttgggca tccactaggg tatctggagt cccttctcgc ttctctggat 300
   ccgggtctgg gacggatttc actctgacca tcagcagtct gcagccggaa gacttcgcaa 360
   cttattactg tcagcagtcc tacagccgcc cgtacacgtt cggacagggt accaaggtgg 420
   agatcaaacg tacggtggct gcaccatctg tcttcatctt cccgccatct gatgagcagt 480
   tgaaatctgg aactgcctct gttgtgtgcc tgctgaataa cttctatccc agagaggcca 540
   aagtacagtg gaaggtggat aacgccctcc aatcgggtaa ctcccaggag agtgtcacag 600
   agcaggacag caaggacagc acctacagcc tcagcagcac cctgacgctg agcaaagcag 660
   actacgagaa acacaaagtc tacgcctgcg aagtcaccca tcagggcctg agctcgcccg 720
   tcacaaagag cttcaacagg ggagagtgtt gactcgag 758
<210> 70
   <211> 240
   <212> PRT
   <213> Artificial Sequence
<220>
   <223> polypeptide consisting of light chain of huAbF46-H4-A1 and human kappa constant region
<400> 70
   Met Asp Ser Gln Ala Gln Val Leu Met Leu Leu Leu Leu Ser Val Ser
   1 5 10 15
   Gly Thr Cys Gly Asp Ile Gln Met Thr Gln Ser Pro Ser Ser Leu Ser
   20 25 30
   Ala Ser Val Gly Asp Arg Val Thr Ile Thr Cys Lys Ser Ser Gln Ser
   35 40 45
   Leu Leu Ala Ser Gly Asn Gln Asn Asn His Leu Ala Trp Tyr Gln Gln
   50 55 60
   Lys Pro Gly Lys Ala Pro Lys Met Leu Ile Ile Trp Ala Ser Thr Arg
   65 70 75 80
   Val Ser Gly Val Pro Ser Arg Phe Ser Gly Ser Gly Ser Gly Thr Asp
   85 90 95
   Phe Thr Leu Thr Ile Ser Ser Leu Gln Pro Glu Asp Phe Ala Thr Tyr
   100 105 110
   Tyr Cys Gln Gln Ser Tyr Ser Arg Pro Tyr Thr Phe Gly Gln Gly Thr
   115 120 125
   Lys Val Glu Ile Lys Arg Thr Val Ala Ala Pro Ser Val Phe Ile Phe
   130 135 140
   Pro Pro Ser Asp Glu Gln Leu Lys Ser Gly Thr Ala Ser Val Val Cys
   145 150 155 160
   Leu Leu Asn Asn Phe Tyr Pro Arg Glu Ala Lys Val Gln Trp Lys Val
   165 170 175
   Asp Asn Ala Leu Gln Ser Gly Asn Ser Gln Glu Ser Val Thr Glu Gln
   180 185 190
   Asp Ser Lys Asp Ser Thr Tyr Ser Leu Ser Ser Thr Leu Thr Leu Ser
   195 200 205
   Lys Ala Asp Tyr Glu Lys His Lys Val Tyr Ala Cys Glu Val Thr His
   210 215 220
   Gln Gly Leu Ser Ser Pro Val Thr Lys Ser Phe Asn Arg Gly Glu Cys
   225 230 235 240
<210> 71
   <211> 19
   <212> PRT
   <213> Artificial Sequence
<220>
   <223> epitope in SEMA domain of c-Met
<400> 71
   Phe Ser Pro Gln Ile Glu Glu Pro Ser Gln Cys Pro Asp Cys Val Val
   1 5 10 15
   Ser Ala Leu
<210> 72
   <211> 10
   <212> PRT
   <213> Artificial Sequence
<220>
   <223> epitope in SEMA domain of c-Met
<400> 72
   Pro Gln Ile Glu Glu Pro Ser Gln Cys Pro
   1 5 10
<210> 73
   <211> 5
   <212> PRT
   <213> Artificial Sequence
<220>
   <223> epitope in SEMA domain of c-Met
<400> 73
   Glu Glu Pro Ser Gln
   1 5
<210> 74
   <211> 117
   <212> PRT
   <213> Artificial Sequence
<220>
   <223> heavy chain variable region of anti-c-Met antibody (AbF46 or huAbF46-H1)
<400> 74
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Thr Asp Tyr
               20                  25                  30
   Tyr Met Ser Trp Val Arg Gln Ala Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Asn Ser
   65                  70                  75                  80
   Leu Tyr Leu Gln Met Asn Ser Leu Lys Thr Glu Asp Thr Ala Val Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser
           115
<210> 75
   <211> 114
   <212> PRT
   <213> Artificial Sequence
<220>
   <223> light chain variable region of anti-c-Met antibody (AbF46 or huAbF46-H1)
<400> 75
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Leu Leu Ala Ser
               20                  25                  30
   Gly Asn Gln Asn Asn Tyr Leu Ala Trp His Gln Gln Lys Pro Gly Gln
           35                  40                  45
   Pro Pro Lys Met Leu Ile Ile Trp Ala Ser Thr Arg Val Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Ser Tyr Ser Ala Pro Leu Thr Phe Gly Gly Gly Thr Lys Val Glu Ile
               100                 105                 110
   Lys Arg
<210> 76
   <211> 1416
   <212> DNA
   <213> Artificial Sequence
<220>
   <223> nucleotide sequence of heavy chain of nti-c-Met antibody (AbF46 or huAbF46-H1)
<220>
   <221> misc_feature
   <222> (1)..(6)
   <223> EcoRI restriction site
<220>
   <221> misc_feature
   <222> (7)..(66)
   <223> signal sequence
<220>
   <221> misc_feature
   <222> (67)..(417)
   <223> VH - heavy chain variable region
<220>
   <221> misc_feature
   <222> (418)..(423)
   <223> NdeI restriction site
<220>
   <221> misc_feature
   <222> (418)..(1407)
   <223> CH - heavy chain constant region
<220>
   <221> misc_feature
   <222> (1408)..(1410)
   <223> TGA - stop sodon
<220>
   <221> misc_feature
   <222> (1411)..(1416)
   <223> XhoI restriction site
<400> 76
   gaattcgccg ccaccatgga atggagctgg gtttttctcg taacactttt aaatggtatc 60
   cagtgtgagg tgaagctggt ggagtctgga ggaggcttgg tacagcctgg gggttctctg 120
   agactctcct gtgcaacttc tgggttcacc ttcactgatt actacatgag ctgggtccgc 180
   cagcctccag gaaaggcact tgagtggttg ggttttatta gaaacaaagc taatggttac 240
   acaacagagt acagtgcatc tgtgaagggt cggttcacca tctccagaga taattcccaa 300
   agcatcctct atcttcaaat ggacaccctg agagctgagg acagtgccac ttattactgt 360
   gcaagagata actggtttgc ttactggggc caagggactc tggtcactgt ctctgcagct 420
   agcaccaagg gcccatcggt cttccccctg gcaccctcct ccaagagcac ctctgggggc 480
   acagcggccc tgggctgcct ggtcaaggac tacttccccg aaccggtgac ggtgtcgtgg 540
   aactcaggcg ccctgaccag cggcgtgcac accttcccgg ctgtcctaca gtcctcagga 600
   ctctactccc tcagcagcgt ggtgaccgtg ccctccagca gcttgggcac ccagacctac 660
   atctgcaacg tgaatcacaa gcccagcaac accaaggtgg acaagaaagt tgagcccaaa 720
   tcttgtgaca aaactcacac atgcccaccg tgcccagcac ctgaactcct ggggggaccg 780
   tcagtcttcc tcttcccccc aaaacccaag gacaccctca tgatctcccg gacccctgag 840
   gtcacatgcg tggtggtgga cgtgagccac gaagaccctg aggtcaagtt caactggtac 900
   gtggacggcg tggaggtgca taatgccaag acaaagccgc gggaggagca gtacaacagc 960
   acgtaccgtg tggtcagcgt cctcaccgtc ctgcaccagg actggctgaa tggcaaggag 1020
   tacaagtgca aggtctccaa caaagccctc ccagccccca tcgagaaaac catctccaaa 1080
   gccaaagggc agccccgaga accacaggtg tacaccctgc ccccatcccg ggaggagatg 1140
   accaagaacc aggtcagcct gacctgcctg gtcaaaggct tctatcccag cgacatcgcc 1200
   gtggagtggg agagcaatgg gcagccggag aacaactaca agaccacgcc tcccgtgctg 1260
   gactccgacg gctccttctt cctctacagc aagctcaccg tggacaagag caggtggcag 1320
   caggggaacg tcttctcatg ctccgtgatg catgaggctc tgcacaacca ctacacgcag 1380
   aagagcctct ccctgtctcc gggtaaatga ctcgag 1416
<210> 77
   <211> 759
   <212> DNA
   <213> Artificial Sequence
<220>
   <223> nucleotide sequence of light chain of anti-c-Met antibody (AbF46 or huAbF46-H1)
<220>
   <221> misc_difference
   <222> (1)..(6)
   <223> EcoRI restriction site
<220>
   <221> misc_difference
   <222> (7)..(90)
   <223> signal sequence
<220>
   <221> misc_difference
   <222> (91)..(432)
   <223> VL - light chain variable region
<220>
   <221> misc_difference
   <222> (430)..(435)
   <223> BsiWI restriction site
<220>
   <221> misc_difference
   <222> (433)..(750)
   <223> CL - light chain constant region
<220>
   <221> misc_difference
   <222> (751)..(753)
   <223> stop codon
<220>
   <221> misc_difference
   <222> (754)..(759)
   <223> XhoI restriction site
<400> 77
   gaattcacta gtgattaatt cgccgccacc atggattcac aggcccaggt cctcatgttg 60
   ctgctgctat cggtatctgg tacctgtgga gacattttga tgacccagtc tccatcctcc 120
   ctgactgtgt cagcaggaga gaaggtcact atgagctgca agtccagtca gagtctttta 180
   gctagtggca accaaaataa ctacttggcc tggcaccagc agaaaccagg acgatctcct 240
   aaaatgctga taatttgggc atccactagg gtatctggag tccctgatcg cttcataggc 300
   agtggatctg ggacggattt cactctgacc atcaacagtg tgcaggctga agatctggct 360
   gtttattact gtcagcagtc ctacagcgct ccgctcacgt tcggtgctgg gaccaagctg 420
   gagctgaaac gtacggtggc tgcaccatct gtcttcatct tcccgccatc tgatgagcag 480
   ttgaaatctg gaactgcctc tgttgtgtgc ctgctgaata acttctatcc cagagaggcc 540
   aaagtacagt ggaaggtgga taacgccctc caatcgggta actcccagga gagtgtcaca 600
   gagcaggaca gcaaggacag cacctacagc ctcagcagca ccctgacgct gagcaaagca 660
   gactacgaga aacacaaagt ctacgcctgc gaagtcaccc atcagggcct gagctcgccc 720
   gtcacaaaga gcttcaacag gggagagtgt tgactcgag 759
<210> 78
   <211> 4170
   <212> DNA
   <213> Artificial Sequence
<220>
   <223> polynucleotide encoding c-Met protein
<400> 78
   atgaaggccc ccgctgtgct tgcacctggc atcctcgtgc tcctgtttac cttggtgcag 60
   aggagcaatg gggagtgtaa agaggcacta gcaaagtccg agatgaatgt gaatatgaag 120
   tatcagcttc ccaacttcac cgcggaaaca cccatccaga atgtcattct acatgagcat 180
   cacattttcc ttggtgccac taactacatt tatgttttaa atgaggaaga ccttcagaag 240
   gttgctgagt acaagactgg gcctgtgctg gaacacccag attgtttccc atgtcaggac 300
   tgcagcagca aagccaattt atcaggaggt gtttggaaag ataacatcaa catggctcta 360
   gttgtcgaca cctactatga tgatcaactc attagctgtg gcagcgtcaa cagagggacc 420
   tgccagcgac atgtctttcc ccacaatcat actgctgaca tacagtcgga ggttcactgc 480
   atattctccc cacagataga agagcccagc cagtgtcctg actgtgtggt gagcgccctg 540
   ggagccaaag tcctttcatc tgtaaaggac cggttcatca acttctttgt aggcaatacc 600
   ataaattctt cttatttccc agatcatcca ttgcattcga tatcagtgag aaggctaaag 660
   gaaacgaaag atggttttat gtttttgacg gaccagtcct acattgatgt tttacctgag 720
   ttcagagatt cttaccccat taagtatgtc catgcctttg aaagcaacaa ttttatttac 780
   ttcttgacgg tccaaaggga aactctagat gctcagactt ttcacacaag aataatcagg 840
   ttctgttcca taaactctgg attgcattcc tacatggaaa tgcctctgga gtgtattctc 900
   acagaaaaga gaaaaaagag atccacaaag aaggaagtgt ttaatatact tcaggctgcg 960
   tatgtcagca agcctggggc ccagcttgct agacaaatag gagccagcct gaatgatgac 1020
   attcttttcg gggtgttcgc acaaagcaag ccagattctg ccgaaccaat ggatcgatct 1080
   gccatgtgtg cattccctat caaatatgtc aacgacttct tcaacaagat cgtcaacaaa 1140
   aacaatgtga gatgtctcca gcatttttac ggacccaatc atgagcactg ctttaatagg 1200
   acacttctga gaaattcatc aggctgtgaa gcgcgccgtg atgaatatcg aacagagttt 1260
   accacagctt tgcagcgcgt tgacttattc atgggtcaat tcagcgaagt cctcttaaca 1320
   tctatatcca ccttcattaa aggagacctc accatagcta atcttgggac atcagagggt 1380
   cgcttcatgc aggttgtggt ttctcgatca ggaccatcaa cccctcatgt gaattttctc 1440
   ctggactccc atccagtgtc tccagaagtg attgtggagc atacattaaa ccaaaatggc 1500
   tacacactgg ttatcactgg gaagaagatc acgaagatcc cattgaatgg cttgggctgc 1560
   agacatttcc agtcctgcag tcaatgcctc tctgccccac cctttgttca gtgtggctgg 1620
   tgccacgaca aatgtgtgcg atcggaggaa tgcctgagcg ggacatggac tcaacagatc 1680
   tgtctgcctg caatctacaa ggttttccca aatagtgcac cccttgaagg agggacaagg 1740
   ctgaccatat gtggctggga ctttggattt cggaggaata ataaatttga tttaaagaaa 1800
   actagagttc tccttggaaa tgagagctgc accttgactt taagtgagag cacgatgaat 1860
   acattgaaat gcacagttgg tcctgccatg aataagcatt tcaatatgtc cataattatt 1920
   tcaaatggcc acgggacaac acaatacagt acattctcct atgtggatcc tgtaataaca 1980
   agtatttcgc cgaaatacgg tcctatggct ggtggcactt tacttacttt aactggaaat 2040
   tacctaaaca gtgggaattc tagacacatt tcaattggtg gaaaaacatg tactttaaaa 2100
   agtgtgtcaa acagtattct tgaatgttat accccagccc aaaccatttc aactgagttt 2160
   gctgttaaat tgaaaattga cttagccaac cgagagacaa gcatcttcag ttaccgtgaa 2220
   gatcccattg tctatgaaat tcatccaacc aaatctttta ttagtggtgg gagcacaata 2280
   acaggtgttg ggaaaaacct gaattcagtt agtgtcccga gaatggtcat aaatgtgcat 2340
   gaagcaggaa ggaactttac agtggcatgt caacatcgct ctaattcaga gataatctgt 2400
   tgtaccactc cttccctgca acagctgaat ctgcaactcc ccctgaaaac caaagccttt 2460
   ttcatgttag atgggatcct ttccaaatac tttgatctca tttatgtaca taatcctgtg 2520
   tttaagcctt ttgaaaagcc agtgatgatc tcaatgggca atgaaaatgt actggaaatt 2580
   aagggaaatg atattgaccc tgaagcagtt aaaggtgaag tgttaaaagt tggaaataag 2640
   agctgtgaga atatacactt acattctgaa gccgttttat gcacggtccc caatgacctg 2700
   ctgaaattga acagcgagct aaatatagag tggaagcaag caatttcttc aaccgtcctt 2760
   ggaaaagtaa tagttcaacc agatcagaat ttcacaggat tgattgctgg tgttgtctca 2820
   atatcaacag cactgttatt actacttggg tttttcctgt ggctgaaaaa gagaaagcaa 2880
   attaaagatc tgggcagtga attagttcgc tacgatgcaa gagtacacac tcctcatttg 2940
   gataggcttg taagtgcccg aagtgtaagc ccaactacag aaatggtttc aaatgaatct 3000
   gtagactacc gagctacttt tccagaagat cagtttccta attcatctca gaacggttca 3060
   tgccgacaag tgcagtatcc tctgacagac atgtccccca tcctaactag tggggactct 3120
   gatatatcca gtccattact gcaaaatact gtccacattg acctcagtgc tctaaatcca 3180
   gagctggtcc aggcagtgca gcatgtagtg attgggccca gtagcctgat tgtgcatttc 3240
   aatgaagtca taggaagagg gcattttggt tgtgtatatc atgggacttt gttggacaat 3300
   gatggcaaga aaattcactg tgctgtgaaa tccttgaaca gaatcactga cataggagaa 3360
   gtttcccaat ttctgaccga gggaatcatc atgaaagatt ttagtcatcc caatgtcctc 3420
   tcgctcctgg gaatctgcct gcgaagtgaa gggtctccgc tggtggtcct accatacatg 3480
   aaacatggag atcttcgaaa tttcattcga aatgagactc ataatccaac tgtaaaagat 3540
   cttattggct ttggtcttca agtagccaaa ggcatgaaat atcttgcaag caaaaagttt 3600
   gtccacagag acttggctgc aagaaactgt atgctggatg aaaaattcac agtcaaggtt 3660
   gctgattttg gtcttgccag agacatgtat gataaagaat actatagtgt acacaacaaa 3720
   acaggtgcaa agctgccagt gaagtggatg gctttggaaa gtctgcaaac tcaaaagttt 3780
   accaccaagt cagatgtgtg gtcctttggc gtgctcctct gggagctgat gacaagagga      3840
   gccccacctt atcctgacgt aaacaccttt gatataactg tttacttgtt gcaagggaga      3900
   agactcctac aacccgaata ctgcccagac cccttatatg aagtaatgct aaaatgctgg      3960
   caccctaaag ccgaaatgcg cccatccttt tctgaactgg tgtcccggat atcagcgatc      4020
   ttctctactt tcattgggga gcactatgtc catgtgaacg ctacttatgt gaacgtaaaa      4080
   tgtgtcgctc cgtatccttc tctgttgtca tcagaagata acgctgatga tgaggtggac      4140
   acacgaccag cctccttctg ggagacatca                                       4170
<210> 79
   <211> 444
   <212> PRT
   <213> Artificial Sequence
<220>
   <223> SEMA domain of c-Met
<400> 79
   Leu His Glu His His Ile Phe Leu Gly Ala Thr Asn Tyr Ile Tyr Val
    1               5                  10                  15
   Leu Asn Glu Glu Asp Leu Gln Lys Val Ala Glu Tyr Lys Thr Gly Pro
               20                  25                  30
   Val Leu Glu His Pro Asp Cys Phe Pro Cys Gln Asp Cys Ser Ser Lys
           35                  40                  45
   Ala Asn Leu Ser Gly Gly Val Trp Lys Asp Asn Ile Asn Met Ala Leu
       50                  55                  60
   Val Val Asp Thr Tyr Tyr Asp Asp Gln Leu Ile Ser Cys Gly Ser Val
   65                  70                  75                  80
   Asn Arg Gly Thr Cys Gln Arg His Val Phe Pro His Asn His Thr Ala
                   85                  90                  95
   Asp Ile Gln Ser Glu Val His Cys Ile Phe Ser Pro Gln Ile Glu Glu
               100                 105                 110
   Pro Ser Gln Cys Pro Asp Cys Val Val Ser Ala Leu Gly Ala Lys Val
           115                 120                 125
   Leu Ser Ser Val Lys Asp Arg Phe Ile Asn Phe Phe Val Gly Asn Thr
       130                 135                 140
   Ile Asn Ser Ser Tyr Phe Pro Asp His Pro Leu His Ser Ile Ser Val
   145                 150                 155                 160
   Arg Arg Leu Lys Glu Thr Lys Asp Gly Phe Met Phe Leu Thr Asp Gln
                   165                 170                 175
   Ser Tyr Ile Asp Val Leu Pro Glu Phe Arg Asp Ser Tyr Pro Ile Lys
               180                 185                 190
   Tyr Val His Ala Phe Glu Ser Asn Asn Phe Ile Tyr Phe Leu Thr Val
           195                 200                 205
   Gln Arg Glu Thr Leu Asp Ala Gln Thr Phe His Thr Arg Ile Ile Arg
       210                 215                 220
   Phe Cys Ser Ile Asn Ser Gly Leu His Ser Tyr Met Glu Met Pro Leu
   225                 230                 235                 240
   Glu Cys Ile Leu Thr Glu Lys Arg Lys Lys Arg Ser Thr Lys Lys Glu
                   245                 250                 255
   Val Phe Asn Ile Leu Gln Ala Ala Tyr Val Ser Lys Pro Gly Ala Gln
               260                 265                 270
   Leu Ala Arg Gln Ile Gly Ala Ser Leu Asn Asp Asp Ile Leu Phe Gly
           275                 280                 285
   Val Phe Ala Gln Ser Lys Pro Asp Ser Ala Glu Pro Met Asp Arg Ser
       290                 295                 300
   Ala Met Cys Ala Phe Pro Ile Lys Tyr Val Asn Asp Phe Phe Asn Lys
   305                 310                 315                 320
   Ile Val Asn Lys Asn Asn Val Arg Cys Leu Gln His Phe Tyr Gly Pro
                   325                 330                 335
   Asn His Glu His Cys Phe Asn Arg Thr Leu Leu Arg Asn Ser Ser Gly
               340                 345                 350
   Cys Glu Ala Arg Arg Asp Glu Tyr Arg Thr Glu Phe Thr Thr Ala Leu
           355                 360                 365
   Gln Arg Val Asp Leu Phe Met Gly Gln Phe Ser Glu Val Leu Leu Thr
       370                 375                 380
   Ser Ile Ser Thr Phe Ile Lys Gly Asp Leu Thr Ile Ala Asn Leu Gly
   385                 390                 395                 400
   Thr Ser Glu Gly Arg Phe Met Gln Val Val Val Ser Arg Ser Gly Pro
                   405                 410                 415
   Ser Thr Pro His Val Asn Phe Leu Leu Asp Ser His Pro Val Ser Pro
               420                 425                 430
   Glu Val Ile Val Glu His Thr Leu Asn Gln Asn Gly
           435                 440
<210> 80
   <211> 451
   <212> PRT
   <213> Artificial Sequence
<220>
   <223> PSI-IPT domain of c-Met
<400> 80
   Tyr Thr Leu Val Ile Thr Gly Lys Lys Ile Thr Lys Ile Pro Leu Asn
   1               5                   10                  15
   Gly Leu Gly Cys Arg His Phe Gln Ser Cys Ser Gln Cys Leu Ser Ala
               20                  25                  30
   Pro Pro Phe Val Gln Cys Gly Trp Cys His Asp Lys Cys Val Arg Ser
           35                  40                  45
   Glu Glu Cys Leu Ser Gly Thr Trp Thr Gln Gln Ile Cys Leu Pro Ala
       50                  55                  60
   Ile Tyr Lys Val Phe Pro Asn Ser Ala Pro Leu Glu Gly Gly Thr Arg
   65                  70                  75                  80
   Leu Thr Ile Cys Gly Trp Asp Phe Gly Phe Arg Arg Asn Asn Lys Phe
                   85                  90                  95
   Asp Leu Lys Lys Thr Arg Val Leu Leu Gly Asn Glu Ser Cys Thr Leu
               100                 105                 110
   Thr Leu Ser Glu Ser Thr Met Asn Thr Leu Lys Cys Thr Val Gly Pro
           115                 120                 125
   Ala Met Asn Lys His Phe Asn Met Ser Ile Ile Ile Ser Asn Gly His
       130                 135                 140
   Gly Thr Thr Gln Tyr Ser Thr Phe Ser Tyr Val Asp Pro Val Ile Thr
   145                 150                 155                 160
   Ser Ile Ser Pro Lys Tyr Gly Pro Met Ala Gly Gly Thr Leu Leu Thr
                   165                 170                 175
   Leu Thr Gly Asn Tyr Leu Asn Ser Gly Asn Ser Arg His Ile Ser Ile
               180                 185                 190
   Gly Gly Lys Thr Cys Thr Leu Lys Ser Val Ser Asn Ser Ile Leu Glu
           195                 200                 205
   Cys Tyr Thr Pro Ala Gln Thr Ile Ser Thr Glu Phe Ala Val Lys Leu
       210                 215                 220
   Lys Ile Asp Leu Ala Asn Arg Glu Thr Ser Ile Phe Ser Tyr Arg Glu
   225                 230                 235                 240
   Asp Pro Ile Val Tyr Glu Ile His Pro Thr Lys Ser Phe Ile Ser Thr
                   245                 250                 255
   Trp Trp Lys Glu Pro Leu Asn Ile Val Ser Phe Leu Phe Cys Phe Ala
               260                 265                 270
   Ser Gly Gly Ser Thr Ile Thr Gly Val Gly Lys Asn Leu Asn Ser Val
           275                 280                 285
   Ser Val Pro Arg Met Val Ile Asn Val His Glu Ala Gly Arg Asn Phe
       290                 295                 300
   Thr Val Ala Cys Gln His Arg Ser Asn Ser Glu Ile Ile Cys Cys Thr
   305                 310                 315                 320
   Thr Pro Ser Leu Gln Gln Leu Asn Leu Gln Leu Pro Leu Lys Thr Lys
                   325                 330                 335
   Ala Phe Phe Met Leu Asp Gly Ile Leu Ser Lys Tyr Phe Asp Leu Ile
               340                 345                 350
   Tyr Val His Asn Pro Val Phe Lys Pro Phe Glu Lys Pro Val Met Ile
           355                 360                 365
   Ser Met Gly Asn Glu Asn Val Leu Glu Ile Lys Gly Asn Asp Ile Asp
       370                 375                 380
   Pro Glu Ala Val Lys Gly Glu Val Leu Lys Val Gly Asn Lys Ser Cys
   385                 390                 395                 400
   Glu Asn Ile His Leu His Ser Glu Ala Val Leu Cys Thr Val Pro Asn
                   405                 410                 415
   Asp Leu Leu Lys Leu Asn Ser Glu Leu Asn Ile Glu Trp Lys Gln Ala
               420                 425                 430
   Ile Ser Ser Thr Val Leu Gly Lys Val Ile Val Gln Pro Asp Gln Asn
           435                 440                 445
   Phe Thr Gly
       450
<210> 81
   <211> 313
   <212> PRT
   <213> Artificial Sequence
<220>
   <223> TyrKc domain of c-Met
<400> 81
   Val His Phe Asn Glu Val Ile Gly Arg Gly His Phe Gly Cys Val Tyr
    1               5                  10                  15
   His Gly Thr Leu Leu Asp Asn Asp Gly Lys Lys Ile His Cys Ala Val
               20                  25                  30
   Lys Ser Leu Asn Arg Ile Thr Asp Ile Gly Glu Val Ser Gln Phe Leu
           35                  40                  45
   Thr Glu Gly Ile Ile Met Lys Asp Phe Ser His Pro Asn Val Leu Ser
       50                  55                  60
   Leu Leu Gly Ile Cys Leu Arg Ser Glu Gly Ser Pro Leu Val Val Leu
   65                  70                  75                  80
   Pro Tyr Met Lys His Gly Asp Leu Arg Asn Phe Ile Arg Asn Glu Thr
                   85                  90                  95
   His Asn Pro Thr Val Lys Asp Leu Ile Gly Phe Gly Leu Gln Val Ala
               100                 105                 110
   Lys Gly Met Lys Tyr Leu Ala Ser Lys Lys Phe Val His Arg Asp Leu
           115                 120                 125
   Ala Ala Arg Asn Cys Met Leu Asp Glu Lys Phe Thr Val Lys Val Ala
       130                 135                 140
   Asp Phe Gly Leu Ala Arg Asp Met Tyr Asp Lys Glu Tyr Tyr Ser Val
   145                 150                 155                 160
   His Asn Lys Thr Gly Ala Lys Leu Pro Val Lys Trp Met Ala Leu Glu
                   165                 170                 175
   Ser Leu Gln Thr Gln Lys Phe Thr Thr Lys Ser Asp Val Trp Ser Phe
               180                 185                 190
   Gly Val Leu Leu Trp Glu Leu Met Thr Arg Gly Ala Pro Pro Tyr Pro
           195                 200                 205
   Asp Val Asn Thr Phe Asp Ile Thr Val Tyr Leu Leu Gln Gly Arg Arg
       210                 215                 220
   Leu Leu Gln Pro Glu Tyr Cys Pro Asp Pro Leu Tyr Glu Val Met Leu
   225                 230                 235                 240
   Lys Cys Trp His Pro Lys Ala Glu Met Arg Pro Ser Phe Ser Glu Leu
                   245                 250                 255
   Val Ser Arg Ile Ser Ala Ile Phe Ser Thr Phe Ile Gly Glu His Tyr
               260                 265                 270
   Val His Val Asn Ala Thr Tyr Val Asn Val Lys Cys Val Ala Pro Tyr
           275                 280                 285
   Pro Ser Leu Leu Ser Ser Glu Asp Asn Ala Asp Asp Glu Val Asp Thr
       290                 295                 300
   Arg Pro Ala Ser Phe Trp Glu Thr Ser
   305                 310
<210> 82
   <211> 1332
   <212> DNA
   <213> Artificial Sequence
<220>
   <223> polynucleotide encoding SEMA domain of c-Met
<400> 82
   ctacatgagc atcacatttt ccttggtgcc actaactaca tttatgtttt aaatgaggaa      60
   gaccttcaga aggttgctga gtacaagact gggcctgtgc tggaacaccc agattgtttc     120
   ccatgtcagg actgcagcag caaagccaat ttatcaggag gtgtttggaa agataacatc     180
   aacatggctc tagttgtcga cacctactat gatgatcaac tcattagctg tggcagcgtc     240
   aacagaggga cctgccagcg acatgtcttt ccccacaatc atactgctga catacagtcg     300
   gaggttcact gcatattctc cccacagata gaagagccca gccagtgtcc tgactgtgtg     360
   gtgagcgccc tgggagccaa agtcctttca tctgtaaagg accggttcat caacttcttt     420
   gtaggcaata ccataaattc ttcttatttc ccagatcatc cattgcattc gatatcagtg     480
   agaaggctaa aggaaacgaa agatggtttt atgtttttga cggaccagtc ctacattgat     540
   gttttacctg agttcagaga ttcttacccc attaagtatg tccatgcctt tgaaagcaac     600
   aattttattt acttcttgac ggtccaaagg gaaactctag atgctcagac ttttcacaca     660
   agaataatca ggttctgttc cataaactct ggattgcatt cctacatgga aatgcctctg     720
   gagtgtattc tcacagaaaa gagaaaaaag agatccacaa agaaggaagt gtttaatata     780
   cttcaggctg cgtatgtcag caagcctggg gcccagcttg ctagacaaat aggagccagc     840
   ctgaatgatg acattctttt cggggtgttc gcacaaagca agccagattc tgccgaacca     900
   atggatcgat ctgccatgtg tgcattccct atcaaatatg tcaacgactt cttcaacaag     960
   atcgtcaaca aaaacaatgt gagatgtctc cagcattttt acggacccaa tcatgagcac    1020
   tgctttaata ggacacttct gagaaattca tcaggctgtg aagcgcgccg tgatgaatat    1080
   cgaacagagt ttaccacagc tttgcagcgc gttgacttat tcatgggtca attcagcgaa    1140
   gtcctcttaa catctatatc caccttcatt aaaggagacc tcaccatagc taatcttggg    1200
   acatcagagg gtcgcttcat gcaggttgtg gtttctcgat caggaccatc aacccctcat    1260
   gtgaattttc tcctggactc ccatccagtg tctccagaag tgattgtgga gcatacatta    1320
   aaccaaaatg gc                                                        1332
<210> 83
   <211> 1299
   <212> DNA
   <213> Artificial Sequence
<220>
   <223> polynucleotide encoding PSI-IPT domain of c-Met
<400> 83
   tacacactgg ttatcactgg gaagaagatc acgaagatcc cattgaatgg cttgggctgc      60
   agacatttcc agtcctgcag tcaatgcctc tctgccccac cctttgttca gtgtggctgg     120
   tgccacgaca aatgtgtgcg atcggaggaa tgcctgagcg ggacatggac tcaacagatc     180
   tgtctgcctg caatctacaa ggttttccca aatagtgcac cccttgaagg agggacaagg     240
   ctgaccatat gtggctggga ctttggattt cggaggaata ataaatttga tttaaagaaa     300
   actagagttc tccttggaaa tgagagctgc accttgactt taagtgagag cacgatgaat     360
   acattgaaat gcacagttgg tcctgccatg aataagcatt tcaatatgtc cataattatt     420
   tcaaatggcc acgggacaac acaatacagt acattctcct atgtggatcc tgtaataaca     480
   agtatttcgc cgaaatacgg tcctatggct ggtggcactt tacttacttt aactggaaat     540
   tacctaaaca gtgggaattc tagacacatt tcaattggtg gaaaaacatg tactttaaaa     600
   agtgtgtcaa acagtattct tgaatgttat accccagccc aaaccatttc aactgagttt     660
   gctgttaaat tgaaaattga cttagccaac cgagagacaa gcatcttcag ttaccgtgaa     720
   gatcccattg tctatgaaat tcatccaacc aaatctttta ttagtggtgg gagcacaata     780
   acaggtgttg ggaaaaacct gaattcagtt agtgtcccga gaatggtcat aaatgtgcat     840
   gaagcaggaa ggaactttac agtggcatgt caacatcgct ctaattcaga gataatctgt     900
   tgtaccactc cttccctgca acagctgaat ctgcaactcc ccctgaaaac caaagccttt     960
   ttcatgttag atgggatcct ttccaaatac tttgatctca tttatgtaca taatcctgtg    1020
   tttaagcctt ttgaaaagcc agtgatgatc tcaatgggca atgaaaatgt actggaaatt    1080
   aagggaaatg atattgaccc tgaagcagtt aaaggtgaag tgttaaaagt tggaaataag    1140
   agctgtgaga atatacactt acattctgaa gccgttttat gcacggtccc caatgacctg    1200
   ctgaaattga acagcgagct aaatatagag tggaagcaag caatttcttc aaccgtcctt    1260
   ggaaaagtaa tagttcaacc agatcagaat ttcacagga                           1299
<210> 84
   <211> 939
   <212> DNA
   <213> Artificial Sequence
<220>
   <223> polynucleotide encoding TyrKc domain of c-Met
<400> 84
   gtgcatttca atgaagtcat aggaagaggg cattttggtt gtgtatatca tgggactttg      60
   ttggacaatg atggcaagaa aattcactgt gctgtgaaat ccttgaacag aatcactgac     120
   ataggagaag tttcccaatt tctgaccgag ggaatcatca tgaaagattt tagtcatccc     180
   aatgtcctct cgctcctggg aatctgcctg cgaagtgaag ggtctccgct ggtggtccta     240
   ccatacatga aacatggaga tcttcgaaat ttcattcgaa atgagactca taatccaact     300
   gtaaaagatc ttattggctt tggtcttcaa gtagccaaag gcatgaaata tcttgcaagc     360
   aaaaagtttg tccacagaga cttggctgca agaaactgta tgctggatga aaaattcaca     420
   gtcaaggttg ctgattttgg tcttgccaga gacatgtatg ataaagaata ctatagtgta     480
   cacaacaaaa caggtgcaaa gctgccagtg aagtggatgg ctttggaaag tctgcaaact     540
   caaaagttta ccaccaagtc agatgtgtgg tcctttggcg tgctcctctg ggagctgatg     600
   acaagaggag ccccacctta tcctgacgta aacacctttg atataactgt ttacttgttg     660
   caagggagaa gactcctaca acccgaatac tgcccagacc ccttatatga agtaatgcta     720
   aaatgctggc accctaaagc cgaaatgcgc ccatcctttt ctgaactggt gtcccggata     780
   tcagcgatct tctctacttt cattggggag cactatgtcc atgtgaacgc tacttatgtg     840
   aacgtaaaat gtgtcgctcc gtatccttct ctgttgtcat cagaagataa cgctgatgat     900
   gaggtggaca cacgaccagc ctccttctgg gagacatca                            939
<210> 85
   <211> 13
   <212> PRT
   <213> Artificial Sequence
<220>
   <223> heavy chain CDR3 of anti-c-Met antibody
<400> 85
   Asp Asn Trp Phe Ala Tyr Trp Gly Gln Gly Thr Leu Val
   1               5                   10
<210> 86
   <211> 10
   <212> PRT
   <213> Artificial Sequence
<220>
   <223> light chain CDR3 of anti-c-Met antibody
<400> 86
   Leu Thr Phe Gly Ala Gly Thr Lys Leu Glu
   1               5                   10
<210> 87
   <211> 117
   <212> PRT
   <213> Artificial Sequence
<220>
   <223> heavy chain variable region of monoclonal antibody AbF46
<400> 87
   Glu Val Lys Leu Val Glu Ser Gly Gly Gly Leu Val Gln Pro Gly Gly
   1               5                   10                  15
   Ser Leu Arg Leu Ser Cys Ala Thr Ser Gly Phe Thr Phe Thr Asp Tyr
               20                  25                  30
   Tyr Met Ser Trp Val Arg Gln Pro Pro Gly Lys Ala Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Gln Ser Ile
   65                  70                  75                  80
   Leu Tyr Leu Gln Met Asp Thr Leu Arg Ala Glu Asp Ser Ala Thr Tyr
                   85                  90                  95
   Tyr Cys Ala Arg Asp Asn Trp Phe Ala Tyr Trp Gly Gln Gly Thr Leu
               100                 105                 110
   Val Thr Val Ser Ala
           115
<210> 88
   <211> 114
   <212> PRT
   <213> Artificial Sequence
<220>
   <223> light chain variable region of anti-c-Met antibody
<400> 88
   Asp Ile Leu Met Thr Gln Ser Pro Ser Ser Leu Thr Val Ser Ala Gly
   1 5 10 15
   Glu Lys Val Thr Met Ser Cys Lys Ser Ser Gln Ser Leu Leu Ala Ser
   20 25 30
   Gly Asn Gln Asn Asn Tyr Leu Ala Trp His Gln Gln Lys Pro Gly Arg
   35 40 45
   Ser Pro Lys Met Leu Ile Ile Trp Ala Ser Thr Arg Val Ser Gly Val
   50 55 60
   Pro Asp Arg Phe Ile Gly Ser Gly Ser Gly Thr Asp Phe Thr Leu Thr
   65 70 75 80
   Ile Asn Ser Val Gln Ala Glu Asp Leu Ala Val Tyr Tyr Cys Gln Gln
   85 90 95
   Ser Tyr Ser Ala Pro Leu Thr Phe Gly Ala Gly Thr Lys Leu Glu Leu
   100 105 110
   Lys Arg
<210> 89
   <211> 17
   <212> PRT
   <213> Artificial Sequence
<220>
   <223> light chain CDR3 of anti-c-Met antibody
<400> 89
   Gln Gln Ser Tyr Ser Ala Pro Leu Thr Phe Gly Ala Gly Thr Lys Leu
   1 5 10 15
   Glu
<210> 90
   <211> 117
   <212> PRT
   <213> Artificial Sequence
<220>
   <223> heavy chain variable region of AT-VH1
<400> 90
   Glu Val Lys Leu Val Glu Ser Gly Gly Gly Leu Val Gln Pro Gly Gly
   1 5 10 15
   Ser Leu Arg Leu Ser Cys Ala Thr Ser Gly Phe Thr Phe Thr Asp Tyr
   20 25 30
   Tyr Met Ser Trp Val Arg Gln Pro Pro Gly Lys Gly Leu Glu Trp Leu
   35 40 45
   Gly Phe Ile Arg Asn Lys Ala Asn Gly Tyr Thr Thr Glu Tyr Ser Ala
   50 55 60
   Ser Val Lys Gly Arg Phe Thr Ile Ser Arg Asp Asn Ser Lys Ser Thr
   65 70 75 80
   Leu Tyr Leu Gln Met Asn Ser Leu Arg Ala Glu Asp Ser Ala Thr Tyr
   85 90 95
   Tyr Cys Ala Arg Asp Asn Trp Phe Ala Tyr Trp Gly Gln Gly Thr Leu
   100 105 110
   Val Thr Val Ser Ser
   115
<210> 91
   <211> 117
   <212> PRT
   <213> Artificial Sequence
<220>
   <223> heavy chain variable region of AT-VH2
<400> 91
   Glu Val Lys Leu Val Glu Ser Gly Gly Gly Leu Val Gln Pro Gly Gly
   1 5 10 15
   Ser Leu Arg Leu Ser Cys Ala Thr Ser Gly Phe Thr Phe Thr Asp Tyr
   20 25 30
   Tyr Met Ser Trp Val Arg Gln Pro Pro Gly Lys Gly Leu Glu Trp Leu
   35 40 45
   Gly Phe Ile Arg Asn Lys Ala Asn Gly Tyr Thr Thr Glu Tyr Ser Ala
   50 55 60
   Ser Val Lys Gly Arg Phe Thr Ile Ser Arg Asp Asn Ser Lys Ser Thr
   65 70 75 80
   Leu Tyr Leu Gln Met Asn Ser Leu Arg Ala Glu Asp Thr Ala Thr Tyr
   85 90 95
   Tyr Cys Ala Arg Asp Asn Trp Phe Ala Tyr Trp Gly Gln Gly Thr Leu
   100 105 110
   Val Thr Val Ser Ser
   115
<210> 92
   <211> 117
   <212> PRT
   <213> Artificial Sequence
<220>
   <223> heavy chain variable region of AT-VH3
<400> 92
   Glu Val Gln Leu Val Glu Ser Gly Gly Gly Leu Val Gln Pro Gly Gly
   1               5                   10                  15
   Ser Leu Arg Leu Ser Cys Ala Thr Ser Gly Phe Thr Phe Thr Asp Tyr
               20                  25                  30
   Tyr Met Ser Trp Val Arg Gln Pro Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Ser Thr
   65                  70                  75                  80
   Leu Tyr Leu Gln Met Asn Ser Leu Arg Ala Glu Asp Thr Ala Thr Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser
           115
<210> 93
   <211> 117
   <212> PRT
   <213> Artificial Sequence
<220>
   <223> heavy chain variable region of AT-VH4
<400> 93
   Glu Val Gln Leu Val Glu Ser Gly Gly Gly Leu Val Gln Pro Gly Gly
   1               5                   10                  15
   Ser Leu Arg Leu Ser Cys Ala Thr Ser Gly Phe Thr Phe Thr Asp Tyr
               20                  25                  30
   Tyr Met Ser Trp Val Arg Gln Pro Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Thr Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser
           115
<210> 94
   <211> 117
   <212> PRT
   <213> Artificial Sequence
<220>
   <223> heavy chain variable region of AT-VH5
<400> 94
   Glu Val Gln Leu Val Glu Ser Gly Gly Gly Leu Val Gln Pro Gly Gly
   1               5                   10                  15
   Ser Leu Arg Leu Ser Cys Ala Thr Ser Gly Phe Thr Phe Thr Asp Tyr
               20                  25                  30
   Tyr Met Ser Trp Val Arg Gln Pro Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser
           115
<210> 95
   <211> 114
   <212> PRT
   <213> Artificial Sequence
<220>
   <223> light chain variable region of anti c-Met humanized antibody(huAbF46-H4)
<400> 95
   Asp Ile Gln Met Thr Gln Ser Pro Ser Ser Leu Ser Ala Ser Val Gly
   1 5 10 15
   Asp Arg Val Thr Ile Thr Cys Lys Ser Ser Gln Ser Leu Leu Ala Ser
   20 25 30
   Gly Asn Gln Asn Asn Tyr Leu Ala Trp His Gln Gln Lys Pro Gly Lys
   35 40 45
   Ala Pro Lys Met Leu Ile Ile Trp Ala Ser Thr Arg Val Ser Gly Val
   50 55 60
   Pro Ser Arg Phe Ser Gly Ser Gly Ser Gly Thr Asp Phe Thr Leu Thr
   65 70 75 80
   Ile Ser Ser Leu Gln Pro Glu Asp Phe Ala Thr Tyr Tyr Cys Gln Gln
   85 90 95
   Ser Tyr Ser Ala Pro Leu Thr Phe Gly Gln Gly Thr Lys Val Glu Ile
   100 105 110
   Lys Arg
<210> 96
   <211> 113
   <212> PRT
   <213> Artificial Sequence
<220>
   <223> light chain variable region of AT-Vk1
<400> 96
   Asp Ile Leu Met Thr Gln Ser Pro Ser Ser Leu Thr Ala Ser Val Gly
   1 5 10 15
   Asp Arg Val Thr Met Thr Cys Lys Ser Ser Gln Ser Leu Leu Ala Ser
   20 25 30
   Gly Asn Gln Asn Asn Tyr Leu Ala Trp His Gln Gln Lys Pro Gly Lys
   35 40 45
   Ala Pro Lys Met Leu Ile Ile Trp Ala Ser Thr Arg Val Ser Gly Val
   50 55 60
   Pro Asp Arg Phe Ile Gly Ser Gly Ser Gly Thr Asp Phe Thr Leu Thr
   65 70 75 80
   Ile Ser Ser Leu Gln Ala Glu Asp Val Ala Val Tyr Tyr Cys Gln Gln
   85 90 95
   Ser Tyr Ser Ala Pro Leu Thr Phe Gly Gln Gly Thr Lys Leu Glu Ile
   100 105 110
   Lys
<210> 97
   <211> 113
   <212> PRT
   <213> Artificial Sequence
<220>
   <223> light chain variable region of AT-Vk2
<400> 97
   Asp Ile Leu Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Asp Arg Phe Ile Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Ser Tyr Ser Ala Pro Leu Thr Phe Gly Gln Gly Thr Lys Leu Glu Ile
               100                 105                 110
   Lys
<210> 98
   <211> 113
   <212> PRT
   <213> Artificial Sequence
<220>
   <223> light chain variable region of AT-Vk3
<400> 98
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Asp Arg Phe Ile Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Ser Tyr Ser Ala Pro Leu Thr Phe Gly Gln Gly Thr Lys Leu Glu Ile
               100                 105                 110
   Lys
<210> 99
   <211> 113
   <212> PRT
   <213> Artificial Sequence
<220>
   <223> light chain variable region of AT-Vk4
<400> 99
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Ser Tyr Ser Ala Pro Leu Thr Phe Gly Gln Gly Thr Lys Leu Glu Ile
               100                 105                 110
   Lys
<210> 100
   <211> 13
   <212> PRT
   <213> Artificial Sequence
<220>
   <223> modified hinge region(U7-HC6)
<400> 100
   Glu Pro Ser Cys Asp Lys His Cys Cys Pro Pro Cys Pro
   1               5                   10
<210> 101
   <211> 13
   <212> PRT
   <213> Artificial Sequence
<220>
   <223> modified hinge region(U6-HC7)
<400> 101
   Glu Pro Lys Ser Cys Asp Cys His Cys Pro Pro Cys Pro
   1               5                   10
<210> 102
   <211> 12
   <212> PRT
   <213> Artificial Sequence
<220>
   <223> modified hinge region(U3-HC9)
<400> 102
   Glu Arg Lys Cys Cys Val Glu Cys Pro Pro Cys Pro
   1               5                   10
<210> 103
   <211> 14
   <212> PRT
   <213> Artificial Sequence
<220>
   <223> modified hinge region(U6-HC8)
<400> 103
   Glu Pro Arg Asp Cys Gly Cys Lys Pro Cys Pro Pro Cys Pro
   1               5                   10
<210> 104
   <211> 13
   <212> PRT
   <213> Artificial Sequence
<220>
   <223> modified hinge region(U8-HC5)
<400> 104
   Glu Lys Cys Asp Lys Thr His Thr Cys Pro Pro Cys Pro
   1 5 10
<210> 105
   <211> 15
   <212> PRT
   <213> Artificial Sequence
<220>
   <223> human hinge region
<400> 105
   Glu Pro Lys Ser Cys Asp Lys Thr His Thr Cys Pro Pro Cys Pro
   1 5 10 15
<210> 106
   <211> 17
   <212> PRT
   <213> Artificial Sequence
<220>
   <223> CDR-L1 of antibody L3-11Y
<400> 106
   Lys Ser Ser Gln Ser Leu Leu Ala Trp Gly Asn Gln Asn Asn Tyr Leu
   1 5 10 15
   Ala
<210> 107
   <211> 114
   <212> PRT
   <213> Artificial Sequence
<220>
   <223> amino acid sequence of light chain variable region of antibody L3-11Y
<400> 107
   Asp Ile Gln Met Thr Gln Ser Pro Ser Ser Leu Ser Ala Ser Val Gly
   1 5 10 15
   Asp Arg Val Thr Ile Thr Cys Lys Ser Ser Gln Ser Leu Leu Ala Trp
   20 25 30
   Gly Asn Gln Asn Asn Tyr Leu Ala Trp Tyr Gln Gln Lys Pro Gly Lys
   35 40 45
   Ala Pro Lys Met Leu Ile Ile Trp Ala Ser Thr Arg Val Ser Gly Val
   50 55 60
   Pro Ser Arg Phe Ser Gly Ser Gly Ser Gly Thr Asp Phe Thr Leu Thr
   65 70 75 80
   Ile Ser Ser Leu Gln Pro Glu Asp Phe Ala Thr Tyr Tyr Cys Gln Gln
   85 90 95
   Ser Tyr Ser Arg Pro Tyr Thr Phe Gly Gln Gly Thr Lys Val Glu Ile
   100 105 110
   Lys Arg
<210> 108
   <211> 220
   <212> PRT
   <213> Artificial Sequence
<220>
   <223> amino acid sequence of light chain of antibody L3-11Y
<400> 108
   Asp Ile Gln Met Thr Gln Ser Pro Ser Ser Leu Ser Ala Ser Val Gly
   1 5 10 15
   Asp Arg Val Thr Ile Thr Cys Lys Ser Ser Gln Ser Leu Leu Ala Trp
   20 25 30
   Gly Asn Gln Asn Asn Tyr Leu Ala Trp Tyr Gln Gln Lys Pro Gly Lys
   35 40 45
   Ala Pro Lys Met Leu Ile Ile Trp Ala Ser Thr Arg Val Ser Gly Val
   50 55 60
   Pro Ser Arg Phe Ser Gly Ser Gly Ser Gly Thr Asp Phe Thr Leu Thr
   65 70 75 80
   Ile Ser Ser Leu Gln Pro Glu Asp Phe Ala Thr Tyr Tyr Cys Gln Gln
   85 90 95
   Ser Tyr Ser Arg Pro Tyr Thr Phe Gly Gln Gly Thr Lys Val Glu Ile
   100 105 110
   Lys Arg Thr Val Ala Ala Pro Ser Val Phe Ile Phe Pro Pro Ser Asp
   115 120 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 109
   <211> 5
   <212> PRT
   <213> Artificial Sequence
<220>
   <223> CDR-H1 of anti-idiotype antibody against anti-c-Met antibody
<220>
   <221> MOD_RES
   <222> (1)
   <223> Xaa is Asp(D), Ans(N), or Gly(G)
<220>
   <221> MOD_RES
   <222> (3)
   <223> Xaa is Tyr(Y), Ala(A), Asp (D), or Ser(S)
<400> 109
   Xaa Tyr Xaa Met Ser
   1               5
<210> 110
   <211> 5
   <212> PRT
   <213> Artificial Sequence
<220>
   <223> CDR-H1 of anti-idiotype antibody against anti-c-Met antibody
<220>
   <221> MOD_RES
   <222> (3)
   <223> Xaa is Ala(A) or Gly(G)
<220>
   <221> MOD_RES
   <222> (4)
   <223> Xaa is Met(M) or Ile(I)
<220>
   <221> MOD_RES
   <222> (5)
   <223> Xaa is Ser(S) or His(H)
<400> 110
   Ser Tyr Xaa Xaa Xaa
   1               5
<210> 111
   <211> 17
   <212> PRT
   <213> Artificial Sequence
<220>
   <223> CDR-H2 of anti-idiotype antibody against anti-c-Met antibody
<220>
   <221> MOD_RES
   <222> (1)
   <223> Xaa is Gly(G), Ser(S), Leu(L), Ala(A), or Val(V)
<220>
   <221> MOD_RES
   <222> (3)
   <223> Xaa is Tyr(Y) or Ser(S)
<220>
   <221> MOD_RES
   <222> (4)
   <223> Xaa is Ser(S), Tyr(Y), His(H), Pro(P), or Gly(G)
<220>
   <221> MOD_RES
   <222> (5)
   <223> Xaa is Ser(S), Gly(G), Gly(N), or Asp(D)
<220>
   <221> MOD_RES
   <222> (6)
   <223> Xaa is Ser(S), Gly(G), or Asp(D)
<220>
   <221> MOD_RES
   <222> (7)
   <223> Xaa is Ser(S) or Gly(G)
<220>
   <221> MOD_RES
   <222> (8)
   <223> Xaa is Ans(N) or Ser(S)
<220>
   <221> MOD_RES
   <222> (9)
   <223> Xaa is Ile(I), Thr(T), or Lys(K)
<220>
   <221> MOD_RES
   <222> (16)
   <223> Xaa is Lys(K) or Glu(E)
<400> 111
   Xaa Ile Xaa Xaa Xaa Xaa Xaa Xaa Xaa Tyr Tyr Ala Asp Ser Val Xaa
    1               5                  10                  15
   Gly
<210> 112
   <211> 13
   <212> PRT
   <213> Artificial Sequence
<220>
   <223> CDR-L1 of anti-idiotype antibody against anti-c-Met antibody
<220>
   <221> MOD_RES
   <222> (1)
   <223> Xaa is Ser(S) or Thr(T)
<220>
   <221> MOD_RES
   <222> (9)
   <223> Xaa is Ans(N) or Ser(S)
<220>
   <221> MOD_RES
   <222> (11)
   <223> Xaa is Ser(S), Tyr(Y), or Asp(D)
<220>
   <221> MOD_RES
   <222> (13)
   <223> Xaa is Tyr(Y), Thr(T), Ans(N), or Ser(S)
<400> 112
   Xaa Gly Ser Ser Ser Asn Ile Gly Xaa Asn Xaa Val Xaa
    1               5                  10
<210> 113
   <211> 7
   <212> PRT
   <213> Artificial Sequence
<220>
   <223> CDR-L2 of anti-idiotype antibody against anti-c-Met antibody
<220>
   <221> MOD_RES
   <222> (1)
   <223> Xaa is Ser(S), Ala(A), Ans(N), or Glu(E)
<220>
   <221> MOD_RES
   <222> (2)
   <223> Xaa is Asp(D), Ans(N), Thr(T), or Val(V)
<220>
   <221> MOD_RES
   <222> (3)
   <223> Xaa is Ser(S) or Ans(N)
<220>
   <221> MOD_RES
   <222> (4)
   <223> Xaa is Gln(Q), Ans(N), His(H), or Gly(G)
<400> 113
   Xaa Xaa Xaa Xaa Arg Pro Ser
   1               5
<210> 114
   <211> 9
   <212> PRT
   <213> Artificial Sequence
<220>
   <223> CDR-L3 of anti-idiotype antibody against anti-c-Met antibody
<220>
   <221> MOD_RES
   <222> (1)
   <223> Xaa is Gly(G) or Ala(A)
<220>
   <221> MOD_RES
   <222> (2)
   <223> Xaa is Thr(T), Ala(A), or Ser(S)
<220>
   <221> MOD_RES
   <222> (5)
   <223> Xaa is Tyr(Y), Asp(D), Ser(S), or Ala(A)
<220>
   <221> MOD_RES
   <222> (8)
   <223> Xaa is Asp(N) or Ser(S)
<220>
   <221> MOD_RES
   <222> (9)
   <223> Xaa is Gly(G) or Ala(A)
<400> 114
   Xaa Xaa Trp Asp Xaa Ser Leu Xaa Xaa
   1               5
<210> 115
   <211> 5
   <212> PRT
   <213> Artificial Sequence
<220>
   <223> CDR-H1 of anti-idiotype antibody against anti-c-Met antibody
<400> 115
   Asp Tyr Tyr Met Ser
   1 5
<210> 116
   <211> 5
   <212> PRT
   <213> Artificial Sequence
<220>
   <223> CDR-H1 of anti-idiotype antibody against anti-c-Met antibody
<400> 116
   Asn Tyr Ala Met Ser
   1 5
<210> 117
   <211> 5
   <212> PRT
   <213> Artificial Sequence
<220>
   <223> CDR-H1 of anti-idiotype antibody against anti-c-Met antibody
<400> 117
   Asp Tyr Asp Met Ser
   1 5
<210> 118
   <211> 5
   <212> PRT
   <213> Artificial Sequence
<220>
   <223> CDR-H1 of anti-idiotype antibody against anti-c-Met antibody
<400> 118
   Gly Tyr Asp Met Ser
   1 5
<210> 119
   <211> 5
   <212> PRT
   <213> Artificial Sequence
<220>
   <223> CDR-H1 of anti-idiotype antibody against anti-c-Met antibody
<400> 119
   Asp Tyr Ala Met Ser
   1 5
<210> 120
   <211> 5
   <212> PRT
   <213> Artificial Sequence
<220>
   <223> CDR-H1 of anti-idiotype antibody against anti-c-Met antibody
<400> 120
   Asn Tyr Ser Met Ser
   1 5
<210> 121
   <211> 5
   <212> PRT
   <213> Artificial Sequence
<220>
   <223> CDR-H1 of anti-idiotype antibody against anti-c-Met antibody
<400> 121
   Ser Tyr Ala Met His
   1 5
<210> 122
   <211> 5
   <212> PRT
   <213> Artificial Sequence
<220>
   <223> CDR-H1 of anti-idiotype antibody against anti-c-Met antibody
<400> 122
   Ser Tyr Ala Ile Ser
   1 5
<210> 123
   <211> 5
   <212> PRT
   <213> Artificial Sequence
<220>
   <223> CDR-H1 of anti-idiotype antibody against anti-c-Met antibody
<400> 123
   Ser Tyr Gly Met His
   1 5
<210> 124
   <211> 5
   <212> PRT
   <213> Artificial Sequence
<220>
   <223> CDR-H1 of anti-idiotype antibody against anti-c-Met antibody
<400> 124
   Ser Tyr Ala Met Ser
   1               5
<210> 125
   <211> 17
   <212> PRT
   <213> Artificial Sequence
<220>
   <223> CDR-H2 of anti-idiotype antibody against anti-c-Met antibody
<400> 125
   Gly Ile Tyr Ser Ser Ser Ser Asn Ile Tyr Tyr Ala Asp Ser Val Lys
   1               5                   10                  15
   Gly
<210> 126
   <211> 17
   <212> PRT
   <213> Artificial Sequence
<220>
   <223> CDR-H2 of anti-idiotype antibody against anti-c-Met antibody
<400> 126
   Ser Ile Ser Ser Ser Gly Gly Asn Thr Tyr Tyr Ala Asp Ser Val Lys
   1               5                   10                  15
   Gly
<210> 127
   <211> 17
   <212> PRT
   <213> Artificial Sequence
<220>
   <223> CDR-H2 of anti-idiotype antibody against anti-c-Met antibody
<400> 127
   Leu Ile Ser Tyr Gly Gly Ser Asn Thr Tyr Tyr Ala Asp Ser Val Lys
   1               5                   10                  15
   Gly
<210> 128
   <211> 17
   <212> PRT
   <213> Artificial Sequence
<220>
   <223> CDR-H2 of anti-idiotype antibody against anti-c-Met antibody
<400> 128
   Gly Ile Ser His Gly Asp Gly Asn Ile Tyr Tyr Ala Asp Ser Val Lys
    1               5                  10                  15
   Gly
<210> 129
   <211> 17
   <212> PRT
   <213> Artificial Sequence
<220>
   <223> CDR-H2 of anti-idiotype antibody against anti-c-Met antibody
<400> 129
   Ser Ile Ser Tyr Gly Gly Gly Ser Ile Tyr Tyr Ala Asp Ser Val Lys
    1               5                  10                  15
   Gly
<210> 130
   <211> 17
   <212> PRT
   <213> Artificial Sequence
<220>
   <223> CDR-H2 of anti-idiotype antibody against anti-c-Met antibody
<400> 130
   Gly Ile Ser Tyr Asn Gly Gly Ser Lys Tyr Tyr Ala Asp Ser Val Lys
    1               5                  10                  15
   Gly
<210> 131
   <211> 17
   <212> PRT
   <213> Artificial Sequence
<220>
   <223> CDR-H2 of anti-idiotype antibody against anti-c-Met antibody
<400> 131
   Ala Ile Ser His Ser Ser Gly Asn Thr Tyr Tyr Ala Asp Ser Val Lys
   1 5 10 15
   Gly
<210> 132
   <211> 17
   <212> PRT
   <213> Artificial Sequence
<220>
   <223> CDR-H2 of anti-idiotype antibody against anti-c-Met antibody
<400> 132
   Ala Ile Tyr Pro Gly Gly Gly Asn Thr Tyr Tyr Ala Asp Ser Val Lys
   1 5 10 15
   Gly
<210> 133
   <211> 17
   <212> PRT
   <213> Artificial Sequence
<220>
   <223> CDR-H2 of anti-idiotype antibody against anti-c-Met antibody
<400> 133
   Ala Ile Ser Ser Gly Asp Gly Asn Thr Tyr Tyr Ala Asp Ser Val Lys
   1 5 10 15
   Gly
<210> 134
   <211> 17
   <212> PRT
   <213> Artificial Sequence
<220>
   <223> CDR-H2 of anti-idiotype antibody against anti-c-Met antibody
<400> 134
   Ser Ile Tyr Pro Asp Asp Gly Asn Thr Tyr Tyr Ala Asp Ser Val Lys
   1 5 10 15
   Gly
<210> 135
   <211> 17
   <212> PRT
   <213> Artificial Sequence
<220>
   <223> CDR-H2 of anti-idiotype antibody against anti-c-Met antibody
<400> 135
   Val Ile Ser Tyr Asp Gly Ser Asn Lys Tyr Tyr Ala Asp Ser Val Lys
   1 5 10 15
   Gly
<210> 136
   <211> 17
   <212> PRT
   <213> Artificial Sequence
<220>
   <223> CDR-H2 of anti-idiotype antibody against anti-c-Met antibody
<400> 136
   Ala Ile Ser Gly Ser Gly Gly Ser Thr Tyr Tyr Ala Asp Ser Val Glu
   1 5 10 15
   Gly
<210> 137
   <211> 17
   <212> PRT
   <213> Artificial Sequence
<220>
   <223> CDR-H2 of anti-idiotype antibody against anti-c-Met antibody
<400> 137
   Ala Ile Ser Gly Ser Gly Gly Ser Thr Tyr Tyr Ala Asp Ser Val Lys
   1 5 10 15
   Gly
<210> 138
   <211> 17
   <212> PRT
   <213> Artificial Sequence
<220>
   <223> CDR-H2 of anti-idiotype antibody against anti-c-Met antibody
<400> 138
   Gly Ile Ile Pro Ile Phe Gly Thr Ala Asn Tyr Ala Gln Lys Phe Gln
   1 5 10 15
   Gly
<210> 139
   <211> 19
   <212> PRT
   <213> Artificial Sequence
<220>
   <223> CDR-H3 of anti-idiotype antibody against anti-c-Met antibody
<400> 139
   Lys Ala Leu Gly Asn Gln Glu Asn Glu Pro Thr Ser Tyr Ser Asn Gly
   1 5 10 15
   Met Asp Val
<210> 140
   <211> 8
   <212> PRT
   <213> Artificial Sequence
<220>
   <223> CDR-H3 of anti-idiotype antibody against anti-c-Met antibody
<400> 140
   Lys Tyr His Ser Val Phe Asp Tyr
   1 5
<210> 141
   <211> 19
   <212> PRT
   <213> Artificial Sequence
<220>
   <223> CDR-H3 of anti-idiotype antibody against anti-c-Met antibody
<400> 141
   Lys Phe Arg Ser Glu Phe Asn Glu Asn Glu Pro Ser Ser Tyr Tyr Gly
   1 5 10 15
   Met Asp Val
<210> 142
   <211> 19
   <212> PRT
   <213> Artificial Sequence
<220>
   <223> CDR-H3 of anti-idiotype antibody against anti-c-Met antibody
<400> 142
   Lys Val Gly Leu Leu Phe Val Gln Glu Glu Pro Ser Tyr Tyr Asn Ala
   1 5 10 15
   Met Asp Val
<210> 143
   <211> 8
   <212> PRT
   <213> Artificial Sequence
<220>
   <223> CDR-H3 of anti-idiotype antibody against anti-c-Met antibody
<400> 143
   Arg Asp Ala Ala Tyr Phe Asp Tyr
   1 5
<210> 144
   <211> 19
   <212> PRT
   <213> Artificial Sequence
<220>
   <223> CDR-H3 of anti-idiotype antibody against anti-c-Met antibody
<400> 144
   Lys Tyr Leu Leu Pro Val Leu Glu Glu Pro Gly Tyr Ser Ala Asp Gly
   1 5 10 15
   Met Asp Val
<210> 145
   <211> 19
   <212> PRT
   <213> Artificial Sequence
<220>
   <223> CDR-H3 of anti-idiotype antibody against anti-c-Met antibody
<400> 145
   Lys His Leu Gly Ala Gln Ser Asp Glu Pro Asp Ser Ser Ser Asn Gly
   1 5 10 15
   Met Asp Val
<210> 146
   <211> 19
   <212> PRT
   <213> Artificial Sequence
<220>
   <223> CDR-H3 of anti-idiotype antibody against anti-c-Met antibody
<400> 146
   Lys Ser Leu Ser Thr His Ser Val Asp Glu Pro Ser Ser Asp Asn Ala
   1 5 10 15
   Met Asp Val
<210> 147
   <211> 19
   <212> PRT
   <213> Artificial Sequence
<220>
   <223> CDR-H3 of anti-idiotype antibody against anti-c-Met antibody
<400> 147
   Arg Tyr Leu Gly Thr Thr Ser Asp Glu Pro Ala Ser Tyr Ser Asn Gly
   1 5 10 15
   Met Asp Val
<210> 148
   <211> 19
   <212> PRT
   <213> Artificial Sequence
<220>
   <223> CDR-H3 of anti-idiotype antibody against anti-c-Met antibody
<400> 148
   Lys Tyr Arg Leu Val Asp Arg Trp Glu Glu Pro Ser Ser Asp Tyr Gly
   1 5 10 15
   Met Asp Val
<210> 149
   <211> 8
   <212> PRT
   <213> Artificial Sequence
<220>
   <223> CDR-H3 of anti-idiotype antibody against anti-c-Met antibody
<400> 149
   Arg Val His Leu Tyr Phe Asp Tyr
   1 5
<210> 150
   <211> 18
   <212> PRT
   <213> Artificial Sequence
<220>
   <223> CDR-H3 of anti-idiotype antibody against anti-c-Met antibody
<400> 150
   Arg Glu Asp Asn Thr Arg Tyr Phe Glu Glu Pro Asn Tyr Tyr Gly Met
   1 5 10 15
   Asp Val
<210> 151
   <211> 16
   <212> PRT
   <213> Artificial Sequence
<220>
   <223> CDR-H3 of anti-idiotype antibody against anti-c-Met antibody
<400> 151
   Arg Asp Arg Asn Ser Tyr Tyr Glu Glu Pro Met Tyr Tyr Phe Asp Tyr
   1 5 10 15
<210> 152
   <211> 16
   <212> PRT
   <213> Artificial Sequence
<220>
   <223> CDR-H3 of anti-idiotype antibody against anti-c-Met antibody
<400> 152
   Arg Asp Leu Val Ala Asp Asp Tyr Gly Asp Tyr Gly Thr Val Asp Tyr
   1 5 10 15
<210> 153
   <211> 12
   <212> PRT
   <213> Artificial Sequence
<220>
   <223> CDR-H3 of anti-idiotype antibody against anti-c-Met antibody
<400> 153
   Lys Glu Arg Leu Glu Glu Pro Gly Phe Phe Asp Tyr
   1 5 10
<210> 154
   <211> 21
   <212> PRT
   <213> Artificial Sequence
<220>
   <223> CDR-H3 of anti-idiotype antibody against anti-c-Met antibody
<400> 154
   Ala Arg Gly Gly Gly Tyr Ser Tyr Gly Tyr Glu Glu Pro Tyr Tyr Tyr
   1 5 10 15
   Tyr Gly Met Asp Val
   20
<210> 155
   <211> 13
   <212> PRT
   <213> Artificial Sequence
<220>
   <223> CDR-L1 of anti-idiotype antibody against anti-c-Met antibody
<400> 155
   Ser Gly Ser Ser Ser Asn Ile Gly Asn Asn Ser Val Tyr
   1 5 10
<210> 156
   <211> 13
   <212> PRT
   <213> Artificial Sequence
<220>
   <223> CDR-L1 of anti-idiotype antibody against anti-c-Met antibody
<400> 156
   Ser Gly Ser Ser Ser Asn Ile Gly Asn Asn Tyr Val Tyr
   1 5 10
<210> 157
   <211> 13
   <212> PRT
   <213> Artificial Sequence
<220>
   <223> CDR-L1 of anti-idiotype antibody against anti-c-Met antibody
<400> 157
   Ser Gly Ser Ser Ser Asn Ile Gly Asn Asn Asp Val Thr
   1 5 10
<210> 158
   <211> 13
   <212> PRT
   <213> Artificial Sequence
<220>
   <223> CDR-L1 of anti-idiotype antibody against anti-c-Met antibody
<400> 158
   Thr Gly Ser Ser Ser Asn Ile Gly Ser Asn Asn Val Thr
   1 5 10
<210> 159
   <211> 13
   <212> PRT
   <213> Artificial Sequence
<220>
   <223> CDR-L1 of anti-idiotype antibody against anti-c-Met antibody
<400> 159
   Ser Gly Ser Ser Ser Asn Ile Gly Asn Asn Ser Val Asn
   1 5 10
<210> 160
   <211> 13
   <212> PRT
   <213> Artificial Sequence
<220>
   <223> CDR-L1 of anti-idiotype antibody against anti-c-Met antibody
<400> 160
   Thr Gly Ser Ser Ser Asn Ile Gly Ser Asn Tyr Val Ser
   1 5 10
<210> 161
   <211> 13
   <212> PRT
   <213> Artificial Sequence
<220>
   <223> CDR-L1 of anti-idiotype antibody against anti-c-Met antibody
<400> 161
   Thr Gly Ser Ser Ser Asn Ile Gly Asn Asn Asp Val Tyr
   1 5 10
<210> 162
   <211> 13
   <212> PRT
   <213> Artificial Sequence
<220>
   <223> CDR-L1 of anti-idiotype antibody against anti-c-Met antibody
<400> 162
   Thr Gly Ser Ser Ser Asn Ile Gly Ser Asn Ser Val Ser
   1 5 10
<210> 163
   <211> 13
   <212> PRT
   <213> Artificial Sequence
<220>
   <223> CDR-L1 of anti-idiotype antibody against anti-c-Met antibody
<400> 163
   Ser Gly Ser Ser Ser Asn Ile Gly Ser Asn Asp Val Tyr
   1 5 10
<210> 164
   <211> 13
   <212> PRT
   <213> Artificial Sequence
<220>
   <223> CDR-L1 of anti-idiotype antibody against anti-c-Met antibody
<400> 164
   Thr Gly Ser Ser Ser Asn Ile Gly Ser Asn Asn Val Asn
   1 5 10
<210> 165
   <211> 13
   <212> PRT
   <213> Artificial Sequence
<220>
   <223> CDR-L1 of anti-idiotype antibody against anti-c-Met antibody
<400> 165
   Ser Gly Ser Ser Ser Asn Ile Gly Ser Asn Ser Val Asn
   1 5 10
<210> 166
   <211> 14
   <212> PRT
   <213> Artificial Sequence
<220>
   <223> CDR-L1 of anti-idiotype antibody against anti-c-Met antibody
<400> 166
   Thr Gly Ser Ser Ser Asn Ile Gly Ala Ala Tyr Glu Val His
   1 5 10
<210> 167
   <211> 11
   <212> PRT
   <213> Artificial Sequence
<220>
   <223> CDR-L1 of anti-idiotype antibody against anti-c-Met antibody
<400> 167
   Ser Gly Asp Lys Leu Gly Asp Arg Tyr Val Phe
   1 5 10
<210> 168
   <211> 13
   <212> PRT
   <213> Artificial Sequence
<220>
   <223> CDR-L1 of anti-idiotype antibody against anti-c-Met antibody
<400> 168
   Ser Gly Ser Gly Ser Asn Ile Gly Ser Asn Ala Val Asn
   1 5 10
<210> 169
   <211> 11
   <212> PRT
   <213> Artificial Sequence
<220>
   <223> CDR-L1 of anti-idiotype antibody against anti-c-Met antibody
<400> 169
   Gly Gly Asn Asn Ile Ala Thr Lys Gly Val His
    1               5                   10
<210> 170
   <211> 14
   <212> PRT
   <213> Artificial Sequence
<220>
   <223> CDR-L1 of anti-idiotype antibody against anti-c-Met antibody
<400> 170
   Thr Gly Thr Ser Ser Asp Val Gly Gly Tyr Asn Tyr Val Ser
    1               5                   10
<210> 171
   <211> 17
   <212> PRT
   <213> Artificial Sequence
<220>
   <223> CDR-L1 of anti-idiotype antibody against anti-c-Met antibody
<400> 171
   Lys Ser Ser Gln Ser Leu Leu Asn Ser Gly Asn Gln Lys Asn Asp Leu
    1               5                   10                  15
   Ala
<210> 172
   <211> 7
   <212> PRT
   <213> Artificial Sequence
<220>
   <223> CDR-L2 of anti-idiotype antibody against anti-c-Met antibody
<400> 172
   Ser Asp Ser Gln Arg Pro Ser
    1               5
<210> 173
   <211> 7
   <212> PRT
   <213> Artificial Sequence
<220>
   <223> CDR-L2 of anti-idiotype antibody against anti-c-Met antibody
<400> 173
   Ala Asn Asn Gln Arg Pro Ser
   1 5
<210> 174
   <211> 7
   <212> PRT
   <213> Artificial Sequence
<220>
   <223> CDR-L2 of anti-idiotype antibody against anti-c-Met antibody
<400> 174
   Ser Asp Ser Asn Arg Pro Ser
   1 5
<210> 175
   <211> 7
   <212> PRT
   <213> Artificial Sequence
<220>
   <223> CDR-L2 of anti-idiotype antibody against anti-c-Met antibody
<400> 175
   Ser Asn Ser His Arg Pro Ser
   1 5
<210> 176
   <211> 7
   <212> PRT
   <213> Artificial Sequence
<220>
   <223> CDR-L2 of anti-idiotype antibody against anti-c-Met antibody
<400> 176
   Ala Asn Asn Asn Arg Pro Ser
   1 5
<210> 177
   <211> 7
   <212> PRT
   <213> Artificial Sequence
<220>
   <223> CDR-L2 of anti-idiotype antibody against anti-c-Met antibody
<400> 177
   Ser Asp Ser Asn Arg Pro Ser
   1 5
<210> 178
   <211> 7
   <212> PRT
   <213> Artificial Sequence
<220>
   <223> CDR-L2 of anti-idiotype antibody against anti-c-Met antibody
<400> 178
   Asp Asp Ser Asn Arg Pro Ser
   1 5
<210> 179
   <211> 7
   <212> PRT
   <213> Artificial Sequence
<220>
   <223> CDR-L2 of anti-idiotype antibody against anti-c-Met antibody
<400> 179
   Ser Asp Asn Asn Arg Pro Ser
   1 5
<210> 180
   <211> 7
   <212> PRT
   <213> Artificial Sequence
<220>
   <223> CDR-L2 of anti-idiotype antibody against anti-c-Met antibody
<400> 180
   Ala Asp Ser Gln Arg Pro Ser
   1 5
<210> 181
   <211> 7
   <212> PRT
   <213> Artificial Sequence
<220>
   <223> CDR-L2 of anti-idiotype antibody against anti-c-Met antibody
<400> 181
   Ser Asp Ser His Arg Pro Ser
   1 5
<210> 182
   <211> 7
   <212> PRT
   <213> Artificial Sequence
<220>
   <223> CDR-L2 of anti-idiotype antibody against anti-c-Met antibody
<400> 182
   Asp Thr Ser Asn Arg Pro Ser
   1 5
<210> 183
   <211> 7
   <212> PRT
   <213> Artificial Sequence
<220>
   <223> CDR-L2 of anti-idiotype antibody against anti-c-Met antibody
<400> 183
   Asp Asp Ser Asp Arg Pro Ser
   1 5
<210> 184
   <211> 7
   <212> PRT
   <213> Artificial Sequence
<220>
   <223> CDR-L2 of anti-idiotype antibody against anti-c-Met antibody
<400> 184
   Ser Asn Asn Gln Arg Pro Ser
   1 5
<210> 185
   <211> 7
   <212> PRT
   <213> Artificial Sequence
<220>
   <223> CDR-L2 of anti-idiotype antibody against anti-c-Met antibody
<400> 185
   Asp Asp Ser Gly Arg Pro Ser
   1 5
<210> 186
   <211> 7
   <212> PRT
   <213> Artificial Sequence
<220>
   <223> CDR-L2 of anti-idiotype antibody against anti-c-Met antibody
<400> 186
   Glu Val Ser Asn Arg Pro Ser
   1 5
<210> 187
   <211> 7
   <212> PRT
   <213> Artificial Sequence
<220>
   <223> CDR-L2 of anti-idiotype antibody against anti-c-Met antibody
<400> 187
   Gly Ala Ser Thr Arg Glu Ser
   1 5
<210> 188
   <211> 9
   <212> PRT
   <213> Artificial Sequence
<220>
   <223> CDR-L3 of anti-idiotype antibody against anti-c-Met antibody
<400> 188
   Gly Thr Trp Asp Tyr Ser Leu Asn Gly
   1 5
<210> 189
   <211> 9
   <212> PRT
   <213> Artificial Sequence
<220>
   <223> CDR-L3 of anti-idiotype antibody against anti-c-Met antibody
<400> 189
   Gly Ala Trp Asp Asp Ser Leu Ser Gly
   1 5
<210> 190
   <211> 9
   <212> PRT
   <213> Artificial Sequence
<220>
   <223> CDR-L3 of anti-idiotype antibody against anti-c-Met antibody
<400> 190
   Gly Thr Trp Asp Ser Ser Leu Ser Ala
   1 5
<210> 191
   <211> 9
   <212> PRT
   <213> Artificial Sequence
<220>
   <223> CDR-L3 of anti-idiotype antibody against anti-c-Met antibody
<400> 191
   Gly Thr Trp Asp Asp Ser Leu Asn Gly
   1               5
<210> 192
   <211> 9
   <212> PRT
   <213> Artificial Sequence
<220>
   <223> CDR-L3 of anti-idiotype antibody against anti-c-Met antibody
<400> 192
   Gly Ala Trp Asp Ala Ser Leu Asn Gly
   1               5
<210> 193
   <211> 9
   <212> PRT
   <213> Artificial Sequence
<220>
   <223> CDR-L3 of anti-idiotype antibody against anti-c-Met antibody
<400> 193
   Ala Thr Trp Asp Ala Ser Leu Ser Ala
   1               5
<210> 194
   <211> 9
   <212> PRT
   <213> Artificial Sequence
<220>
   <223> CDR-L3 of anti-idiotype antibody against anti-c-Met antibody
<400> 194
   Ala Ser Trp Asp Tyr Ser Leu Asn Ala
   1               5
<210> 195
   <211> 9
   <212> PRT
   <213> Artificial Sequence
<220>
   <223> CDR-L3 of anti-idiotype antibody against anti-c-Met antibody
<400> 195
   Gly Ser Trp Asp Ser Ser Leu Ser Gly
   1               5
<210> 196
   <211> 9
   <212> PRT
   <213> Artificial Sequence
<220>
   <223> CDR-L3 of anti-idiotype antibody against anti-c-Met antibody
<400> 196
   Gly Ser Trp Asp Asp Ser Leu Ser Gly
    1               5
<210> 197
   <211> 9
   <212> PRT
   <213> Artificial Sequence
<220>
   <223> CDR-L3 of anti-idiotype antibody against anti-c-Met antibody
<400> 197
   Ala Ala Trp Asp Asp Ser Leu Asn Gly
    1               5
<210> 198
   <211> 9
   <212> PRT
   <213> Artificial Sequence
<220>
   <223> CDR-L3 of anti-idiotype antibody against anti-c-Met antibody
<400> 198
   Gln Val Trp Asp Ser Val Asn Asp His
    1               5
<210> 199
   <211> 9
   <212> PRT
   <213> Artificial Sequence
<220>
   <223> CDR-L3 of anti-idiotype antibody against anti-c-Met antibody
<400> 199
   Gln Leu Trp Asp Gly Arg Ser Asp Gln
    1               5
<210> 200
   <211> 8
   <212> PRT
   <213> Artificial Sequence
<220>
   <223> CDR-L3 of anti-idiotype antibody against anti-c-Met antibody
<400> 200
   Ser Ser Tyr Thr Thr Asp Asn Ala
   1 5
<210> 201
   <211> 7
   <212> PRT
   <213> Artificial Sequence
<220>
   <223> CDR-L3 of anti-idiotype antibody against anti-c-Met antibody
<400> 201
   Gln Asn Asp His Ser Tyr Pro
   1 5
<210> 202
   <211> 127
   <212> PRT
   <213> Artificial Sequence
<220>
   <223> heavy chain variable region of anti-idiotype antibody (EW01) against anti-c-Met antibody
<400> 202
   Glu Val Gln Leu Leu Glu Ser Gly Gly Gly Leu Val Gln Pro Gly Gly
   1 5 10 15
   Ser Leu Arg Leu Ser Cys Ala Val Ser Gly Phe Thr Phe Ser Asp Tyr
   20 25 30
   Tyr Met Ser Trp Val Arg Gln Ala Pro Gly Lys Gly Leu Glu Trp Val
   35 40 45
   Ser Gly Ile Tyr Ser Ser Ser Ser Asn Ile Tyr Tyr Ala Asp Ser Val
   50 55 60
   Lys Gly Arg Phe Thr Ile Ser Arg Asp Asn Ser Glu Asn Thr Leu Tyr
   65 70 75 80
   Leu Gln Met Asn Ser Leu Arg Ala Glu Asp Thr Ala Val Tyr Tyr Cys
   85 90 95
   Ala Lys Ala Leu Gly Asn Gln Glu Asn Glu Pro Thr Ser Tyr Ser Asn
   100 105 110
   Gly Met Asp Val Trp Gly Gln Gly Thr Leu Val Thr Val Ser Ser
   115 120 125
<210> 203
   <211> 116
   <212> PRT
   <213> Artificial Sequence
<220>
   <223> heavy chain variable region of anti-idiotype antibody (EW02) against anti-c-Met antibody
<400> 203
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Tyr
               20                  25                  30
   Ala Met Ser Trp Val Arg Gln Ala Pro Gly Lys Gly Leu Glu Trp Val
           35                  40                  45
   Ser Ser Ile Ser Ser Ser Gly Gly Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Gly Ala Glu Asp Thr Ala Val Tyr Tyr Cys
                   85                  90                  95
   Ala Lys Tyr His Ser Val Phe Asp Tyr Trp Gly Gln Gly Thr Leu Val
               100                 105                 110
   Thr Val Ser Ser
           115
<210> 204
   <211> 127
   <212> PRT
   <213> Artificial Sequence
<220>
   <223> heavy chain variable region of anti-idiotype antibody (EW03) against anti-c-Met antibody
<400> 204
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Tyr
               20                  25                  30
   Asp Met Ser Trp Val Arg Gln Ala Pro Gly Lys Gly Leu Glu Trp Val
           35                  40                  45
   Ser Leu Ile Ser Tyr Gly Gly Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Phe Arg Ser Glu Phe Asn Glu Asn Glu Pro Ser Ser Tyr Tyr
               100                 105                 110
   Gly Met Asp Val Trp Gly Gln Gly Thr Leu Val Thr Val Ser Ser
           115                 120                 125
<210> 205
   <211> 127
   <212> PRT
   <213> Artificial Sequence
<220>
   <223> heavy chain variable region of anti-idiotype antibody (EW06) against anti-c-Met antibody
<400> 205
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Gly Tyr
               20                  25                  30
   Asp Met Ser Trp Val Arg Gln Ala Pro Gly Lys Gly Leu Glu Trp Val
           35                  40                  45
   Ser Gly Ile Ser His Gly Asp Gly Asn Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Gly Leu Leu Phe Val Gln Glu Glu Pro Ser Tyr Tyr Asn
               100                 105                 110
   Ala Met Asp Val Trp Gly Gln Gly Thr Leu Val Thr Val Ser Ser
           115                 120                 125
<210> 206
   <211> 116
   <212> PRT
   <213> Artificial Sequence
<220>
   <223> heavy chain variable region of anti-idiotype antibody (EW09) against anti-c-Met antibody
<400> 206
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Asp Tyr
   20 25 30
   Asp Met Ser Trp Val Arg Gln Ala Pro Gly Lys Gly Leu Glu Trp Val
   35 40 45
   Ser Ser Ile Ser Tyr Gly Gly Gly Ser Ile Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Met Tyr Tyr Cys
   85 90 95
   Ala Arg Asp Ala Ala Tyr Phe Asp Tyr Trp Gly Gln Gly Thr Leu Val
   100 105 110
   Thr Val Ser Ser
   115
<210> 207
   <211> 127
   <212> PRT
   <213> Artificial Sequence
<220>
   <223> heavy chain variable region of anti-idiotype antibody (EW10) against anti-c-Met antibody
<400> 207
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Gly Tyr
   20 25 30
   Asp Met Ser Trp Val Arg Gln Ala Pro Gly Lys Gly Leu Glu Trp Val
   35 40 45
   Ser Gly Ile Ser Tyr Asn Gly Gly Ser Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Tyr Leu Leu Pro Val Leu Glu Glu Pro Gly Tyr Ser Ala Asp
   100 105 110
   Gly Met Asp Val Trp Gly Gln Gly Thr Leu Val Thr Val Ser Ser
   115 120 125
<210> 208
   <211> 127
   <212> PRT
   <213> Artificial Sequence
<220>
   <223> heavy chain variable region of anti-idiotype antibody (EW16) against anti-c-Met antibody
<400> 208
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Tyr
               20                  25                  30
   Tyr Met Ser Trp Val Arg Leu Ala Pro Gly Lys Gly Leu Glu Trp Val
           35                  40                  45
   Ser Ala Ile Ser His Ser Ser Gly Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys His Leu Gly Ala Gln Ser Asp Glu Pro Asp Ser Ser Ser Asn
               100                 105                 110
   Gly Met Asp Val Trp Gly Gln Gly Thr Leu Val Thr Val Ser Ser
           115                 120                 125
<210> 209
   <211> 127
   <212> PRT
   <213> Artificial Sequence
<220>
   <223> heavy chain variable region of anti-idiotype antibody (EW26) against anti-c-Met antibody
<400> 209
   Glu Val Gln Leu Leu Glu Ser Gly Gly Gly Leu Val Gln Pro Gly Gly
    1               5                  10                  15
   Ser Leu Arg Leu Ser Cys Ala Ala Ser Gly Phe Thr Phe Ser Asn Tyr
               20                  25                  30
   Ala Met Ser Trp Val Arg Gln Ala Pro Gly Lys Gly Leu Glu Trp Val
           35                  40                  45
   Ser Ala Ile Tyr Pro Gly Gly Gly Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Leu Ser Thr His Ser Val Asp Glu Pro Ser Ser Asp Asn
               100                 105                 110
   Ala Met Asp Val Trp Gly Gln Gly Thr Leu Val Thr Val Ser Ser
           115                 120                 125
<210> 210
   <211> 127
   <212> PRT
   <213> Artificial Sequence
<220>
   <223> heavy chain variable region of anti-idiotype antibody (EW28) against anti-c-Met antibody
<400> 210
   Glu Val Gln Leu Leu Glu Ser Gly Gly Gly Leu Val Gln Thr Gly Gly
    1               5                  10                  15
   Ser Leu Arg Leu Ser Cys Ala Val Ser Gly Phe Thr Phe Ser Asp Tyr
               20                  25                  30
   Ala Met Ser Trp Val Arg Gln Ala Pro Gly Lys Gly Leu Glu Trp Val
           35                  40                  45
   Ser Ala Ile Ser Ser Gly Asp Gly Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Tyr Leu Gly Thr Thr Ser Asp Glu Pro Ala Ser Tyr Ser Asn
               100                 105                 110
   Gly Met Asp Val Trp Gly Gln Gly Thr Leu Val Thr Val Ser Ser
           115                 120                 125
<210> 211
   <211> 127
   <212> PRT
   <213> Artificial Sequence
<220>
   <223> heavy chain variable region of anti-idiotype antibody (EW34) against anti-c-Met antibody
<400> 211
   Glu Val Gln Leu Leu Glu Ser Gly Gly Gly Leu Val Gln Thr Gly Gly
   1               5                   10                  15
   Ser Leu Arg Leu Ser Cys Ala Ala Ser Gly Phe Thr Phe Ser Asp Tyr
               20                  25                  30
   Ala Met Ser Trp Val Arg Gln Ala Pro Gly Lys Gly Leu Glu Trp Val
           35                  40                  45
   Ser Ser Ile Tyr Pro Asp Asp Gly Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Tyr Arg Leu Val Asp Arg Trp Glu Glu Pro Ser Ser Asp Tyr
               100                 105                 110
   Gly Met Asp Val Trp Gly Gln Gly Thr Leu Val Thr Val Ser Ser
           115                 120                 125
<210> 212
   <211> 116
   <212> PRT
   <213> Artificial Sequence
<220>
   <223> heavy chain variable region of anti-idiotype antibody (EW37) against anti-c-Met antibody
<400> 212
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Tyr
               20                  25                  30
   Ser Met Ser Trp Val Arg Gln Ala Pro Gly Lys Gly Leu Glu Trp Val
           35                  40                  45
   Ser Ser Ile Ser Ser Ser Gly Gly Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val His Leu Tyr Phe Asp Tyr Trp Gly Gln Gly Thr Leu Val
               100                 105                 110
   Thr Val Ser Ser
           115
<210> 213
   <211> 126
   <212> PRT
   <213> Artificial Sequence
<220>
   <223> heavy chain variable region of anti-idiotype antibody (HAL 7-1) against anti-c-Met antibodyy
<400> 213
   Gln Val Gln Leu Gln Gln Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Asp Asn Thr Arg Tyr Phe Glu Glu Pro Asn Tyr Tyr Gly
               100                 105                 110
   Met Asp Val Trp Gly Gln Gly Thr Thr Val Thr Val Ser Ser
           115                 120                 125
<210> 214
   <211> 124
   <212> PRT
   <213> Artificial Sequence
<220>
   <223> heavy chain variable region of anti-idiotype antibody (HAL 7-2) against anti-c-Met antibodyy
<400> 214
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Ile Pro Ile Phe Gly Thr Ala Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asp Arg Asn Ser Tyr Tyr Glu Glu Pro Met Tyr Tyr Phe Asp
               100                 105                 110
   Tyr Trp Gly Gln Gly Thr Leu Val Thr Val Ser Ser
           115                 120
<210> 215
   <211> 124
   <212> PRT
   <213> Artificial Sequence
<220>
   <223> heavy chain variable region of anti-idiotype antibody (HAL 7-5) against anti-c-Met antibodyy
<400> 215
   Gln Val Gln Leu Val Gln Ser Gly Ala Glu Val Lys Lys Pro Gly Ala
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Ile Pro Ile Phe Gly Thr Ala Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asp Arg Asn Ser Tyr Tyr Glu Glu Pro Met Tyr Tyr Phe Asp
   100 105 110
   Tyr Trp Gly Gln Gly Thr Leu Val Thr Val Ser Ser
   115 120
<210> 216
   <211> 124
   <212> PRT
   <213> Artificial Sequence
<220>
   <223> heavy chain variable region of anti-idiotype antibody (HAL 7-7) against anti-c-Met antibodyy
<400> 216
   Gln Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Ile Ser Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp Leu Val Ala Asp Asp Tyr Gly Asp Tyr Gly Thr Val Asp
   100 105 110
   Tyr Trp Gly Gln Gly Thr Leu Val Thr Val Ser Ser
   115 120
<210> 217
   <211> 120
   <212> PRT
   <213> Artificial Sequence
<220>
   <223> heavy chain variable region of anti-idiotype antibody (HAL 7-12) against anti-c-Met antibodyy
<400> 217
   Gln Leu Gln Leu Gln Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
       50                  55                  60
   Glu Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Glu Arg Leu Glu Glu Pro Gly Phe Phe Asp Tyr Trp Gly Gln
               100                 105                 110
   Gly Thr Leu Val Thr Val Ser Ser
           115                 120
<210> 218
   <211> 128
   <212> PRT
   <213> Artificial Sequence
<220>
   <223> heavy chain variable region of anti-idiotype antibody (HAL 8-7) against anti-c-Met antibodyy
<400> 218
   Glu Val Gln Leu Val Glu Thr Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Gly Tyr Ser Tyr Gly Tyr Glu Glu Pro Tyr Tyr Tyr
               100                 105                 110
   Tyr Gly Met Asp Val Trp Gly Gln Gly Thr Thr Val Thr Val Ser Ser
           115                 120                 125
<210> 219
   <211> 381
   <212> DNA
   <213> Artificial Sequence
<220>
   <223> nucleic acid sequence encoding heavy chain variable region of anti-idiotype antibody (EW01) against anti-c-Met antibody
<400> 219
   gaggtgcagc tgttggagtc tgggggaggc ttggtacagc ctggggggtc cctgagactc 60
   tcctgtgcag tctctggatt cacctttagc gattattata tgagctgggt ccgccaggct 120
   ccagggaagg ggctggagtg ggtctcaggg atctattcta gtagtagtaa tatatattac 180
   gctgattctg taaaaggtcg gttcaccatc tccagagaca attccgagaa cacgctgtat 240
   ctgcaaatga acagcctgag agccgaggac acggccgtgt attactgtgc gaaagctctt 300
   ggtaatcagg agaatgagcc gacttcttat tctaatggta tggacgtctg gggccagggt 360
   acactggtca ccgtgagctc a 381
<210> 220
   <211> 348
   <212> DNA
   <213> Artificial Sequence
<220>
   <223> nucleic acid sequence encoding heavy chain variable region of anti-idiotype antibody (EW02) against anti-c-Met antibody
<400> 220
   gaggtgcagc tgttggagtc tgggggaggc ttggtacagc ctggggggtc cctgagactc 60
   tcctgtgcag cctctggatt cacctttagc aattatgcta tgagctgggt ccgccaggct 120
   ccagggaagg ggctggagtg ggtctcatcg atctcttcta gtggtggtaa tacatattac 180
   gctgattctg taaaaggtcg gttcaccatc tccagagaca attccaagaa cacgctgtat 240
   ctgcaaatga acagcctggg agccgaggac acggccgtgt attactgtgc gaaatatcat 300
   tcggttttcg actactgggg ccagggtaca ctggtcaccg tgagctca 348
<210> 221
   <211> 381
   <212> DNA
   <213> Artificial Sequence
<220>
   <223> nucleic acid sequence encoding heavy chain variable region of anti-idiotype antibody (EW03) against anti-c-Met antibody
<400> 221
   gaggtgcagc tgttggagtc cgggggaggc ttggtacagc ctggggggtc cctgagactc 60
   tcctgtgcag cctctggatt cacctttagc gattatgata tgagctgggt ccgccaggct 120
   ccagggaagg ggctggagtg ggtctcattg atctcttatg gtggtagtaa tacatattac 180
   gctgattctg taaaaggtcg gttcaccatc tccagagaca attccaagaa cacgctgtat 240
   ctgcaaatga acagcctgag agccgaggac acggccgtgt attactgtgc gaaatttcgt 300
   agtgagttta atgagaatga gccgtcttct tattatggta tggacgtctg gggccagggt 360
   acactggtca ccgtgagctc a 381
<210> 222
   <211> 381
   <212> DNA
   <213> Artificial Sequence
<220>
   <223> nucleic acid sequence encoding heavy chain variable region of anti-idiotype antibody (EW06) against anti-c-Met antibody
<400> 222
   gaggtgcagc tgttggagtc ggggggaggc ttggtacagc ctggggggtc cctgagactc 60
   tcctgtgcag cctctggatt cacctttagc ggttatgata tgagctgggt ccgccaggct 120
   ccagggaagg ggctggagtg ggtctcaggg atctctcatg gtgatggtaa tatatattac 180
   gctgattctg taaaaggtcg gttcaccatc tccagagaca attccaagaa cacgctgtat 240
   ctgcaaatga acagcctgag agccgaggac acggccgtgt attactgtgc gaaagttggt 300
   cttctttttg tgcaggagga gccgtcttat tataatgcta tggacgtctg gggccagggt 360
   acactggtca ccgtgagctc a 381
<210> 223
   <211> 348
   <212> DNA
   <213> Artificial Sequence
<220>
   <223> nucleic acid sequence encoding heavy chain variable region of anti-idiotype antibody (EW09) against anti-c-Met antibody
<400> 223
   gaggtgcagc tgttggagtc tgggggaggc ttggtacagc ctggggggtc cctgagactc 60
   tcctgtgcag cctctggatt cacctttagc gattatgata tgagctgggt ccgccaggct 120
   ccagggaagg ggctggagtg ggtctcatcg atctcttatg gtggtggtag tatatattac 180
   gctgattctg taaaaggtcg gttcaccatc tccagagaca attccaagaa cacgctgtat 240
   ctgcaaatga acagcctgag agccgaggac acggccatgt attactgtgc gagagatgct 300
   gcttatttcg actactgggg ccagggtaca ctggtcaccg tgagctca 348
<210> 224
   <211> 381
   <212> DNA
   <213> Artificial Sequence
<220>
   <223> nucleic acid sequence encoding heavy chain variable region of anti-idiotype antibody (EW10) against anti-c-Met antibody
<400> 224
   gaggtgcagc tgttggagtc tgggggaggc ttggtacagc ctggggggtc cctgagactc 60
   tcctgtgcag cctctggatt cacctttagc ggttatgata tgagctgggt ccgccaggct 120
   ccagggaagg ggctggagtg ggtctcaggg atctcttata atggtggtag taaatattac 180
   gctgattctg taaaaggtcg gttcaccatc tccagagaca attccaagaa cacgctgtat 240
   ctgcaaatga acagcctgag agccgaggac acggccgtgt attactgtgc gaaatatctt 300
   cttccggttc tggaggagcc ggggtattct gctgatggta tggacgtctg gggccagggt 360
   acactggtca ccgtgagctc a 381
<210> 225
   <211> 381
   <212> DNA
   <213> Artificial Sequence
<220>
   <223> nucleic acid sequence encoding heavy chain variable region of anti-idiotype antibody (EW16) against anti-c-Met antibodyy
<400> 225
   gaggtgcagc tgttggagtc tgggggaggc ttggtacagc ctggggggtc cctgagactc 60
   tcctgtgcag cctctggatt cacctttagc gattattata tgagctgggt ccgcctggct 120
   ccagggaagg ggctggagtg ggtctcagcg atctctcata gtagtggtaa tacatattac 180
   gctgattctg taaaaggtcg gttcaccatc tccagagaca attccaagaa cacgctgtat 240
   ctgcaaatga acagcctgag agccgaggac acggccgtgt attactgtgc gaaacatctt 300
   ggtgcgcagt cggatgagcc ggattcttct tctaatggta tggacgtctg gggccagggt 360
   acactggtca ccgtgagctc a 381
<210> 226
   <211> 381
   <212> DNA
   <213> Artificial Sequence
<220>
   <223> nucleic acid sequence encoding heavy chain variable region of anti-idiotype antibody (EW26) against anti-c-Met antibody
<400> 226
   gaggtgcagc tgttggagtc tgggggaggc ttggtacagc ctggggggtc cctgagactc 60
   tcctgtgcag cctctggatt cacctttagc aattatgcta tgagctgggt ccgccaggct 120
   ccagggaagg ggctggagtg ggtctcagcg atctatcctg gtggtggtaa tacatattac 180
   gctgattctg taaaaggtcg gttcaccatc tccagagaca attccaagaa cacgctgtat 240
   ctgcaaatga acagcctgag agccgaggac acggccgtgt attactgtgc gaaatctctt 300
   agtactcata gtgtggatga gccgtcttct gataatgcta tggacgtctg gggccagggt 360
   acactggtca ccgtgagctc a 381
<210> 227
   <211> 381
   <212> DNA
   <213> Artificial Sequence
<220>
   <223> nucleic acid sequence encoding heavy chain variable region of anti-idiotype antibody (EW28) against anti-c-Met antibody
<400> 227
   gaggtgcagc tgttggagtc tgggggaggc ttggtacaga ctggggggtc cctgagactc 60
   tcctgtgcag tctctggatt cacctttagc gattatgcta tgagctgggt ccgccaggct 120
   ccagggaagg ggctggagtg ggtctcagcg atctcttctg gtgatggtaa tacatattac 180
   gctgattctg taaaaggtcg gttcaccatc tccagagaca attccaagaa cacgctgtat 240
   ctgcaaatga acagcctgag agccgaggac acggccgtgt attactgtgc gagatatctt 300
   ggtactacga gtgatgagcc ggcttcttat tctaatggta tggacgtctg gggccagggt 360
   acactggtca ccgtgagctc a 381
<210> 228
   <211> 381
   <212> DNA
   <213> Artificial Sequence
<220>
   <223> nucleic acid sequence encoding heavy chain variable region of anti-idiotype antibody (EW34) against anti-c-Met antibody
<400> 228
   gaggtgcagc tgttggagtc tgggggaggc ttggtacaga ctggggggtc cctgagactc 60
   tcctgtgcag cctctggatt cacctttagc gattatgcta tgagctgggt ccgccaggct 120
   ccagggaagg ggctggagtg ggtctcatcg atctatcctg atgatggtaa tacatattac 180
   gctgattctg taaaaggtcg gttcaccatc tccagagaca attccaagaa cacgctgtat 240
   ctgcaaatga acagcctgag agccgaggac acggccgtgt attactgtgc gaaatatcgt 300
   cttgtggata ggtgggagga gccgtcttct gattatggta tggacgtctg gggccagggt 360
   acactggtca ccgtgagctc a 381
<210> 229
   <211> 348
   <212> DNA
   <213> Artificial Sequence
<220>
   <223> nucleic acid sequence encoding heavy chain variable region of anti-idiotype antibody (EW37) against anti-c-Met antibody
<400> 229
   gaggtgcagc tgttggagtc cgggggaggc ttggtacagc ctggggggtc cctgagactc 60
   tcctgtgcag cctctggatt cacctttagc aattattcta tgagctgggt ccgccaggct 120
   ccagggaagg ggctggagtg ggtctcatcg atctcttcta gtggtggtaa tacatattac 180
   gctgattctg taaaaggtcg gttcaccatc tccagagaca attccaagaa cacgctgtat 240
   ctgcaaatga acagcctgag agccgaggac acggccgtgt attactgtgc gagagtgcat 300
   ttgtatttcg actactgggg ccagggtaca ctggtcaccg tgagctca 348
<210> 230
   <211> 378
   <212> DNA
   <213> Artificial Sequence
<220>
   <223> nucleic acid sequence encoding heavy chain variable region of anti-idiotype antibody (HAL7-1) against anti-c-Met antibody
<400> 230
   caggtacagc tgcagcagtc agggggaggc gtggtccagc ctgggaggtc cctgagactc 60
   tcctgtgcag cctctggatt caccttcagt agctatgcta tgcactgggt ccgccaggct 120
   ccaggcaagg ggctggagtg ggtggcagtt atatcatatg atggaagcaa taaatactac 180
   gcagactccg tgaagggccg attcaccatc tccagagaca attccaagaa cacgctgtat 240
   ctgcaaatga acagcctgag agctgaggac acggctgtgt attactgtgc gagagaggat 300
   aatacgcgat attttgaaga accgaactac tacggtatgg acgtctgggg ccaagggacc 360
   acggtcaccg tctcctca 378
<210> 231
   <211> 372
   <212> DNA
   <213> Artificial Sequence
<220>
   <223> nucleic acid sequence encoding heavy chain variable region of anti-idiotype antibody (HAL7-2) against anti-c-Met antibody
<400> 231
   caggtccagc tggtgcagtc tggggctgag gtgaagaagc ctgggtcctc ggtgaaggtc 60
   tcctgcaagg cttctggagg caccttcagc agctatgcta tcagctgggt gcgacaggcc 120
   cctggacaag ggcttgagtg gatgggaggg atcatcccta tctttggtac agcaaactac 180
   gcacagaagt tccagggcag agtcacgatt accgcggacg aatccacgag cacagcctac 240
   atggagctga gcagcctgag atctgaggac acggccgtgt attactgtgc gagagatcgt 300
   aatagctact acgaggagcc aatgtactac tttgactact ggggccaggg aaccctggtc 360
   accgtctcct ca 372
<210> 232
   <211> 372
   <212> DNA
   <213> Artificial Sequence
<220>
   <223> nucleic acid sequence encoding heavy chain variable region of anti-idiotype antibody (HAL7-5) against anti-c-Met antibody
<400> 232
   caggttcagc tggtgcagtc tggagctgag gtgaagaagc ctggggcctc agtgaaggtc     60
   tcctgcaagg cttctggagg caccttcagc agctatgcta tcagctgggt gcgacaggcc    120
   cctggacaag ggcttgagtg gatgggaggg atcatcccta tctttggtac agcaaactac    180
   gcacagaagt tccagggcag agtcacgatt accgcggacg aatccacgag cacagcctac    240
   atggagctga gcagcctgag atctgaggac acggccgtgt attactgtgc gagagatcgt    300
   aatagctact acgaggagcc aatgtactac tttgactact ggggccaggg aaccctggtc    360
   accgtctcct ca                                                        372
<210> 233
   <211> 372
   <212> DNA
   <213> Artificial Sequence
<220>
   <223> nucleic acid sequence encoding heavy chain variable region of anti-idiotype antibody (HAL7-7) against anti-c-Met antibody
<400> 233
   caggtgcagc tggtggagtc tgggggaggc gtggtccagc ctgggaggtc cctgagactc     60
   tcctgtgcag cctctggatt caccttcagt agctatggca tgcactgggt ccgccaggct    120
   ccaggcaagg ggctggagtg ggtggcagtt atatcatatg atggaagtaa taaatactat    180
   gcagactccg tgaagggccg attcaccatc tccagagaca attccaagaa cacgctgtat    240
   ctgcaaatga acagcctgag agctgaggac acggctgtgt attactgtgc gagagatctc    300
   gtcgccgatg actacggtga ctacgggacc gttgactact ggggccaggg aaccctggtc    360
   accgtctcct ca                                                        372
<210> 234
   <211> 360
   <212> DNA
   <213> Artificial Sequence
<220>
   <223> nucleic acid sequence encoding heavy chain variable region of anti-idiotype antibody (HAL7-12) against anti-c-Met antibody
<400> 234
   cagctgcagc ttcaggagtc ggggggaggc ttggtacagc ctggggggtc cctgagactc 60
   tcctgtgcag cctctggatt cacctttagc agctatgcca tgagctgggt ccgccaggct 120
   ccagggaagg ggctggagtg ggtctcagct attagtggta gtggtggtag cacatactac 180
   gcagactccg tggagggccg gttcaccatc tccagagaca attccaagaa cacgctgtat 240
   ctgcaaatga acagcctgag agccgaggac acggccgtat attactgtgc gaaagagagg 300
   cttgaggagc ccggtttctt tgattactgg ggccagggaa ccctggtcac cgtctcctca         360
<210> 235
   <211> 384
   <212> DNA
   <213> Artificial Sequence
<220>
   <223> nucleic acid sequence encoding heavy chain variable region of anti-idiotype antibody (HAL8-7) against anti-c-Met antibody
<400> 235
   gaggtgcagc tggtggagac tgggggaggc ttggtacagc ctggggggtc cctgagactc 60
   tcctgtgcag cctctggatt cacctttagc agctatgcca tgagctgggt ccgccaggct 120
   ccagggaagg ggctggagtg ggtctcagct attagtggta gtggtggtag cacatactac 180
   gcagactccg tgaagggccg gttcaccatc tccagagaca attccaagaa cacgctgtat 240
   ctgcaaatga acagcctgag agccgaggac acggctgtgt attactgtgc gagagggggt 300
   ggatacagct atggttacga ggaaccctac tactactacg gtatggacgt ctggggccaa 360
   gggaccacgg tcaccgtctc ctca 384
<210> 236
   <211> 111
   <212> PRT
   <213> Artificial Sequence
<220>
   <223> light chain variable region of anti-idiotype (EW01) antibody against anti-c-Met antibody
<400> 236
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Asn Asn
               20                  25                  30
   Ser Val Tyr Trp Tyr Gln Gln Leu Pro Gly Thr Ala Pro Lys Leu Leu
           35                  40                  45
   Ile Tyr Ser Asp Ser Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Gly Thr Trp Asp Tyr Ser Leu
                   85                  90                  95
   Asn Gly Tyr Val Phe Gly Gly Gly Thr Lys Leu Thr Val Leu Gly
               100                 105                 110
<210> 237
   <211> 111
   <212> PRT
   <213> Artificial Sequence
<220>
   <223> light chain variable region of anti-idiotype (EW02) antibody against anti-c-Met antibody
<400> 237
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Asn Asn
               20                  25                  30
   Tyr Val Tyr Trp Tyr Gln Gln Leu Pro Gly Thr Ala Pro Lys Leu Leu
           35                  40                  45
   Ile Tyr Ala Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Gly Ala Trp Asp Asp Ser Leu
                   85                  90                  95
   Ser Gly Tyr Val Phe Gly Gly Gly Thr Lys Leu Thr Val Leu Gly
               100                 105                 110
<210> 238
   <211> 111
   <212> PRT
   <213> Artificial Sequence
<220>
   <223> light chain variable region of anti-idiotype (EW03) antibody against anti-c-Met antibody
<400> 238
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Asn Asn
   20 25 30
   Asp Val Thr Trp Tyr Gln Gln Leu Pro Gly Thr Ala Pro Lys Leu Leu
   35 40 45
   Ile Tyr Ser Asp Ser Asn Arg Pro Ser Gly Val Pro Asp Arg Phe Ser
   50 55 60
   Gly Ser Lys Ser Gly Thr Ser Ala Ser Leu Ala Ile Ser Gly Leu Arg
   65 70 75 80
   Ser Glu Asp Glu Ala Asp Tyr Tyr Cys Gly Thr Trp Asp Ser Ser Leu
   85 90 95
   Ser Ala Tyr Val Phe Gly Gly Gly Thr Lys Leu Thr Val Leu Gly
   100 105 110
<210> 239
   <211> 111
   <212> PRT
   <213> Artificial Sequence
<220>
   <223> light chain variable region of anti-idiotype (EW06) antibody against anti-c-Met antibody
<400> 239
   Gln Ser Val Leu Thr Gln Pro Pro Ser Ala Ser Gly Thr Pro Gly Gln
   1 5 10 15
   Arg Val Thr Ile Ser Cys Thr Gly Ser Ser Ser Asn Ile Gly Ser Asn
   20 25 30
   Asn Val Thr Trp Tyr Gln Gln Leu Pro Gly Thr Ala Pro Lys Leu Leu
   35 40 45
   Ile Tyr Ser Asn Ser His Arg Pro Ser Gly Val Pro Asp Arg Phe Ser
   50 55 60
   Gly Ser Lys Ser Gly Thr Ser Ala Ser Leu Ala Ile Ser Gly Leu Arg
   65 70 75 80
   Ser Glu Asp Gly Ala Asp Tyr Tyr Cys Gly Thr Trp Asp Asp Ser Leu
   85 90 95
   Asn Gly Tyr Val Phe Gly Gly Gly Thr Lys Leu Thr Val Leu Gly
   100 105 110
<210> 240
   <211> 111
   <212> PRT
   <213> Artificial Sequence
<220>
   <223> light chain variable region of anti-idiotype (EW09) antibody against anti-c-Met antibody
<400> 240
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Asn Asn
               20                  25                  30
   Ser Val Asn Trp Tyr Gln Gln Leu Pro Gly Thr Ala Pro Lys Leu Leu
           35                  40                  45
   Ile Tyr Ala Asn Asn Asn Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Gly Ala Trp Asp Ala Ser Leu
                   85                  90                  95
   Asn Gly Tyr Val Phe Gly Gly Gly Thr Lys Leu Thr Val Leu Gly
               100                 105                 110
<210> 241
   <211> 111
   <212> PRT
   <213> Artificial Sequence
<220>
   <223> light chain variable region of anti-idiotype (EW10) antibody against anti-c-Met antibody
<400> 241
   Gln Ser Val Leu Thr Gln Pro Pro Ser Ala Ser Gly Thr Pro Gly Gln
   1               5                   10                  15
   Arg Val Thr Ile Ser Cys Thr Gly Ser Ser Ser Asn Ile Gly Ser Asn
               20                  25                  30
   Tyr Val Ser Trp Tyr Arg Gln Leu Pro Gly Thr Ala Pro Lys Leu Leu
           35                  40                  45
   Ile Tyr Ser Asp Ser Asn Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Thr Trp Asp Ala Ser Leu
                   85                  90                  95
   Ser Ala Tyr Val Phe Gly Gly Gly Thr Lys Leu Thr Val Leu Gly
               100                 105                 110
<210> 242
   <211> 111
   <212> PRT
   <213> Artificial Sequence
<220>
   <223> light chain variable region of anti-idiotype (EW16) antibody against anti-c-Met antibody
<400> 242
   Gln Ser Val Leu Thr Gln Pro Pro Ser Ala Ser Gly Thr Pro Gly Gln
   1               5                   10                  15
   Arg Val Thr Ile Ser Cys Thr Gly Ser Ser Ser Asn Ile Gly Asn Asn
               20                  25                  30
   Asp Val Tyr Trp Tyr Gln Gln Leu Pro Gly Thr Ala Pro Lys Leu Leu
           35                  40                  45
   Ile Tyr Ser Asp Ser Asn Arg Pro Ser Gly Ile Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Gly Thr Trp Asp Asp Ser Leu
                   85                  90                  95
   Asn Gly Tyr Val Phe Gly Gly Gly Thr Lys Leu Thr Val Leu Gly
               100                 105                 110
<210> 243
   <211> 111
   <212> PRT
   <213> Artificial Sequence
<220>
   <223> light chain variable region of anti-idiotype (EW26) antibody against anti-c-Met antibody
<400> 243
   Gln Ser Val Leu Thr Gln Pro Pro Ser Ala Ser Gly Thr Pro Gly Gln
   1               5                   10                  15
   Arg Val Thr Ile Ser Cys Thr Gly Ser Ser Ser Asn Ile Gly Ser Asn
               20                  25                  30
   Ser Val Ser Trp Tyr Gln Gln Leu Pro Gly Thr Ala Pro Lys Leu Leu
           35                  40                  45
   Ile Tyr Asp Asp Ser Asn Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Ser Trp Asp Tyr Ser Leu
                   85                  90                  95
   Asn Ala Tyr Val Phe Gly Gly Gly Thr Lys Leu Thr Val Leu Gly
               100                 105                 110
<210> 244
   <211> 111
   <212> PRT
   <213> Artificial Sequence
<220>
   <223> light chain variable region of anti-idiotype (EW28) antibody against anti-c-Met antibody
<400> 244
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Asp Val Tyr Trp Tyr Gln Gln Leu Pro Gly Thr Ala Pro Lys Leu Leu
           35                  40                  45
   Ile Tyr Ser Asp Asn Asn Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Gly Ala Trp Asp Asp Ser Leu
                   85                  90                  95
   Ser Gly Tyr Val Phe Gly Gly Gly Thr Lys Leu Thr Val Leu Gly
               100                 105                 110
<210> 245
   <211> 111
   <212> PRT
   <213> Artificial Sequence
<220>
   <223> light chain variable region of anti-idiotype (EW34) antibody against anti-c-Met antibody
<400> 245
   Gln Ser Val Leu Thr Gln Pro Pro Ser Ala Ser Gly Thr Pro Gly Gln
   1               5                   10                  15
   Arg Val Thr Ile Ser Cys Thr Gly Ser Ser Ser Asn Ile Gly Ser Asn
               20                  25                  30
   Asn Val Asn Trp Tyr Gln Gln Leu Pro Gly Thr Ala Pro Lys Leu Leu
           35                  40                  45
   Ile Tyr Ala Asp Ser Gln Arg Pro Ser Gly Val Pro Asp Arg Phe Ser
       50                  55                  60
   Gly Pro Lys Ser Gly Thr Ser Ala Ser Leu Ala Ile Ser Gly Leu Arg
   65                  70                  75                  80
   Ser Glu Asp Glu Ala Asp Tyr Tyr Cys Gly Ser Trp Asp Ser Ser Leu
                   85                  90                  95
   Ser Gly Tyr Val Leu Gly Gly Gly Thr Lys Leu Thr Val Leu Gly
               100                 105                 110
<210> 246
   <211> 111
   <212> PRT
   <213> Artificial Sequence
<220>
   <223> light chain variable region of anti-idiotype (EW37) antibody against anti-c-Met antibody
<400> 246
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Ser Val Asn Trp Tyr Gln Gln Leu Pro Gly Thr Ala Pro Lys Leu Leu
           35                  40                  45
   Ile Tyr Ser Asp Ser His Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Gly Ser Trp Asp Asp Ser Leu
                   85                  90                  95
   Ser Gly Tyr Val Phe Gly Gly Gly Thr Lys Leu Thr Val Leu Gly
               100                 105                 110
<210> 247
   <211> 112
   <212> PRT
   <213> Artificial Sequence
<220>
   <223> light chain variable region of anti-idiotype (HAL7-1) antibody against anti-c-Met antibody
<400> 247
   Gln Ala Val Leu Thr Gln Pro Pro Ser Val Ser Gly Ala Pro Gly Gln
    1               5                   10                  15
   Arg Val Thr Ile Ser Cys Thr Gly Ser Ser Ser Asn Ile Gly Ala Ala
               20                  25                  30
   Tyr Glu Val His Trp Tyr Gln Gln Leu Pro Gly Thr Ala Pro Lys Leu
           35                  40                  45
   Leu Ile Tyr Asp Thr Ser Asn Arg Pro Ser Gly Val Pro Asp Arg Phe
       50                  55                  60
   Ser Gly Ser Lys Ser Gly Thr Ser Ala Ser Leu Ala Ile Ser Gly Leu
    65                  70                  75                  80
   Gln Ser Glu Asp Glu Ala Leu Tyr Tyr Cys Ala Ala Trp Asp Asp Ser
                   85                  90                  95
   Leu Asn Gly Pro Val Phe Arg Arg Arg Asp Lys Leu Thr Val Leu Gly
               100                 105                 110
<210> 248
   <211> 109
   <212> PRT
   <213> Artificial Sequence
<220>
   <223> light chain variable region of anti-idiotype (HAL7-2) antibody against anti-c-Met antibody
<400> 248
   Gln Ala Gly Leu Thr Gln Pro Pro Ser Val Ser Val Ser Pro Gly Gln
    1               5                   10                  15
   Thr Ala Ser Ile Thr Cys Ser Gly Asp Lys Leu Gly Asp Arg Tyr Val
               20                  25                  30
   Phe Trp Tyr Gln Gln Lys Pro Gly Gln Ala Pro Val Leu Val Val His
           35                  40                  45
   Asp Asp Ser Asp Arg Pro Ser Gly Ile Pro Glu Arg Phe Ser Gly Ser
       50                  55                  60
   Asn Ser Gly Asp Thr Ala Thr Leu Thr Ile Ser Arg Val Glu Ala Gly
    65                  70                  75                  80
   Asp Glu Ala Asp Tyr Tyr Cys Gln Val Trp Asp Ser Val Asn Asp His
                   85                  90                  95
   Pro Val Phe Gly Gly Gly Thr Lys Leu Thr Val Leu Gly
               100                 105
<210> 249
   <211> 111
   <212> PRT
   <213> Artificial Sequence
<220>
   <223> light chain variable region of anti-idiotype (HAL7-5) antibody against anti-c-Met antibody
<400> 249
   Gln Leu Val Leu Thr Gln Ser Ser Ser Ala Ser Gly Thr Pro Gly Gln
   1               5                   10                  15
   Arg Val Thr Ile Ser Cys Ser Gly Ser Gly Ser Asn Ile Gly Ser Asn
               20                  25                  30
   Ala Val Asn Trp Tyr Gln Gln Leu Pro Gly Ala Ala Pro Lys Leu Leu
           35                  40                  45
   Ile His Ser Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Pro Gln
   65                  70                  75                  80
   Ser Glu Asp Glu Ala Asp Tyr Tyr Cys Ala Ala Trp Asp Asp Ser Leu
                   85                  90                  95
   Asn Gly Val Val Phe Gly Gly Gly Thr Lys Leu Thr Val Leu Gly
               100                 105                 110
<210> 250
   <211> 109
   <212> PRT
   <213> Artificial Sequence
<220>
   <223> light chain variable region of anti-idiotype (HAL7-7) antibody against anti-c-Met antibody
<400> 250
   Gln Ser Val Leu Thr Gln Pro Pro Ser Val Ser Met Ala Pro Gly Gln
   1               5                   10                  15
   Thr Ala Arg Ile Thr Cys Gly Gly Asn Asn Ile Ala Thr Lys Gly Val
               20                  25                  30
   His Trp Tyr Gln Gln Lys Ala Gly Gln Ala Pro Val Leu Val Val Tyr
           35                  40                  45
   Asp Asp Ser Gly Arg Pro Ser Gly Ile Pro Asp Arg Phe Ser Gly Ser
       50                  55                  60
   Lys Ser Gly Asn Thr Ala Thr Leu Thr Ile Ser Arg Val Glu Ala Gly
   65                  70                  75                  80
   Asp Glu Ala Asp Tyr Tyr Cys Gln Leu Trp Asp Gly Arg Ser Asp Gln
                   85                  90                  95
   Val Leu Phe Gly Gly Gly Thr Lys Leu Thr Val Leu Gly
               100                 105
<210> 251
   <211> 111
   <212> PRT
   <213> Artificial Sequence
<220>
   <223> light chain variable region of anti-idiotype (HAL7-12) antibody against anti-c-Met antibody
<400> 251
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Glu Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala His Tyr Tyr Cys Ser Ser Tyr Thr Thr Asp
                   85                  90                  95
   Asn Ala Trp Val Phe Gly Gly Gly Thr Gln Leu Thr Val Leu Gly
               100                 105                 110
<210> 252
   <211> 114
   <212> PRT
   <213> Artificial Sequence
<220>
   <223> light chain variable region of anti-idiotype (HAL8-7) antibody against anti-c-Met antibody
<400> 252
   Ala Ile Gln Leu Thr Gln Ser Pro Leu Ser Leu Ser Val Ser Ala Gly
   1               5                   10                  15
   Glu Lys Val Thr Met Ser Cys Lys Ser Ser Gln Ser Leu Leu Asn Ser
               20                  25                  30
   Gly Asn Gln Lys Asn Asp Leu Ala Trp Tyr Gln Gln Lys Pro Gly Gln
           35                  40                  45
   Arg Pro Lys Leu Leu Ile Tyr Gly Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Ser Ser Val Gln Ala Glu Asp Leu Ala Val Tyr Tyr Cys Gln Asn
                   85                  90                  95
   Asp His Ser Tyr Pro Leu Thr Phe Gly Ala Gly Thr Lys Leu Glu Ile
               100                 105                 110
   Lys Arg
<210> 253
   <211> 333
   <212> DNA
   <213> Artificial Sequence
<220>
   <223> nucleic acid sequence encoding light chain variable region of anti-idiotype antibody (EW01) against anti-c-Met antibody
<400> 253
   cagtctgtgc tgactcagcc accctcagcg tctgggaccc ccgggcagag ggtcaccatc     60
   tcttgtagtg gctcttcatc taatattggc aataattctg tctactggta ccagcagctc    120
   ccaggaacgg cccccaaact cctcatctat tctgatagtc agcggccaag cggggtccct    180
   gaccgattct ctggctccaa gtctggcacc tcagcctccc tggccatcag tgggctccgg    240
   tccgaggatg aggctgatta ttactgtggt acttgggatt atagcctgaa tggttatgtc    300
   ttcggcggag gcaccaagct tacggtccta ggc                                 333
<210> 254
   <211> 333
   <212> DNA
   <213> Artificial Sequence
<220>
   <223> nucleic acid sequence encoding light chain variable region of anti-idiotype antibody (EW02) against anti-c-Met antibody
<400> 254
   cagtctgtgc tgactcagcc accctcagcg tctgggaccc ccgggcagag ggtcaccatc     60
   tcttgtagtg gctcttcatc taatattggc aataattatg tctactggta ccagcagctc    120
   ccaggaacgg cccccaaact cctcatctat gctaataatc agcggccaag cggggtccct    180
   gaccgattct ctggctccaa gtctggcacc tcagcctccc tggccatcag tgggctccgg    240
   tccgaggatg aggctgatta ttactgtggt gcttgggatg atagcctgag tggttatgtc    300
   ttcggcggag gcaccaagct gacggtccta ggc                                 333
<210> 255
   <211> 333
   <212> DNA
   <213> Artificial Sequence
<220>
   <223> nucleic acid sequence encoding light chain variable region of anti-idiotype antibody (EW03) against anti-c-Met antibody
<400> 255
   cagtctgtgc tgactcagcc accctcagcg tctgggaccc ccgggcagag ggtcaccatc      60
   tcttgtagtg gctcttcatc taatattggc aataatgatg tcacctggta ccagcagctc     120
   ccaggaacgg cccccaaact cctcatctat tctgatagta atcggccaag cggggtccct     180
   gaccgattct ctggctccaa gtctggcacc tcagcctccc tggccatcag tgggctccgg     240
   tccgaggatg aggctgatta ttactgtggt acttgggatt ctagcctgag tgcttatgtc     300
   ttcggcggag gcaccaagct gacggtccta ggc                                  333
<210> 256
   <211> 333
   <212> DNA
   <213> Artificial Sequence
<220>
   <223> nucleic acid sequence encoding light chain variable region of anti-idiotype antibody (EW06) against anti-c-Met antibody
<400> 256
   cagtctgtgc tgactcagcc accctcagcg tctgggaccc ccgggcagag ggtcaccatc      60
   tcttgtactg gctcttcatc taatattggc agtaataatg tcacctggta ccagcagctc     120
   ccaggaacgg cccccaaact cctcatctat tctaatagtc atcggccaag cggggtccct     180
   gaccgattct ctggctccaa gtctggcacc tcagcctccc tggccatcag tgggctccgg     240
   tccgaggatg gggctgatta ttactgtggt acttgggatg atagcctgaa tggttatgtc     300
   ttcggcggag gcaccaagct gacggtccta ggc                                  333
<210> 257
   <211> 333
   <212> DNA
   <213> Artificial Sequence
<220>
   <223> nucleic acid sequence encoding light chain variable region of anti-idiotype antibody (EW09) against anti-c-Met antibody
<400> 257
   cagtctgtgc tgactcagcc accctcagcg tctgggaccc ccgggcagag ggtcaccatc      60
   tcttgtagtg gctcttcatc taatattggc aataattctg tcaactggta ccagcagctc     120
   ccaggaacgg cccccaaact cctcatctat gctaataata atcggccaag cggggtccct     180
   gaccgattct ctggctccaa gtctggcacc tcagcctccc tggccatcag tgggctccgg     240
   tccgaggatg aggctgatta ttactgtggt gcttgggatg ctagcctgaa tggttatgtc     300
   ttcggcggag gcaccaagct gacggtccta ggc                                  333
<210> 258
   <211> 333
   <212> DNA
   <213> Artificial Sequence
<220>
   <223> nucleic acid sequence encoding light chain variable region of anti-idiotype antibody (EW10) against anti-c-Met antibody
<400> 258
   cagtctgtgc tgactcagcc accctcagcg tctgggaccc ccgggcagag ggtcaccatc      60
   tcttgtactg gctcttcatc taatattggc agtaattatg tctcctggta ccggcagctc     120
   ccaggaacgg cccccaaact cctcatctat tctgatagta atcggccaag cggggtccct     180
   gaccgattct ctggctccaa gtctggcacc tcagcctccc tggccatcag tgggctccgg     240
   tccgaggatg aggctgatta ttactgtgct acttgggatg ctagcctgag tgcttatgtc     300
   ttcggcggag gcaccaagct gacggtccta ggc                                  333
<210> 259
   <211> 333
   <212> DNA
   <213> Artificial Sequence
<220>
   <223> nucleic acid sequence encoding light chain variable region of anti-idiotype antibody (EW16) against anti-c-Met antibody
<400> 259
   cagtctgtgc tgactcagcc accctcagcg tctgggaccc ccgggcagag ggtcaccatc      60
   tcttgtactg gctcttcatc taatattggc aataatgatg tctactggta ccagcagctc     120
   ccaggaacgg cacccaaact cctcatctat tctgatagta atcggccaag cgggatccct     180
   gaccgattct ctggctccaa gtctggcacc tcagcctccc tggccatcag tgggctccgg     240
   tccgaggatg aggctgatta ttactgtggt acttgggatg atagcctgaa tggttatgtc     300
   ttcggcggag gcaccaagct gacggtccta ggc                                  333
<210> 260
   <211> 333
   <212> DNA
   <213> Artificial Sequence
<220>
   <223> nucleic acid sequence encoding light chain variable region of anti-idiotype antibody (EW26) against anti-c-Met antibody
<400> 260
   cagtctgtgc tgactcagcc accctcagcg tctgggaccc ccgggcagag ggtcaccatc          60
   tcttgtactg gctcttcatc taatattggc agtaattctg tctcctggta ccagcagctc         120
   ccaggaacgg cccccaaact cctcatctat gatgatagta atcggccaag cggggtccct         180
   gaccgattct ctggctccaa gtctggcacc tcagcctccc tggccatcag tgggctccgg         240
   tccgaggatg aggctgatta ttactgtgct tcttgggatt atagcctgaa tgcttatgtc         300
   ttcggcggag gcaccaagct gacggtccta ggc                                      333
<210> 261
   <211> 333
   <212> DNA
   <213> Artificial Sequence
<220>
   <223> nucleic acid sequence encoding light chain variable region of anti-idiotype antibody (EW28) against anti-c-Met antibody
<400> 261
   cagtctgtgc tgactcagcc accctcagcg tctgggaccc ccgggcagag ggtcaccatc          60
   tcttgtagtg gctcttcatc taatattggc agtaatgatg tctactggta ccagcagctc         120
   ccaggaacgg cccccaaact cctcatctat tctgataata atcggccaag cggggtccct         180
   gaccgattct ctggctccaa gtctggcacc tcagcctccc tggccatcag tgggctccgg         240
   tccgaggatg aggctgatta ttactgtggt gcttgggatg atagcctgag tggttatgtc         300
   ttcggcggag gcaccaagct gacggtccta ggc                                      333
<210> 262
   <211> 333
   <212> DNA
   <213> Artificial Sequence
<220>
   <223> nucleic acid sequence encoding light chain variable region of anti-idiotype antibody (EW34) against anti-c-Met antibody
<400> 262
   cagtctgtgc tgactcagcc accctcagcg tctgggaccc ccgggcagag ggtcaccatc          60
   tcttgtactg gctcttcatc taatattggc agtaataatg tcaactggta ccagcagctc         120
   ccaggaacgg cccccaaact cctcatctat gctgatagtc agcggccaag cggggtccct         180
   gaccgattct ctggccccaa gtctggcacc tcagcctccc tggccatcag tgggctccgg         240
   tccgaggatg aggctgatta ttactgtggt tcttgggatt ctagcctgag tggttatgtc         300
   ttaggcggag gcaccaagct gacggtccta ggc                                      333
<210> 263
   <211> 333
   <212> DNA
   <213> Artificial Sequence
<220>
   <223> nucleic acid sequence encoding light chain variable region of anti-idiotype antibody (EW37) against anti-c-Met antibody
<400> 263
   cagtctgtgc tgactcagcc accctcagcg tctgggaccc ccgggcagag ggtcaccatc      60
   tcttgtagtg gctcttcatc taatattggc agtaattctg tcaactggta ccagcagctc     120
   ccaggaacgg cccccaaact cctcatctat tctgatagtc atcggccaag cggggtccct     180
   gaccgattct ctggctccaa gtctggcacc tcagcctccc tggccatcag tgggctccgg     240
   tccgaggatg aggctgatta ttactgtggt tcttgggatg atagcctgag tggttatgtc     300
   ttcggcggag gcaccaagct gacggtccta ggc                                 333
<210> 264
   <211> 336
   <212> DNA
   <213> Artificial Sequence
<220>
   <223> nucleic acid sequence encoding light chain variable region of anti-idiotype antibody (HAL7-1) against anti-c-Met antibody
<400> 264
   caggctgtgc tgactcagcc accctcagtg tctggggccc cagggcagag ggtcaccatc      60
   tcctgcactg ggagcagctc caacatcggg gcagcttatg aggtgcattg gtatcagcag     120
   cttccaggaa cagcccccaa acttctcatt tatgatactt ccaatcggcc ctcaggggtc     180
   cctgaccgat tctctggctc caagtctggc acctcagcct ccctggccat cagtgggctc     240
   cagtctgagg atgaggctct ttattactgt gcagcatggg atgacagcct gaatggtccg     300
   gtctttcggc ggagggacaa gctgaccgtc ctaggt                              336
<210> 265
   <211> 327
   <212> DNA
   <213> Artificial Sequence
<220>
   <223> nucleic acid sequence encoding light chain variable region of anti-idiotype antibody (HAL7-2) against anti-c-Met antibody
<400> 265
   caggcagggc tgactcagcc accctcagtg tccgtgtccc caggacaaac agccagcata 60
   acctgctctg gagataaatt gggggataga tatgttttct ggtatcagca gaagccaggc 120
   caggcccctg tgctggtcgt ccatgatgat agcgaccggc cctcagggat ccctgagcga 180
   ttctctggct ccaactctgg ggacacggcc accctgacca tcagcagggt cgaggccggg 240
   gatgaggccg actattactg tcaggtgtgg gatagtgtta atgatcatcc ggtgttcggc 300
   ggagggacca agctgaccgt cctaggt 327
<210> 266
   <211> 333
   <212> DNA
   <213> Artificial Sequence
<220>
   <223> nucleic acid sequence encoding light chain variable region of anti-idiotype antibody (HAL7-5) against anti-c-Met antibody
<400> 266
   cagcttgtgc tgactcaatc atcgtcagcg tctgggaccc ccgggcagag ggtcaccatc 60
   tcttgttctg gaagcggctc caacatcgga agtaatgctg taaactggta ccagcagctc 120
   ccaggagcgg cccccaaact cctcatccat agtaataatc agcggccctc aggggtccct 180
   gaccgattct ctggctccaa gtctggcacg tcagcctccc tggccatcag tgggccccag 240
   tcagaggatg aggctgacta ttactgtgca gcttgggatg acagtttgaa tggtgtggtt 300
   ttcggcggag ggaccaagct gaccgtcctc ggt 333
<210> 267
   <211> 327
   <212> DNA
   <213> Artificial Sequence
<220>
   <223> nucleic acid sequence encoding light chain variable region of anti-idiotype antibody (HAL7-7) against anti-c-Met antibody
<400> 267
   cagtctgtgc tgactcagcc accctcggtg tcaatggccc caggacagac ggccaggatc 60
   acctgtgggg gaaacaacat tgcaactaaa ggtgtgcact ggtaccagca gaaggcaggc 120
   caggcccctg tgctggtcgt ctatgatgat agcggccggc cctcagggat ccctgaccga 180
   ttctctggct ccaagtctgg gaacacggcc accctgacca tcagcagggt cgaagccggg 240
   gatgaggccg actattactg tcagctgtgg gatggtagga gtgatcaagt gctattcggc 300
   ggagggacca agctgaccgt cctaggt 327
<210> 268
   <211> 333
   <212> DNA
   <213> Artificial Sequence
<220>
   <223> nucleic acid sequence encoding light chain variable region of anti-idiotype antibody (HAL7-12) against anti-c-Met antibody
<400> 268
   cagtctgccc tgactcagcc tgcctccgtg tctgggtctc ctggacagtc gatcaccatc      60
   tcctgcactg gaaccagcag tgacgttggt ggttataact atgtctcctg gtaccaacag     120
   cacccaggca aagcccccaa actcatgatt tatgaggtca gtaatcggcc ctcaggggtt     180
   tctaatcgct tctctggctc caagtctggc aacacggcct ccctgaccat ctctgggctc     240
   caggctgagg acgaggctca ttattattgc agctcatata caaccgacaa cgcttgggtg     300
   ttcggcggag ggacccagct gaccgtcctg ggt                                  333
<210> 269
   <211> 342
   <212> DNA
   <213> Artificial Sequence
<220>
   <223> nucleic acid sequence encoding light chain variable region of anti-idiotype antibody (HAL8-7) against anti-c-Met antibody
<400> 269
   gccatccagt tgacccagtc tccactctcc ctaagtgtgt cagcaggaga gaaggtcact      60
   atgagctgca agtccagtca gagtctgtta aacagtggaa atcaaaagaa cgacttggcc     120
   tggtaccagc agaaaccagg gcaacgtcct aaactgttga tctacggggc atccactagg     180
   gaatctgggg tccctgatcg cttcacaggc agtggatctg gaaccgattt cactcttacc     240
   atcagcagtg tgcaggctga agacctggca gtttattact gtcagaatga tcatagttat     300
   ccgttaacgt tcggtgctgg caccaagctg gaaatcaaac gt                        342
<210> 270
   <211> 330
   <212> PRT
   <213> Artificial Sequence
<220>
   <223> heavy chain constant region of anti-idiotype antibody against anti-c-Met antibody
<400> 270
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Glu Glu
   225                 230                 235                 240
   Met Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 271
   <211> 990
   <212> DNA
   <213> Artificial Sequence
<220>
   <223> nucleic acid sequence encoding heavy chain constant region of anti-idiotype antibody against anti-c-Met antibody
<210> 272
   <211> 104
   <212> PRT
   <213> Artificial Sequence
<220>
   <223> light chain constant region of anti-idiotype antibody against anti-c-Met antibody
<400> 272
   Gln Pro Lys Ala Ala Pro Ser Val Thr Leu Phe Pro Pro Ser Ser Glu
   1               5                   10                  15
   Glu Leu Gln Ala Asn Lys Ala Thr Leu Val Cys Leu Ile Ser Asp Phe
               20                  25                  30
   Tyr Pro Gly Ala Val Thr Val Ala Trp Lys Ala Asp Ser Ser Pro Val
           35                  40                  45
   Lys Ala Gly Val Glu Thr Thr Thr Pro Ser Lys Gln Ser Asn Asn Lys
       50                  55                  60
   Tyr Ala Ala Ser Ser Tyr Leu Ser Leu Thr Pro Glu Gln Trp Lys Ser
   65                  70                  75                  80
   His Arg Ser Tyr Ser Cys Gln Val Thr His Glu Gly Ser Thr Val Glu
                   85                  90                  95
   Lys Thr Val Ala Pro Thr Glu Cys
               100
<210> 273
   <211> 312
   <212> DNA
   <213> Artificial Sequence
<220>
   <223> nucleic acid sequence encoding light chain constant region of anti-idiotype antibody against anti-c-Met antibody
<400> 273
   cagcccaagg ctgccccctc ggtcactctg ttcccgccct cctctgagga gcttcaagcc        60
   aacaaggcca cactggtgtg tctcataagt gacttctacc cgggagccgt gacagtggcc       120
   tggaaggcag atagcagccc cgtcaaggcg ggagtggaga ccaccacacc ctccaaacaa       180
   agcaacaaca agtacgcggc cagcagctac ctgagcctga cgcccgagca gtggaagtcc       240
   cacagaagct acagctgcca ggtcacgcat gaagggagca ccgtggagaa gacagtggcc       300
   cctacagaat gt                                                           312
<210> 274
   <211> 106
   <212> PRT
   <213> Artificial Sequence
<220>
   <223> light chain constant region of anti-idiotype antibody (HAL8-7) against anti-c-Met antibody
<400> 274
   Thr Val Ala Ala Pro Ser Val Phe Ile Phe Pro Pro Ser Asp Glu Gln
   1               5                   10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu Cys
               100                 105
<210> 275
   <211> 318
   <212> DNA
   <213> Artificial Sequence
<220>
   <223> nucleic acid sequence encoding light chain constant region of anti-idiotype antibody (HAL8-7) against anti-c-Met antibody
<400> 275
   acggtggctg caccatctgt cttcatcttc ccgccatctg atgagcagtt gaaatctgga     60
   actgcctctg ttgtgtgcct gctgaataac ttctatccca gagaggccaa agtacagtgg    120
   aaggtggata acgccctcca atcgggtaac tcccaggaga gtgtcacaga gcaggacagc    180
   aaggacagca cctacagcct cagcagcacc ctgacgctga gcaaagcaga ctacgagaaa    240
   cacaaagtct acgcctgcga agtcacccat cagggcctga gctcgcccgt cacaaagagc    300
   ttcaacaggg gagagtgt                                                   318

## Description

### BACKGROUND

### 1. Field

Provided are anti-idiotype antibodies, polypeptides, compositions, vaccines and polynucleotides useful for the analysis, binding, and detection of anti-c-Met antibodies. Additionally, methods of detection and characterization of anti-c-Met antibodies are also provided.

### 2. Description of the Related Art

In antibody therapy, it is essential to measure the half-life of a therapeutic antibody and an effective concentration thereof after it is administered into body Thus, a technique capable of measuring the amount of an antibody remaining in the body is necessary. When an antibody which functions at the fragment crystallizable (Fc) portion or fragment antigen-binding (Fab) portion of the antibody is used for such a measurement, a polyclonal antibody specific to human immunoglobulin G (IgG) is generally used. If a human serum or a monkey serum is to be analyzed using a polyclonal antibody specific to human IgG, the polyclonal antibody has a limitation in its application and may show high background, thereby causing a decrease in accuracy. Additionally there exist methods of measuring the amount of a remaining antibody using an antigen as a capture, but concerns of probability of missing antigen-antibody complex and causing change in antigen-antibody binding affinity due to a conformational change of the antigen have arisen. Therefore, there is a need for the development of an antibody having specificity against an antibody which needs to be detected.

WO 2011/110642 A2 discloses monoclonal antibodies against c-Met.

Young et al., Molecules and Cells, 2012, vol. 34, no. 6, pages 523-529, discloses the anti-c-Met antibody "F46" that binds to human c-Met with high affinity and specificity and induces the degradation of c-Met in multiple cancer cells.

EP 2 316 484 A1 discloses the mouse anti-human c-Met antibody "AbF46" and its therapeutic use in the treatment of an angiogenesis-related disease or cancer.

WO 2012/030982 A1 discloses a method for producing anti-idiotypic antibodies in mice.

### SUMMARY

The invention is defined in the appended claims.

Provided is an anti-idiotype antibody or antigen-binding fragment thereof, wherein the anti-idiotype antibody or antigen-binding fragment thereof specifically binds to an anti-c-Met antibody or an antibody fragment of the anti-c-Met antibody with an affinity of 50 nM or less, and wherein the anti-c-Met antibody or the antibody fragment of the anti-c-Met antibody comprises:
a CDR-H1 comprising SEQ ID NO: 1;
a CDR-H2 comprising SEQ ID NO: 2;
a CDR-H3 comprising SEQ ID NO: 3;
a CDR-L1 comprising SEQ ID NO: 10;
a CDR-L2 comprising SEQ ID NO: 11; and
a CDR-L3 comprising SEQ ID NO: 13; and
wherein the anti-idiotype antibody or antigen-binding fragment thereof comprises:
(i) a CDR-H1 of SEQ ID NO: 115, a CDR-H2 of SEQ ID NO: 125, a CDR-H3 of SEQ ID NO: 139, a CDR-L1 of SEQ ID NO: 155, a CDR-L2 of SEQ ID NO: 172, and a CDR-L3 of SEQ ID NO: 188; or
(ii) a CDR-H1 of SEQ ID NO: 116, a CDR-H2 of SEQ ID NO: 126, a CDR-H3 of SEQ ID NO: 140, a CDR-L1 of SEQ ID NO: 156, a CDR-L2 of SEQ ID NO: 173, and a CDR-L3 of SEQ ID NO: 189.

Additionally, provided are compositions for detecting an anti-c-Met antibody. Related antigen binding fragments, vaccines, polypeptides, polynucleotides, and methods are also provided.

### BRIEF DESCRIPTION OF THE DRAWINGS

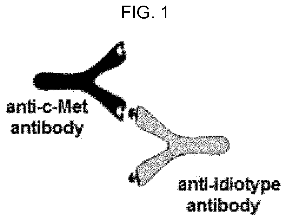

FIG. 1 is a schematic illustrating the interaction between an anti-c-Met antibody and anti-idiotype antibodies according to embodiments of the present invention;.
FIG. 2 is graph displaying the binding affinity of various anti-idiotype antibodies to an anti-c-Met antibody.
FIG. 3 is graph displaying the results of a competitive ELISA, which demonstrates that the binding sites of various anti-idiotype antibodies are idiotope sites of an anti-cMet antibody.
FIG. 4 is graph displaying the detection results of an anti-c-Met antibody using an anti-idiotype antibody in a monkey serum.
FIG. 5 is a graph displaying the neutralizing effects of an anti-idiotype antibody on anti-proliferative efficacy of an anti-cMet antibody.
FIG. 6 is a graph displaying the detection results of an anti-c-Met antibody using an anti-idiotype antibody in a human serum.
FIG. 7 is a schematic illustrating the location of potential binding sites of an anti-idiotype antibody.
FIG. 8 is a graph displaying a standard curve of absorbance measured from a standard sample comprising anti-idiotype antibody EW01, according to various concentrations.

### DETAILED DESCRIPTION

The invention is defined in the appended claims.

Reference will now be made in detail to embodiments, examples of which are illustrated in the accompanying drawings, wherein like reference numerals refer to like elements throughout. In this regard the present embodiments may have different forms and should not be construed as being limited to the descriptions set forth herein. Accordingly, the embodiments are merely described below, by referring to the figures, to explain aspects of the present description.

An anti-idiotype antibody against an anti-c-Met antibody or antigen-binding fragment (i.e., an antibody or antigen-binding fragment thereof that specifically binds to an idiotope of an anti-c-Met antibody) and uses thereof are provided.

More specifically, the invention provides an anti-idiotype antibody or antigen-binding fragment thereof, wherein the anti-idiotype antibody or antigen-binding fragment thereof specifically binds to an anti-c-Met antibody or an antibody fragment of the anti-c-Met antibody with an affinity of 50 nM or less, and wherein the anti-c-Met antibody or the antibody fragment of the anti-c-Met antibody comprises:
a CDR-H1 comprising SEQ ID NO: 1;
a CDR-H2 comprising SEQ ID NO: 2;
a CDR-H3 comprising SEQ ID NO: 3;
a CDR-L1 comprising SEQ ID NO: 10;
a CDR-L2 comprising SEQ ID NO: 11; and
a CDR-L3 comprising SEQ ID NO: 13; and
wherein the anti-idiotype antibody or antigen-binding fragment thereof comprises:
(i) a CDR-H1 of SEQ ID NO: 115, a CDR-H2 of SEQ ID NO: 125, a CDR-H3 of SEQ ID NO: 139, a CDR-L1 of SEQ ID NO: 155, a CDR-L2 of SEQ ID NO: 172, and a CDR-L3 of SEQ ID NO: 188; or
(ii) a CDR-H1 of SEQ ID NO: 116, a CDR-H2 of SEQ ID NO: 126, a CDR-H3 of SEQ ID NO: 140, a CDR-L1 of SEQ ID NO: 156, a CDR-L2 of SEQ ID NO: 173, and a CDR-L3 of SEQ ID NO: 189.

In order to monitor treatment progress during the treatment of c-Met related diseases using an anti-c-Met antibody, a technique for measuring the in vivo state (half-life of antibody, effective concentration, targeting degree, etc.) of the anti-c-Met antibody after the administration thereof into the living body is needed. An anti-idiotype antibody that specifically binds to an idiotope site of an anti-c-Met antibody enables one to measure in vivo state of the anti-c-Met antibody in a simple and accurate way.

In addition, as all therapeutic proteins, including therapeutic antibodies are substances having a potential antigenicity, they all have the possibility of causing an immune response when administered into the body. Once an antibody specific for the therapeutic protein administered into the body (anti-drug antibody; ADA) is generated, there is a high possibility that an antibody-drug (therapeutic protein) complex is formed, and that the drug efficacy of the therapeutic protein is diminished and side effects are caused. Hence, it is very important to monitor and measure any production of such an anti-drug antibody in subjects undergoing treatment with therapeutic proteins, as such an antibody can disrupt the stability and drug efficacy of a therapeutic regimen. Generally, an antibody drug is used as a capture and a detector, wherein it is possible to utilize an anti-idiotype antibody as a positive control for the quantification of the antibody drug or a basis for quantification.

In order to address problems associated with anti-drug antibodies, provided is an anti-idiotype antibody capable of specifically binding an idiotope site of an anti-c-Met antibody useful for the detection and/or the quantification of an anti-drug antibody, as defined in the claims.

In another embodiment, there is provided a method for detecting an anti-c-Met antibody, wherein the anti-c-Met antibody comprises:
a CDR-H1 comprising SEQ ID NO: 1;
a CDR-H2 comprising SEQ ID NO: 2;
a CDR-H3 comprising SEQ ID NO: 3;
a CDR-L1 comprising SEQ ID NO: 10;
a CDR-L2 comprising SEQ ID NO: 11; and
a CDR-L3 comprising SEQ ID NO: 13,
wherein the method comprises the steps of: contacting a biological sample with the anti-idiotype antibody or antigen-binding fragment thereof according to the invention; and determining the presence or absence of an antigen-antibody reaction.

In another embodiment, there is provided a method for the quantitative analysis of an anti-drug antibody using anti-idiotype antibody.

More specifically, the invention provides a method for analyzing an anti-drug antibody against the anti-c-Met antibody which comprises a CDR-H1 comprising SEQ ID NO:1; a CDR-H2 comprising SEQ ID NO:2; a CDR-H3 comprising SEQ ID NO:3; a CDR-L1 comprising SEQ ID NO: 10; a CDR-L2 comprising SEQ ID NO:11; and a CDR-L3 comprising SEQ ID NO:13, in a serum isolated from a patient to whom the anti-c-Met antibody has been administered.

In some disclosures, the variable region (e.g., CDRs) of the anti-idiotypic antibody has a binding site that is structurally similar to a portion of an antigen of the subject antibody. Accordingly, when the subject antibody is an antibody for the treatment of a particular disease, the anti-idiotype antibody thereof may be used as a vaccine capable of inducing an immune response by replacing the antigen which is a protein that causes the disease.

Accordingly, in another embodiment, there is provided a vaccine composition for c-Met related diseases including the anti-idiotype antibody against a anti-c-Met antibody.

Hereafter, the present invention will be described in more detail.

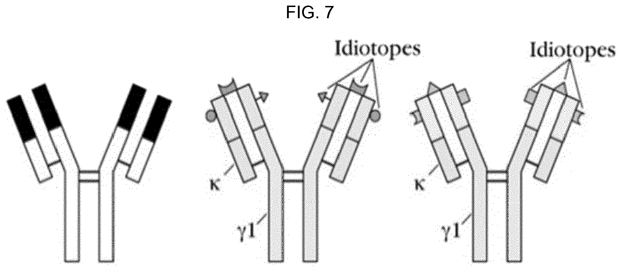

In the leftmost drawing of FIG. 7, anti-c-Met antibody portions shaded in black, which are the variable regions of the antibody that determine antibody specificity, serve as antigenic determinants (i.e., epitopes) with respect to the anti-idiotypic antibody. Within these sites, there exist idiotope sites (see the center and right drawings of FIG. 7) which are individual binding sites for the anti-idiotypic antibody. These portions can distinguish one anti-idiotypic antibody from another antibody (e.g., different anti-idiotypic antibodies can be specific for different idiotopes). Thus, anti-c-Met antibodies, in spite of being capable of reacting with the same antigen, may have different idiotopes from each other, depending on their antigen-binding sites. Accordingly, an anti-idiotypic antibody targeted at such idiotope sites, that is, specifically recognizing and/or binding to the idiotope sites, may acquire antibody specificity.

The anti-idiotype antibody is an antibody capable of recognizing the idiotopes of a c-Met antibody, which refers to an antibody specifically targeted at the idiotopes of the anti-c-Met antibody, or specifically binding to the idiotopes of the anti-c-Met antibody. The idiotopes of the anti-c-Met antibody may be complementarity determining regions (CDR) of the anti-c-Met antibody, variable regions of the anti-c-Met antibody, or partial portions of the variable regions of the anti-c-Met antibody. The CDR may be one or more selected from the group consisting of CDR-H1, CDR-H2, CDR-H3, CDR-L1, CDR-L2, and CDR-L3. The variable regions of the anti-c-Met antibody may be heavy chain variable regions including one or more selected from the group consisting of CDR-H1, CDR-H2, and CDR-H3, light chain variable regions including one or more selected from the group consisting of CDR-L1, CDR-L2, and CDR-L3, or a combination of the heavy chain variable regions and the light chain variable regions. The partial fragments of the heavy chain variable regions and the light chain variable regions of the anti-c-Met antibody may be fragments including 2 or more, 5 or more, or 10 or more contiguous amino acids, for example, from about 2 to about 100, from about 5 to about 100, from about 10 to about 100, from about 2 to about 50, from about 5 to about 50, or from about 10 to about 50 contiguous amino acids within the heavy chain variable regions or the light chain variable regions of the anti-c-Met antibody. The partial fragments of the heavy chain variable regions and the light chain variable regions of the anti-c-Met antibody may be fragments including 2 or more, 5 or more, or 10 or more contiguous amino acids, for example, from about 2 to about 100, from about 5 to about 100, from about 10 to about 100, from about 2 to about 50, from about 5 to about 50, or from about 10 to about 50 contiguous amino acids within the variable regions containing one or more CDR or CDR fragments. The CDR fragments may be consecutive or non-consecutive 2 or more, or 5 or more amino acids within the CDR. Therefore, the idiotopes of the anti-c-Met antibody may be from about 2 to about 100, from about 5 to about 100, from about 10 to about 100, from about 2 to about 50, from about 5 to about 50, or from about 10 to about 50 contiguous amino acids containing one or more CDR or one or more CDR fragments within the heavy chain variable regions or the light chain variable regions of the anti-c-Met antibody. In another embodiment, the idiotopes may be a single amino acid which is located at the variable regions of the anti-c-Met antibody, for example, CDR sites.

As used herein, the phrase "contiguous amino acids" may refer to contiguous amino acid residues on the primary, secondary, or tertiary structure of a protein, wherein the contiguous amino acid residues on the secondary or tertiary structure of a protein may be consecutive or non-consecutive on the primary structure (amino acid sequence) of a protein.

In one embodiment, the anti c-Met antibody may be any antibody or antigen-binding fragment that acts on c-Met to induce c-Met intracellular internalization and degradation. The anti c-Met antibody be any antibody capable of recognizing a specific region of c-Met, e.g., a specific region in the SEMA domain, as an epitope.

"c-Met" or "c-Met protein" refers to a receptor tyrosine kinase (RTK) which binds hepatocyte growth factor (HGF). c-Met may be derived from any species, particularly a mammal, for instance, primates such as human c-Met (e.g., NP_000236), monkey c-Met (e.g., Macaca mulatta, NP_001162100), or rodents such as mouse c-Met (e.g., NP_032617.2), rat c-Met (e.g., NP_113705.1), and the like. The c-Met protein may include a polypeptide encoded by the nucleotide sequence identified as GenBank Accession Number NM_000245, a polypeptide having the amino acid sequence identified as GenBank Accession Number NP_000236 or extracellular domains thereof. The receptor tyrosine kinase c-Met participates in various mechanisms, such as cancer incidence, metastasis, migration of cancer cell, invasion of cancer cell, angiogenesis, and the like.

c-Met, a receptor for hepatocyte growth factor (HGF), may be divided into three portions: extracellular, transmembrane, and intracellular. The extracellular portion is composed of an α-subunit and a β-subunit which are linked to each other through a disulfide bond, and contains a SEMA domain responsible for binding HGF, a PSI domain (plexin-semaphorins-integrin identity/homology domain) and an IPT domain (immunoglobulin-like fold shared by plexins and transcriptional factors domain). The SEMA domain of c-Met protein may have the amino acid sequence of SEQ ID NO: 79, and is an extracellular domain that functions to bind HGF. A specific region of the SEMA domain, that is, a region having the amino acid sequence of SEQ ID NO: 71, which corresponds to a range from amino acid residues 106 to 124 of the amino acid sequence of the SEMA domain (SEQ ID NO: 79), is a loop region between the second and the third propellers within the epitopes of the SEMA domain. This region acts as an epitope for the anti c-Met antibody.

The term "epitope," as used herein, refers to an antigenic determinant, a part of an antigen recognized by an antibody. In one embodiment, the epitope may be a region comprising 5 or more contiguous amino acid residues within the SEMA domain (SEQ ID NO: 79) of c-Met protein, for instance, 5 to 19 consecutive amino acid residues within the amino acid sequence of SEQ ID NO: 71. For example, the epitope may be a polypeptide including 5 to 19 contiguous amino acids selected from among partial combinations of the amino acid sequence of SEQ ID NO: 71, wherein the polypeptide includes the amino sequence of SEQ ID NO: 73 (EEPSQ) serving as an essential element for the epitope. For example, the epitope may be a polypeptide comprising, consisting essentially of, or consisting of the amino acid sequence of SEQ ID NO: 71, SEQ ID NO: 72, or SEQ ID NO: 73.

The epitope including the amino acid sequence of SEQ ID NO: 72 corresponds to the outermost part of the loop between the second and third propellers within the SEMA domain of a c-Met protein. The epitope including the amino acid sequence of SEQ ID NO: 73 is a site to which the antibody or antigen-binding fragment according to one embodiment most specifically binds.

Thus, the anti c-Met antibody may specifically bind to an epitope which has from about 5 to about 19 contiguous amino acids selected from among partial combinations of the amino acid sequence of SEQ ID NO: 71, including SEQ ID NO: 73 as an essential element. For example, the anti c-Met antibody may specifically bind to an epitope including the amino acid sequence of SEQ ID NO: 71, SEQ ID NO: 72, or SEQ ID NO: 73.

The anti-idiotype antibody or antigen-binding fragment thereof according to the invention specifically binds to an anti-c-Met antibody or an antibody fragment of the anti-c-Met antibody, wherein the anti-c-Met antibody or the antibody fragment of the anti-c-Met antibody comprises a CDR-H1 comprising SEQ ID NO: 1; a CDR-H2 comprising SEQ ID NO: 2; a CDR-H3 comprising SEQ ID NO: 3; a CDR-L1 comprising SEQ ID NO: 10; a CDR-L2 comprising SEQ ID NO: 11; and a CDR-L3 comprising SEQ ID NO: 13, as defined in the appended claims.

In one disclosure, the anti c-Met antibody or an antigen-binding fragment thereof may include:
at least one heavy chain complementarity determining region (CDR) selected from the group consisting of (a) a CDR-H1 including the amino acid sequence of SEQ ID NO: 4; (b) a CDR-H2 including the amino acid sequence of SEQ ID NO: 5, SEQ ID NO: 2, or an amino acid sequence including 8-19 consecutive amino acids within SEQ ID NO: 2 including amino acid residues from the 3^{rd} to 10^{th} positions of SEQ ID NO: 2; and (c) a CDR-H3 including the amino acid sequence of SEQ ID NO: 6, SEQ ID NO: 85, or an amino acid sequence including 6-13 consecutive amino acids within SEQ ID NO: 85 including amino acid residues from the 1^{st} to 6^{th} positions of SEQ ID NO: 85, or a heavy chain variable region including the at least one heavy chain complementarity determining region;
at least one light chain complementarity determining region (CDR) selected from the group consisting of (a) a CDR-L1 including the amino acid sequence of SEQ ID NO: 7, (b) a CDR-L2 including the amino acid sequence of SEQ ID NO: 8, and (c) a CDR-L3 including the amino acid sequence of SEQ ID NO: 9, SEQ ID NO: 86, or an amino acid sequence including 9-17 consecutive amino acids within SEQ ID NO: 89 including amino acid residues from the 1^{st} to 9^{th} positions of SEQ ID NO: 89, or a light chain variable region including the at least one light chain complementarity determining region;
a combination of the at least one heavy chain complementarity determining region and at least one light chain complementarity determining region; .or
a combination of the heavy chain variable region and the light chain variable region.

Herein, the amino acid sequences of SEQ ID NOS: 4 to 9 are respectively represented by following Formulas I to VI, below:
Formula I
   Xaa₁-Xaa₂-Tyr-Tyr-Met-Ser (SEQ ID NO: 4),
   wherein Xaa₁ is absent or Pro or Ser, and Xaa₂ is Glu or Asp,
Formula II
   Arg-Asn-Xaa₃-Xaa₄-Asn-Gly-Xaa₅-Thr (SEQ ID NO: 5),
   wherein Xaa₃ is Asn or Lys, Xaa₄ is Ala or Val, and Xaa₅ is Asn or Thr,
Formula III
   Asp-Asn-Trp-Leu-Xaa₆-Tyr (SEQ ID NO: 6),
   wherein Xaa₆ is Ser or Thr,
Formula IV
   Lys-Ser-Ser-Xaa₇-Ser-Leu-Leu-Ala-Xaa₈-Gly-Asn-Xaa₉-Xaa₁₀-Asn-Tyr-Leu-Ala (SEQ ID NO: 7)
   wherein Xaa₇ is His, Arg, Gln, or Lys, Xaa₈ is Ser or Trp, Xaag is His or Gln, and Xaa₁₀ is Lys or Asn,
Formula V
   Trp-Xaa₁₁-Ser-Xaa₁₂-Arg-Val-Xaa₁₃ (SEQ ID NO: 8)
   wherein Xaa₁₁ is Ala or Gly, Xaa₁₂ is Thr or Lys, and Xaa₁₃ is Ser or Pro, and
Formula VI
   Xaa₁₄-Gln-Ser-Tyr-Ser-Xaa₁₅-Pro-Xaa₁₆-Thr (SEQ ID NO: 9)
   wherein Xaa₁₄ is Gly, Ala, or Gin, Xaa₁₅ is Arg, His, Ser, Ala, Gly, or Lys, and Xaa₁₆ is Leu, Tyr, Phe, or Met.

In further disclosures, the COR-H1 may have an amino acid sequence selected from the group consisting of SEQ ID NOS: 22, 23, and 24. The CDR-H2 may have an amino acid sequence selected from the group consisting of SEQ ID NOS: 25, and 26. The CDR-H3 may have an amino acid sequence selected from the group consisting of SEQ ID NOS: 27, 28, and 85.

The CDR-L1 may have an amino acid sequence selected from the group consisting of SEQ ID NOS: 29, 30, 31, 32, 33, and 106. The CDR-L2 may have an amino acid sequence selected from the group consisting of SEQ ID NOS: 34, 35, and 36. The CDR-L3 may have an amino acid sequence selected from the group consisting of SEQ ID NOS: 12, 14, 15, 16, 37, 86, and 89.

In further disclosures, the anti-c-Met antibody or antigen-binding fragment may include a heavy variable region comprising a polypeptide (CDR-H1) including an amino acid sequence selected from the group consisting of SEQ ID NOS: 22, 23, and 24, a polypeptide (CDR-H2) including an amino acid sequence selected from the group consisting of SEQ ID NOS: 25, and 26, and a polypeptide (CDR-H3) including an amino acid sequence selected from the group consisting of SEQ ID NOS: 27, 28, and 85; and a light variable region comprising a polypeptide (CDR-L1) including an amino acid sequence selected from the group consisting of SEQ ID NOS: 29, 30, 31, 32, 33 and 106, a polypeptide (CDR-L2) including an amino acid sequence selected from the group consisting of SEQ ID NOS: 34, 35, and 36, and a polypeptide (CDR-L3) including an amino acid sequence selected from the group consisting of SEQ ID NOS 12, 14, 15, 16, 37, 86, and 89.

In further disclosures of the anti c-Met antibody or antigen-binding fragment, the variable domain of the heavy chain has the amino acid sequence of SEQ ID NO: 17, 87, 90, 91, 92, 93, or 94 and the variable domain of the light chain has the amino acid sequence of SEQ ID NO: 18, 19, 20, 21, 88, 95, 96, 97, 98, 99, or 107.

In one specific embodiment, the variable domain of the heavy chain of the anti-c-Met antibody or antigen-binding fragment has the amino acid sequence of SEQ ID NO: 74 and the variable domain of the light chain of the anti-c-Met antibody or antigen-binding fragment has the amino acid sequence of SEQ ID NO: 75.

Animal-derived antibodies produced by immunizing non-immune animals with a desired antigen generally invoke immunogenicity when injected to humans for the purpose of medical treatment, and thus chimeric antibodies have been developed to inhibit such immunogenicity. Chimeric antibodies are prepared by replacing constant regions of animal-derived antibodies that cause an anti-isotype response with constant regions of human antibodies by genetic engineering. Chimeric antibodies are considerably improved in an anti-isotype response compared to animal-derived antibodies, but animal-derived amino acids still have variable regions, so that chimeric antibodies have side effects with respect to a potential anti-idiotype response. Humanized antibodies have been developed to reduce such side effects. Humanized antibodies are produced by grafting complementarity determining regions (CDR) which serve an important role in antigen-binding in variable regions of chimeric antibodies into a human antibody framework.

The most important aspect of CDR grafting to produce humanized antibodies is choosing the optimized human antibodies for accepting CDRs of animal-derived antibodies. Antibody databases, analysis of antibody crystal structures, and technology for molecule modeling are used. However, even when the CDRs of animal-derived antibodies are grafted to the most optimized human antibody framework, amino acids positioned in a framework of the animal-derived CDRs affecting antigen-binding are present. Therefore, in many cases, antigen-binding affinity is not maintained, and thus application of additional antibody engineering technology for recovering the antigen-binding affinity is necessary.

The anti c-Met antibodies may be a mouse-derived antibody, a mouse-human chimeric antibody, a humanized antibody, or a human antibody. The antibodies or antigen-binding fragments thereof may be isolated from (that is, not originally present in) a living body or non-naturally occurring. The antibody or antigen-binding fragment thereof may be monoclonal or synthetic.

An intact antibody includes two full-length light chains and two full-length heavy chains, in which each light chain is linked to a heavy chain by disulfide bonds. The antibody has a heavy chain constant region and a light chain constant region. The heavy chain constant region is of a gamma (γ), mu (µ), alpha (α), delta (δ), or epsilon (ε) type, which may be further categorized as gamma 1 (γ1), gamma 2(γ2), gamma 3(γ3), gamma 4(γ4), alpha 1(α1), or alpha 2(α2). The light chain constant region is of either a kappa (κ) or lambda (λ) type.

As used herein, the term "heavy chain" refers to full-length heavy chain, and fragments thereof, including a variable region V_{H} that includes amino acid sequences sufficient to provide specificity to antigens, and three constant regions, C_{H1}, C_{H2}, and C_{H3}, and a hinge. The term "light chain" refers to a full-length light chain and fragments thereof, including a variable region V_{L} that includes amino acid sequences sufficient to provide specificity to antigens, and a constant region C_{L}.

The term "complementarity determining region (CDR)" refers to an amino acid sequence found in a hyper variable region of a heavy chain or a light chain of immunoglobulin. The heavy and light chains may respectively include three CDRs (CDRH1, CDRH2, and CDRH3; and CDRL1, CDRL2, and CDRL3). The CDR may provide contact residues that play an important role in the binding of antibodies to antigens or epitopes. The terms "specifically binding" and "specifically recognized" are well known to one of ordinary skill in the art, and indicate that an antibody and an antigen specifically interact with each other to lead to an immunological activity.

The term "antigen-binding fragment" used herein refers to fragments of an intact immunoglobulin including portions of a polypeptide including antigen-binding regions having the ability to specifically bind to the antigen. In a particular embodiment, the antigen-binding fragment may be scFv, (scFv)₂, scFvFc, Fab, Fab', or F(ab')₂, but is not limited thereto.

Among the antigen-binding fragments, Fab includes light chain and heavy chain variable regions, a light chain constant region, and a first heavy chain constant region C_{H1}, and has one antigen-binding site.

The Fab' fragment differs from the Fab fragment, in that Fab' includes a hinge region with at least one cysteine residue at the C-terminal of C_{H1}.

The F(ab')₂ antibody is formed through disulfide bridging of the cysteine residues in the hinge region of the Fab' fragment.

Fv is the smallest antibody fragment with only a heavy chain variable region and a light chain variable region. Recombination techniques of generating the Fv fragment are widely known in the art.

Two-chain Fv includes a heavy chain variable region and a light chain region which are linked by a non-covalent bond. Single-chain Fv generally includes a heavy chain variable region and a light chain variable region which are linked by a covalent bond via a peptide linker or linked at the C-terminals to have a dimer structure like the two-chain Fv. The peptide linker may be the same as described in the above, for example, those having the amino acid length of 1 to 100, 2 to 50, particularly 5 to 25, and any kinds of amino acids may be included without any restrictions.

The antigen-binding fragments may be attainable using protease (for example, the Fab fragment may be obtained by restricted cleavage of a whole antibody with papain, and the F(ab')₂ fragment may be obtained by cleavage with pepsin), or may be prepared by using a genetic recombination technique.

The term "hinge region," as used herein, refers to a region between CH1 and CH2 domains within the heavy chain of an antibody which functions to provide flexibility for the antigen-binding site.

When an animal antibody undergoes a chimerization process, the IgG1 hinge of animal origin is replaced with a human IgG1 hinge or IgG2 hinge while the disulfide bridges between two heavy chains are reduced from three to two in number. In addition, an animal-derived IgG1 hinge is shorter than a human IgG1 hinge. Accordingly, the rigidity of the hinge is changed. Thus, a modification of the hinge region may bring about an improvement in the antigen-binding efficiency of the humanized antibody. The modification of the hinge region through amino acid deletion, addition, or substitution is well-known to those skilled in the art.

In one embodiment, the anti-c-Met antibody or antigen-binding fragment thereof may be modified by the deletion, insertion, addition, or substitution of at least one amino acid residue on the amino acid sequence of the hinge region so that it exhibit enhanced antigen-binding efficiency. For example, the antibody may include a hinge region including the amino acid sequence of SEQ ID NO: 100(U7-HC6), 101(U6-HC7), 102(U3-HC9), 103(U6-HC8), or 104(U8-HC5), or a hinge region including the amino acid sequence of SEQ ID NO: 105 (non-modified human hinge). In particular, the hinge region has the amino acid sequence of SEQ ID NO: 100 or 101.

In one embodiment, the anti c-Met antibody may be a monoclonal antibody. The monoclonal antibody may be produced by the hybridoma cell line deposited with Accession No. KCLRF-BP-00220, which binds specifically to the extracellular region of c-Met protein (refer to Korean Patent Publication No. 2011-0047698). The anti-c-Met antibody may include all the antibodies defined in Korean Patent Publication No. 2011-0047698.

In the anti-c-Met antibody, the remaining portion of the light chain and the heavy chain, excluding the CDRs, the light chain variable region, and the heavy chain variable region as defined above, that is the light chain constant region and the heavy chain constant region, may be those from any subtype of immunoglobulin (e.g., IgG1, IgG2, and the like).

By way of further example, the anti-c-Met antibody or the antibody fragment may include:
a heavy chain including the amino acid sequence selected from the group consisting of the amino acid sequence of SEQ ID NO: 62 (wherein the amino acid sequence from amino acid residues from the 1^{st} to 17^{th} positions is a signal peptide), or the amino acid sequence from the 18^{th} to 462^{nd} positions of SEQ ID NO: 62, the amino acid sequence of SEQ ID NO: 64 (wherein the amino acid sequence from the 1^{st} to 17^{th} positions is a signal peptide), the amino acid sequence from the 18^{th} to 461^{st} positions of SEQ ID NO: 64, the amino acid sequence of SEQ ID NO: 66 (wherein the amino acid sequence from the 1^{st} to 17^{th} positions is a signal peptide), and the amino acid sequence from the 18^{th} to 460^{th} positions of SEQ ID NO: 66; and
a light chain including the amino acid sequence selected from the group consisting of the amino acid sequence of SEQ ID NO: 68 (wherein the amino acid sequence from the 1^{st} to 20^{th} positions is a signal peptide), the amino acid sequence from the 21^{st} to 240^{th} positions of SEQ ID NO: 68, the amino acid sequence of SEQ ID NO: 70 (wherein the amino acid sequence from the 1^{st} to 20^{th} positions is a signal peptide), and the amino acid sequence from the 21^{st} to 240^{th} positions of SEQ ID NO: 70.

For example, the anti-c-Met antibody may be selected from the group consisting of:
an antibody including a heavy chain including the amino acid sequence of SEQ ID NO: 62 or the amino acid sequence from the 18^{th} to 462^{nd} positions of SEQ ID NO: 62 and a light chain including the amino acid sequence of SEQ ID NO: 68 or the amino acid sequence from the 21^{st} to 240^{th} positions of SEQ ID NO: 68;
an antibody including a heavy chain including the amino acid sequence of SEQ ID NO: 64 or the amino acid sequence from the 18^{th} to 461^{st} positions of SEQ ID NO: 64 and a light chain including the amino acid sequence of SEQ ID NO: 68 or the amino acid sequence from the 21^{st} to 240^{th} positions of SEQ ID NO: 68;
an antibody including a heavy chain including the amino acid sequence of SEQ ID NO: 66 or the amino acid sequence from the 18^{th} to 460^{th} positions of SEQ ID NO: 66 and a light chain including the amino acid sequence of SEQ ID NO: 68 or the amino acid sequence from the 21^{st} to 240^{th} positions of SEQ ID NO: 68;
an antibody including a heavy chain including the amino acid sequence of SEQ ID NO: 62 or the amino acid sequence from the 18^{th} to 462^{nd} positions of SEQ ID NO: 62 and a light chain including the amino acid sequence of SEQ ID NO: 70 or the amino acid sequence from the 21^{st} to 240^{th} positions of SEQ ID NO: 70;
an antibody including a heavy chain including the amino acid sequence of SEQ ID NO: 64 or the amino acid sequence from the 18^{th} to 461^{st} positions of SEQ ID NO: 64 and a light chain including the amino acid sequence of SEQ ID NO: 70 or the amino acid sequence from the 21^{st} to 240^{th} positions of SEQ ID NO: 70; and
an antibody including a heavy chain including the amino acid sequence of SEQ ID NO: 66 or the amino acid sequence from the 18^{th} to 460^{th} positions of SEQ ID NO: 66 and a light chain including the amino acid sequence of SEQ ID NO: 70 or the amino acid sequence from the 21^{st} to 240^{th} positions of SEQ ID NO: 70.

The polypeptide of SEQ ID NO: 70 is a light chain including human kappa (κ) constant region, and the polypeptide with the amino acid sequence of SEQ ID NO: 68 is a polypeptide obtained by replacing histidine at position 62 (corresponding to position 36 of SEQ ID NO: 68 according to kabat numbering) of the polypeptide with the amino acid sequence of SEQ ID NO: 70 with tyrosine. The production yield of the antibodies may be increased by the replacement. The polypeptide with the amino acid sequence of SEQ ID NO: 108 is a polypeptide obtained by replacing serine at position 32 (position 27e according to kabat numbering in the amino acid sequence from amino acid residues 21 to 240 of SEQ ID NO: 68; positioned within CDR-L1) with tryptophan. By such replacement, antibodies and antibody fragments including such sequences exhibits increased activities, such as c-Met biding affinity, c-Met degradation activity, Akt phosphorylation inhibition, and the like.

In another disclosure, the anti c-Met antibody may include a light chain complementarity determining region including the amino acid sequence of SEQ ID NO: 106, a light chain variable region including the amino acid sequence of SEQ ID NO: 107, or a light chain including the amino acid sequence of SEQ ID NO: 108.

According to the invention, the anti-idiotype antibody or antigen-binding fragment thereof, that specifically binds to an anti-c-Met antibody or an antibody fragment of the anti-c-Met antibody, as defined in the appended claims, comprises:
(i) a CDR-H1 of SEQ ID NO: 115, a CDR-H2 of SEQ ID NO: 125, a CDR-H3 of SEQ ID NO: 139, a CDR-L1 of SEQ ID NO: 155, a CDR-L2 of SEQ ID NO: 172, and a CDR-L3 of SEQ ID NO: 188; or
(ii) a CDR-H1 of SEQ ID NO: 116, a CDR-H2 of SEQ ID NO: 126, a CDR-H3 of SEQ ID NO: 140, a CDR-L1 of SEQ ID NO: 156, a CDR-L2 of SEQ ID NO: 173, and a CDR-L3 of SEQ ID NO: 189.

Specific examples of amino acid sequences of the heavy chain complementarity determining regions (CDRs) and the light chain complementarity determining regions of the anti-idiotypic antibody are set forth in the following Table 1 and Table 2. The invention is defined in the appended claims.

**Table 1**

| Heavy Chain Complementarity Determining Regions (CDR) | | |
|---|---|---|
| CDR-H1 | CDR-H2 | CDR-H3 |
| DYYMS (SEQ ID NO: 115) | GIYSSSSNIYYADSVKG (SEQ ID NO: 125) | KALGNQENEPTSYSNGMDV (SEQ ID NO:139) |
| NYAMS (SEQ ID NO: 116) | SISSSGGNTYYADSVKG (SEQ ID NO:126) | KYHSVFDY (SEQ ID NO:140) |
| DYDMS (SEQ ID NO: 117) | LISYGGSNTYYADSVKG (SEQ ID NO:127) | KFRSEFNENEPSSYYGMDV (SEQ ID NO:141) |
| GYDMS (SEQ ID NO: 118) | GISHGDGNIYYADSVKG (SEQ ID NO: 128) | KVGLLFVQEEPSYYNAMDV (SEQ ID NO: 142) |
| DYDMS (SEQ ID NO: 117) | SISYGGGSIYYADSVKG (SEQ ID NO: 129) | RDAAYFDY (SEQ ID NO: 143) |
| GYDMS (SEQ ID NO: 118) | GISYNGGSKYYADSVKG (SEQ ID NO: 130) | KYLLPVLEEPGYSADGMDV (SEQ ID NO: 144) |
| DYYMS (SEQ ID NO: 115) | AISHSSGNTYYADSVKG (SEQ ID NO: 131) | KHLGAQSDEPDSSSNGMDV (SEQ ID NO: 145) |
| NYAMS (SEQ ID NO: 116) | AIYPGGGNTYYADSVKG (SEQ ID NO: 132) | KSLSTHSVDEPSSDNAMDV (SEQ ID NO: 146) |
| DYAMS (SEQ ID NO: 119) | AISSGDGNTYYADSVKG (SEQ ID NO: 133) | RYLGTTSDEPASYSNGMDV (SEQ ID NO: 147) |
| DYAMS (SEQ ID NO: 119) | SIYPDDGNTYYADSVKG (SEQ ID NO: 134) | KYRLVDRWEEPSSDYGMDV (SEQ ID NO: 148) |
| NYSMS (SEQ ID NO: 120) | SISSSGGNTYYADSVKG (SEQ ID NO: 126) | RVHLYFDY (SEQ ID NO: 149) |
| SYAMH (SEQ ID NO: 121) | VISYDGSNKYYADSVKG (SEQ ID NO: 135) | REDNTRYFEEPNYYGMDV (SEQ ID NO: 150) |
| SYAIS (SEQ ID NO: 122) | GIIPIFGTANYAQKFQG (SEQ ID NO: 138) | RDRNSYYEEPMYYFDY (SEQ ID NO: 151) |

| | | |
|---|---|---|
| SYAIS (SEQ ID NO: 122) | GIIPIFGTANYAQKFQG (SEQ ID NO: 138) | RDRNSYYEEPMYYFDY (SEQ ID NO: 151) |
| SYGMH (SEQ ID NO: 123) | VISYDGSNKYYADSVKG (SEQ ID NO: 135) | RDLVADDYGDYGTVDY (SEQ ID NO: 152) |
| SYAMS (SEQ ID NO: 124) | AISGSGGSTYYADSVEG (SEQ ID NO: 136) | KERLEEPGFFDY (SEQ ID NO: 153) |
| SYAMS (SEQ ID NO: 124) | AISGSGGSTYYADSVKG (SEQ ID NO: 137) | ARGGGYSYGYEEPYYYYGMD V (SEQ ID NO: 154) |

**Table 2**

| Light Chain Complementarity Determining Regions (CDR) | | |
|---|---|---|
| CDR-L1 | CDR-L2 | CDR-L3 |
| SGSSSNIGNNSVY (SEQ ID NO: 155) | SDSQRPS(SEQ ID NO: 172) | GTWDYSLNG(SEQ ID NO:188) |
| SGSSSNIGNNYVY (SEQ ID NO: 156) | ANNQRPS(SEQ ID NO: 173) | GAWDDSLSG(SEQ ID NO:189) |
| SGSSSNIGNNDVT (SEQ ID NO: 157) | SDSNRPS(SEQ ID NO: 174) | GTWDSSLSA(SEQ ID NO:190) |
| TGSSSNIGSNNVT (SEQ ID NO: 158) | SNSHRPS (SEQ ID NO: 175) | GTWDDSLNG (SEQ ID NO: 191) |
| SGSSSNIGNNSVN (SEQ ID NO: 159) | ANNNRPS (SEQ ID NO: 176) | GAWDASLNG (SEQ ID NO: 192) |
| TGSSSNIGSNYVS (SEQ ID NO: 160) | SDSNRPS (SEQ ID NO: 177) | ATWDASLSA (SEQ ID NO: 193) |
| TGSSSNIGNNDVY (SEQ ID NO: 161) | SDSNRPS (SEQ ID NO: 177) | GTWDDSLNG (SEQ ID NO: 191) |
| TGSSSNIGSNSVS (SEQ ID NO: 162) | DDSNRPS (SEQ ID NO: 178) | ASWDYSLNA (SEQ ID NO: 194) |
| SGSSSNIGSNDVY (SEQ ID NO: 163) | SDNNRPS (SEQ ID NO: 179) | GAWDDSLSG (SEQ ID NO: 189) |
| TGSSSNIGSNNVN (SEQ ID NO: 164) | ADSQRPS (SEQ ID NO: 180) | GSWDSSLSG (SEQ ID NO: 195) |
| SGSSSNIGSNSVN (SEQ ID NO: 165) | SDSHRPS (SEQ ID NO: 181) | GSWDDSLSG (SEQ ID NO: 196) |
| TGSSSNIGAAYEVH (SEQ ID NO: 166) | DTSNRPS (SEQ ID NO: 182) | AAWDDSLNG (SEQ ID NO: 197) |
| SGDKLGDRYVF (SEQ ID NO: 167) | DDSDRPS (SEQ ID NO: 183) | QVWDSVNDH (SEQ ID NO: 198) |
| SGSGSNIGSNAVN (SEQ ID NO: 168) | SNNQRPS (SEQ ID NO: 184) | AAWDDSLNG (SEQ ID NO: 197) |
| GGNNIATKGVH (SEQ ID NO: 169) | DDSGRPS (SEQ ID NO: 185) | QLWDGRSDQ (SEQ ID NO: 199) |
| TGTSSDVGGYNYVS (SEQ ID NO: 170) | EVSNRPS (SEQ ID NO: 186) | SSYTTDNA (SEQ ID NO: 200) |
| KSSQSLLNSGNQKNDLA (SEQ ID NO: 171) | GASTRES (SEQ ID NO: 187) | QNDHSYP (SEQ ID NO: 201) |

In one specific embodiment, the anti-idiotype antibody may include a heavy chain variable region containing an amino acid sequence selected from the group consisting of SEQ ID NO: 202 and SEQ ID NO: 203; a light chain variable region containing an amino acid sequence selected from the group consisting of SEQ ID NO: 236 and SEQ ID NO: 237; or a combination of the heavy chain variable region and the light chain variable region.

In a particular embodiment, the anti-idiotype antibody or antigen-binding fragment thereof that specifically binds to an idiotope site of an anti-c-Met antibody may be a mouse-derived antibody, a mouse-human chimeric antibody, a humanized antibody, or a human antibody. The antibody or antigen-binding fragment thereof may be isolated from (that is, not originally present in) a living body or non-naturally occurring. The antibody or antigen-binding fragment thereof may be monoclonal or synthetic.

In a particular embodiment, the anti-idiotype antibody that specifically binds to an idiotope site of an anti-c-Met antibody may be in the form of an antigen-binding fragment selected from the group consisting of scFv, (scFv)₂, scFvFc, Fab, Fab', and F(ab')₂, as well as in the form of a complete antibody (e.g., a full IgG type, etc.). A definition for the antigen-binding fragment is as described above in relation to the anti-c-Met antibody.

The anti-idiotype antibody that specifically binds to an idiotope site of an anti-c-Met antibody may include a heavy chain constant region and/or a light chain constant region. The heavy chain constant region and/or the light chain constant region may be derived from immunoglobulins of humans or animals except humans (e.g., mice), for example, hIgG1, hIgG2, hIgG3, hIgG4, mIgG1, mIgG2a, mIgG2b, mIgG3, mIgM, etc.

The anti-idiotype antibody that specifically binds to an idiotope site of an anti-c-Met antibody may include a hinge, which may be derived from immunoglobulins of humans or animals except humans (e.g., mice), for example, IgG1, IgG2, etc., which may be identical to or different from that from which the heavy chain constant region is derived.

The anti-idiotype antibody that specifically binds to an idiotope site of an anti-c-Met antibody may be monoclonal antibodies. The monoclonal antibodies may be prepared by a well-known method in the art. For instance, they may be prepared using a phage display technique.

In addition, individual monoclonal antibodies may be screened on the basis of a binding potential to the anti-c-Met antibody using a typical ELISA (Enzyme-Linked ImmunoSorbent Assay) format. An inhibitory activity may be examined through functionality analysis such as Competitive ELISA for examining molecular interaction to an assembled body or functionality analysis such as a cell-based assay. Then, with regard to monoclonal antibodies selected on the basis of their strong inhibitory activities, their individual affinity (Kd value) or binding affinity to the anti-c-Met antibody is examined.

In one embodiment, the affinities (Kd values) of the anti-idiotype antibodies against the anti-c-Met antibody to the anti-c-Met antibody or may be about 50 nM or less, for example, from about 0.001 to about 50 nM, or from about 0.01 to about 40 nM.

Since the anti-idiotype antibody that specifically binds to an idiotope site of an anti-c-Met antibody or an antibody fragment of the anti-c-Met antibody an antigen-binding fragment thereof specifically binds to the anti-c-Met antibody, the anti-c-Met antibody may be detected using the anti-idiotype antibody or an antigen-binding fragment thereof. The detection of the anti-c-Met antibody using the anti-idiotype antibodies that specifically bind to an idiotope site of an anti-c-Met antibody or the antigen-binding fragments thereof may be applied to monitor a half-life of the antibody, an effective concentration thereof, the remaining concentration, success or failure in targeting a target organ, and the like.

Accordingly, one embodiment provides a composition for detecting an anti-c-Met antibody including the anti-idiotype antibody that specifically binds to an idiotope site of an anti-c-Met antibody or the antigen-binding fragment thereof.

Another embodiment provides a method for detecting an anti-c-Met antibody, wherein the anti-c-Met antibody comprises:
a CDR-H1 comprising SEQ ID NO: 1;
a CDR-H2 comprising SEQ ID NO: 2;
a CDR-H3 comprising SEQ ID NO: 3;
a CDR-L1 comprising SEQ ID NO: 10;
a CDR-L2 comprising SEQ ID NO: 11; and
a CDR-L3 comprising SEQ ID NO: 13,
wherein the method comprises the steps of: contacting a biological sample with the anti-idiotype antibody or antigen-binding fragment thereof according to the invention; and determining the presence or absence of an antigen-antibody reaction.

Another embodiment provides a use of the anti-idiotype antibody or antibody fragment thereof according to the invention for use in detecting the anti-c-Met antibody.

The use of the anti-idiotype antibody that specifically binds to an idiotope site of an anti-c-Met antibody and the antigen-binding fragment thereof for detecting the anti-c-Met antibody may be applied to monitor the concentration of the anti-c-Met antibody in body after the administration thereof into the body, success or failure in targeting at a target organ, degree of targeting, etc.

In one particular embodiment, the method for detecting an anti-c-Met antibody may be performed by immunoassay using anti-idiotype antibodies as described above as a capture agent and a detector. The anti-idiotype antibodies used as the capture agent and detector may be identical or different. The anti-idiotype antibodies used as the capture agent and detector may be probed with a different or identical probe. The probe may be selected from any detectable label or tag, e.g., fluorescence substances and luminescence substances, which are ordinarily used in immunoassay.

The biological sample may be selected from the group consisting of cells, tissues, body fluids, and the like obtained (isolated) from a subject. For example, the sample may be a serum, for example, a serum isolated from a subject. The subject may include mammals, including primates such as humans and monkeys and rodents such as mice and rats and for example, the subject may be a patient to whom an anti-c-Met antibody is administered.

The step of determining the presence/absence of an antigen-antibody reaction may be performed through various methods known in the art. For instance, it may be measured through an ordinary enzyme reaction, fluorescence, luminescence and/or radiation detection and in particular, it may be measured by a method selected from the group consisting of immunochromatography, immunohistochemistry, enzyme linked immunosorbent assay (ELISA), radioimmunoassay (RIA), enzyme immunoassay (EIA), fluorescence immunoassay (FIA), luminescence immunoassay (LIA) and western blotting, but is not limited thereto.

The presence/absence of the anti-c-Met antibody in the biological sample and/or the concentration of the anti-c-Met antibody may be determined by the method for detecting the anti-c-Met antibody as described above. When the subject from whom the biological sample is obtained is a patient to whom the anti-c-Met antibody has been administered, the remaining amount of the administered anti-c-Met antibody and/or distribution location thereof can be checked.

Meanwhile, if the occurring frequency of an anti-drug antibody against the anti-c-Met antibody should be measured, an anti-idiotype antibody may be used as a positive control and applied as a standard sample for quantification. After the anti-c-Met antibody is administered via an intravenous injection to a subject to be tested, a serum may be obtained therefrom after a certain period of time, followed by analysis by an enzyme-linked immunosorbent assay. A change in absorption according to the concentrations of the anti-idiotype antibody within the serum is measured, and a formula between the concentration and absorption is thus derived. Next, a drug to be analyzed is (intravenously) administered to a subject, then a serum is obtained from the subject at a desired time, and is then diluted at a certain ratio and then, the absorption thereof is measured on the same plate by the same methods as above. The absorption results may be applied to the formula regarding absorption change according to the concentrations of the anti-idiotype antibody that specifically binds to an idiotope site of an anti-c-Met antibody to quantify the concentration of the antibody in the serum. The concentration may be referred to as a concentration of an anti-drug antibody (ADA) against a test drug. Thus, the amount of a desired anti-drug antibody at a desired time may be measured by back calculation using the absorption change results according to the concentrations of the anti-idiotype antibody that specifically binds to an idiotope site of an anti-c-Met antibody.

Another embodiment of the present invention provides an analysis method of an anti-drug antibody using the anti-idiotype antibody that specifically binds to an idiotope site of an anti-c-Met antibody. For example, the embodiment can be a quantification analysis method. More particularly, the analysis method of an anti-drug antibody includes measuring the absorption of a serum isolated from a patient to whom a test drug has been intravenously administered; and comparing the obtained absorption results with the absorption change of an anti-idiotype antibody in a serum isolated from a patient to whom an anti-c-Met antibody has been administered. The step of measuring absorption of the serum isolated from a patient to whom a test drug has been intravenously administered may be carried out by the same conditions and methods as the absorption measurement of the anti-idiotype antibody in the serum isolated from the patient to whom the anti-c-Met antibody has been administered. The patient may include mammals, including primates such as humans and monkeys and rodents such as mice and rats and for example, the subject may be a patient to whom an anti-c-Met antibody is administered. For example, this method may be applied to an animal (e.g., monkey, etc.) toxicity study as well as to quantification of the anti-drug antibody in human serum.

As the anti-idiotype antibody that specifically binds to an idiotope site of an anti-c-Met antibody competes with a c-Met protein which is an antigen of the anti-c-Met antibody, in binding to the anti-c-Met antibody, it can be said to be structurally similar to the c-Met protein. Using this aspect, the anti-idiotype antibody that specifically binds to an idiotope site of an anti-c-Met antibody or the antigen-binding fragment thereof may be applied as a vaccine for c-Met related diseases.

Another embodiment of the invention provides a vaccine composition for a c-Met related disease including the anti-idiotype antibody that specifically binds to an idiotope site of an anti-c-Met antibody or the antigen-binding fragment thereof as an active ingredient. Another disclosure is a method of immunizing a subject against a c-Met related disease including administering the anti-idiotype antibody or antigen-binding fragment to the subject.

The vaccine composition or the anti-idiotype antibody or antigen-binding fragment may be administered to mammals, including primates such as humans and monkeys and rodents such as mice and rats, for instance, patients who are likely to develop c-Met related diseases or suffer from c-Met related diseases.

The vaccine composition or the anti-idiotype antibody or antigen-binding fragment may further include a pharmaceutically acceptable carrier, and the carrier may be those commonly used for the formulation of drugs and may be one or more selected from the group consisting of lactose, dextrose, sucrose, sorbitol, mannitol, starch, gum acacia, calcium phosphate, alginates, gelatin, calcium silicate, micro-crystalline cellulose, polyvinylpyrrolidone, cellulose, water, syrup, methyl cellulose, methylhydroxy benzoate, propylhydroxy benzoate, talc, magnesium stearate, and mineral oil, but are not limited thereto. The pharmaceutical composition may further include one or more selected from the group consisting of a lubricant, a wetting agent, a sweetener, a flavor enhancer, an emulsifying agent, a suspension agent, and preservative which are commonly used for the preparation of pharmaceutical compositions.

The vaccine composition or the anti-idiotype antibody or antigen-binding fragment may be administered orally or parenterally. The parenteral administration may include intravenous injection, subcutaneous injection, muscular injection, intraperitoneal injection, endothelial administration, local administration, intranasal administration, intrapulmonary administration, and rectal administration. Since oral administration leads to digestion of proteins or peptides, an active ingredient in the compositions for oral administration must be coated or formulated to prevent digestion in stomach. In addition, the compositions may be administered using an optional device that enables an active substance to be delivered to target cells.

The content of the anti-idiotype antibody that specifically binds to an idiotope site of an anti-c-Met antibody or the antigen-binding fragment thereof in the pharmaceutical composition may be prescribed in a variety of ways, depending on factors such as formulation methods, administration methods, age of patients, body weight, gender, pathologic conditions, diets, administration time, administration route, excretion speed, and reaction sensitivity. For instance, a single dosage of the anti-idiotype antibody against the anti-c-Met antibody or the antigen-binding fragment thereof may be in the range of about 0.001 to about 100 mg/kg, particularly about 0.01 to 1 about 00 mg/kg, more particularly about 0.1 to about 50 mg/kg, but is not limited thereto. The single dosage may be formulated into a single formulation in a unit dosage form or formulated in suitably divided dosage forms, or it may be manufactured to be contained in a multiple dosage container.

The vaccine composition may be a solution in oil or an aqueous medium, a suspension, syrup, or an emulsifying solution, or formulated into the form of an extract, powder, granules, a tablet, or a capsule, and it may further include a dispersing or a stabilizing agent.

In particular, since the vaccine composition including the anti-idiotype antibody against the anti-c-Met antibody or the antigen-binding fragment thereof includes an antibody or an antigen-binding fragment thereof, it may be formulated as an immunoliposome. The liposome containing an antibody may be prepared using a well-known method in the pertinent art. The immunoliposome is a lipid composition including phosphatidylcholine, cholesterol, and polyethyleneglycol-derivatized phosphatidylethanolamine, and may be prepared by a reverse phase evaporation method. For example, Fab' fragments of an antibody may be conjugated to the liposome through a disulfide exchange reaction.

The c-Met related diseases refer to any diseases induced by c-Met expression or overexpression, for example, a cancer. The cancer may be caused by c-Met expression or overexpression. The cancer may be a solid cancer or hematological cancer and it may be, but not limited to, one or more selected from the group consisting of squamous cell carcinoma, small-cell lung cancer, non-small-cell lung cancer, adenocarcinoma of the lung, squamous cell carcinoma of the lung, peritoneal carcinoma, skin cancer, melanoma in the skin or eyeball, rectal cancer, cancer near the anus, esophagus cancer, small intestinal tumor, endocrine gland cancer, parathyroid cancer, adrenal cancer, soft-tissue sarcoma, urethral cancer, chronic or acute leukemia, lymphocytic lymphoma, hepatoma, gastrointestinal cancer, gastric cancer, pancreatic cancer, glioblastoma, cervical cancer, ovarian cancer, liver cancer, bladder cancer, hepatocellular adenoma, breast cancer, colon cancer, large intestine cancer, endometrial carcinoma or uterine carcinoma, salivary gland tumor, kidney cancer, prostate cancer, vulvar cancer, thyroid cancer, head or neck cancer, and the like. The cancer may include a metastatic cancer as well as a primary cancer. Besides cancer, the c-Met related diseases may include gestational diabetes.

In another embodiment, there is provided a polypeptide molecule including
the heavy chain complementarity determining region of the anti-idiotype antibody that specifically binds to an idiotope site of an anti-c-Met antibody, the light chain complementarity determining region of the anti-idiotype antibody that specifically binds to an idiotope site of an anti-c-Met antibody, or a combination thereof; or
the heavy chain variable region of the anti-idiotype antibody that specifically binds to an idiotope site of an anti-c-Met antibody, the light chain variable region of the anti-idiotype antibody that specifically binds to an idiotope site of an anti-c-Met antibody, or a combination thereof.

The polypeptide molecule may serve as a precursor of an antibody, which can be not only used to manufacture the antibody but also included as a component of a protein scaffold (e.g., peptibody) having a structural similar to an antibody, a bispecific antibody (constituting the c-Met binding site of the double antigen-binding sites of a double antibody), and a multi-specific antibody (constituting the c-Met binding site of the multiple antigen-binding sites of a multi-specific antibody).

The polypeptide molecule may include
one or more polypeptides selected from the group consisting of a polypeptide including the amino acid sequence of SEQ ID NO: 109 (for example, an amino acid sequence selected from the group consisting of SEQ ID NO: 115 to SEQ ID NO: 120) or the amino acid sequence of SEQ ID NO: 110 (for example, an amino acid sequence selected from the group consisting of SEQ ID NO: 121 to SEQ ID NO: 124), a polypeptide including the amino acid sequence of SEQ ID NO: 111 (for example, an amino acid sequence selected from the group consisting of SEQ ID NO: 125 to SEQ ID NO: 137) or the amino acid sequence of SEQ ID NO: 138, and a polypeptide including an amino acid sequence selected from the group consisting of SEQ ID NO: 139 to SEQ ID NO: 154;
one or more polypeptides selected from the group consisting of a polypeptide including the amino acid sequence of SEQ ID NO: 112 (for example, an amino acid sequence selected from the group consisting of SEQ ID NO: 155 to SEQ ID NO: 165) or an amino acid sequence selected from the group consisting of SEQ ID NO: 166 to SEQ ID NO: 171, a polypeptide including the amino acid sequence of SEQ ID NO: 113 (for example, an amino acid sequence selected from the group consisting of SEQ ID NO: 172 to SEQ ID NO: 186) or the amino acid sequence of SEQ ID NO: 187, and a polypeptide including the amino acid sequence of SEQ ID NO: 114 (for example, an amino acid sequence selected from the group consisting of SEQ ID NO: 188 to SEQ ID NO: 197) or an amino acid sequence selected from the group consisting of SEQ ID NO: 198 to SEQ ID NO: 201; or
a combination thereof.

In a specific embodiment, the polypeptide molecule may include an amino acid sequence selected from the group consisting of SEQ ID NO: 202 and SEQ ID NO: 203; an amino acid sequence selected from the group consisting of SEQ ID NO: 236 and SEQ ID NO: 237; or a combination thereof.

In another embodiment, there is provided a polynucleotide molecule encoding the polypeptide molecule or a recombinant vector including the polynucleotide. In particular, the polynucleotide molecule may include a nucleotide sequence selected from the group consisting of SEQ ID NO: 219 and SEQ ID NO: 220, a nucleotide sequence selected from the group consisting of SEQ ID NO: 253 and SEQ ID NO: 254, or a combination thereof.

The term "vector" used herein refers to a means for expressing a target gene in a host cell. For example, a vector may include a plasmid vector, a cosmid vector, and a virus vector such as a bacteriophage vector, an adenovirus vector, a retrovirus vector and an adeno-associated virus vector. Suitable recombinant vectors may be constructed by manipulating plasmids often used in the art (for example, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19), a phage (for example, λgt4λB, λ-Charon, λΔz1, and M13), or a virus (for example, SV40).

In the recombinant vector, the polynucleotides encoding the protein complex may be operatively linked to a promoter. The term "operatively linked" used herein refers to a functional linkage between a nucleotide expression regulating sequence (for example, a promoter sequence) and other nucleotide sequences. Thus, the regulating sequence may regulate the transcription and/or translation of the other nucleotide sequences by being operatively linked.

The recombinant vector may be constructed typically for either cloning or expression. The expression vector may be any ordinary vectors known in the pertinent art for expressing an exogenous protein in plants, animals, or microorganisms. The recombinant vector may be constructed using various methods known in the art.

The recombinant vector may be constructed using a prokaryotic cell or a eukaryotic cell as a host. For example, when a prokaryotic cell is used as a host cell, the expression vector used generally includes a strong promoter capable of initiating transcription (for example, pL^{λ} promoter, CMV promoter, *trp* promoter, *lac* promoter, *tac* promoter, T7 promoter, etc.), a ribosome binding site for initiating translation, and a transcription/translation termination sequence. When a eukaryotic cell is used as a host cell, the vector used generally includes the origin of replication acting in the eukaryotic cell, for example, a f1 replication origin, a SV40 replication origin, a pMB1 replication origin, an adeno replication origin, an AAV replication origin, or a BBV replication origin, but is not limited thereto. A promoter in an expression vector for a eukaryotic host cell may be a promoter derived from the genomes of mammalian cells (for example, a metallothionein promoter) or a promoter derived from mammalian viruses (for example, an adenovirus late promoter, a vaccinia virus 7.5K promoter, a SV40 promoter, a cytomegalovirus promoter, and a tk promoter of HSV). A transcription termination sequence in an expression vector for a eukaryotic host cell may be, in general, a polyadenylation sequence.

Another embodiment provides a recombinant cell including the recombinant vector.

The recombinant cell may be those obtained by transfecting the recombinant vector into a suitable host cell. Any host cells known in the pertinent art to enable stable and continuous cloning or expression of the recombinant vector may be used as the host cell. Suitable prokaryotic host cells may include *E*. *coli* JM109, *E*. *coli* BL21, *E*. *coli* RR1, *E*. *coli* LE392, *E*. *coli* B, *E. coli* X 1776, *E*. *coli* W3110, Bacillus species strains such as *Bacillus subtilis* or *Bacillus thuringiensis*, intestinal bacteria and strains such as *Salmonella typhymurum*, *Serratia marcescens*, and various Pseudomonas species. Suitable eukaryotic host cells to be transformed may include yeasts, such as *Saccharomyces cerevisiae*, insect cells, plant cells, and animal cells, for example, Sp2/0, Chinese hamster ovary (CHO) K1, CHO DG44, PER.C6, W138, BHK, COS-7, 293, HepG2, Huh7, 3T3, RIN, and MDCK cell lines, but are not limited thereto.

The polynucleotide or the recombinant vector including the same may be transferred (transfected) into a host cell by using known transfer methods. Suitable transfer methods for prokaryotic host cells may include a method using CaCl₂ and electroporation. Suitable transfer methods for eukaryotic host cells may include microinjection, calcium phosphate precipitation, electroporation, liposome-mediated transfection, and gene bombardment, but are not limited thereto.

A transformed host cell may be selected using a phenotype expressed by a selected marker by any methods known in the art. For example, if the selected marker is a gene that is resistant to a specific antibiotic, a transformant may be easily selected by being cultured in a medium including the antibiotic.

The present invention can not only improve the accuracy of anti-c-Met antibody analysis by providing an anti-idiotype antibody that specifically binds to an idiotope site of an anti-c-Met antibody, compared to the pre-existing PK methods, but may also be applied to clinical sample analysis of the anti-c-Met antibody and applied to measurement analysis of an anti-drug antibody (ADA) to be able to learn the presence/absence of ADA production and quantification thereof. Also, it is expected that the anti-idiotype antibody against the anti-c-Met antibody may be utilized as a vaccine for c-Met related diseases.

Hereafter, the present invention will be described in detail by examples.

The following examples are intended merely to illustrate the invention and are not construed to restrict the invention.

### EXAMPLES

### Reference Example 1: Construction of Anti-c-Met Antibody

### 1.1. Production of "AbF46", a Mouse Antibody to c-Met

### 1.1.1. Immunization of mouse

To obtain immunized mice necessary for the development of a hybridoma cell line, each of five BALB/c mice (Japan SLC, Inc.), 4 to 6 weeks old, was intraperitoneally injected with a mixture of 100 µg of human c-Met/Fc fusion protein (R&D Systems) and one volume of complete Freund's adjuvant. Two weeks after the injection, a second intraperitoneal injection was conducted on the same mice with a mixture of 50 µg of human c-Met/Fc protein and one volume of incomplete Freund's adjuvant. One week after the second immunization, the immune response was finally boosted. Three days later, blood was taken from the tails of the mice and the sera were 1/1000 diluted in PBS and used to examine a titer of antibody to c-Met by ELISA. Mice found to have a sufficient antibody titer were selected for use in the cell fusion process.

### 1.1.2. Cell fusion and production of hybridoma

Three days before cell fusion, BALB/c mice (Japan SLC, Inc.) were immunized with an intraperitoneal injection of a mixture of 50 µg of human c-Met/Fc fusion protein and one volume of PBS. The immunized mice were anesthetized before excising the spleen from the left half of the body. The spleen was meshed to separate splenocytes which were then suspended in a culture medium (DMEM, GIBCO, Invitrogen). The cell suspension was centrifuged to recover the cell layer. The splenocytes thus obtained (1x10⁸ cells) were mixed with myeloma cells (Sp2/0) (1x10⁸ cells), followed by spinning to give a cell pellet. The cell pellet was slowly suspended, treated with 45% polyethylene glycol (PEG) (1 mL) in DMEM for 1 min at 37 ºC, and supplemented with 1 mL of DMEM. To the cells was added 10 mL of DMEM over 10 min, after which incubation was conducted in a water bath at 37 ºC for 5 min. Then the cell volume was adjusted to 50 mL before centrifugation. The cell pellet thus formed was resuspended at a density of 1∼2×10⁵ cells/mL in a selection medium (HAT medium) and 0.1 mL of the cell suspension was allocated to each well of 96-well plates which were then incubated at 37 ºC in a CO₂ incubator to establish a hybridoma cell population.

### 1.1.3. Selection of hybridoma cells producing monoclonal antibodies to c-Met protein

From the hybridoma cell population established in Reference Example 1.1.2, hybridoma cells which showed a specific response to c-Met protein were screened by ELISA using human c-Met/Fc fusion protein and human Fc protein as antigens.

Human c-Met/Fc fusion protein was seeded in an amount of 50 µL (2 µg/mL)/well to microtiter plates and allowed to adhere to the surface of each well. The antibody that remained unbound was removed by washing. For use in selecting the antibodies that do not bind c-Met but recognize Fc, human Fc protein was attached to the plate surface in the same manner.

The hybridoma cell culture obtained in Reference Example 1.1.2 was added in an amount of 50 µL to each well of the plates and incubated for 1 hour. The cells remaining unreacted were washed out with a sufficient amount of Tris-buffered saline and Tween 20 (TBST). Goat anti-mouse IgG-horseradish peroxidase (HRP) was added to the plates and incubated for 1 hour at room temperature. The plates were washed with a sufficient amount of TBST, followed by reacting the peroxidase with a substrate (OPD). Absorbance at 450 nm was measured on an ELISA reader.

Hybridoma cell lines which secrete antibodies that specifically and strongly bind to human c-Met but not human Fc were selected repeatedly. From the hybridoma cell lines obtained by repeated selection, a single clone producing a monoclonal antibody was finally separated by limiting dilution. The single clone of the hybridoma cell line producing the monoclonal antibody was deposited with the Korean Cell Line Research Foundation, an international depository authority located at Yungun-Dong, Jongno-Gu, Seoul, Korea, on Oct. 9, 2009, with Accession No. KCLRF-BP-00220 according to the Budapest Treaty (refer to Korean Patent Laid-Open Publication No. 2011-0047698).

### 1.1.4. Production and purification of monoclonal antibody

The hybridoma cell line obtained in Reference Example 1.1.3 was cultured in a serum-free medium, and the monoclonal antibody (AbF46) was produced and purified from the cell culture.

First, the hybridoma cells cultured in 50 mL of a medium (DMEM) supplemented with 10% (v/v) FBS were centrifuged and the cell pellet was washed twice or more with 20 mL of PBS to remove the FBS therefrom. Then, the cells were resuspended in 50 mL of DMEM and incubated for 3 days at 37 ºC in a CO₂ incubator.

After the cells were removed by centrifugation, the supernatant was stored at 4 ºC before use or immediately used for the separation and purification of the antibody. An AKTA system (GE Healthcare) equipped with an affinity column (Protein G agarose column; Pharmacia, USA) was used to purify the antibody from 50 to 300 mL of the supernatant, followed by concentration with a filter (Amicon). The antibody in PBS was stored before use in the following examples.

### 1.2. Construction of chAbF46, a chimeric antibody to c-Met

A mouse antibody induces immunogenicity in humans. To solve this problem, chAbF46, a chimeric antibody, was constructed from the mouse antibody AbF46 produced in Experimental Example 1.1.4 by replacing the constant region, but not the variable region responsible for antibody specificity, with an amino sequence of the human IgG1 antibody.

In this regard, a gene was designed to include the nucleotide sequence of "EcoRI-signal sequence-VH-Nhel-CH-TGA-Xhol" (SEQ ID NO: 38) for a heavy chain and the nucleotide sequence of "EcoRI-signal sequence-VL-BsiWI-CL-TGA-Xhol" (SEQ ID NO: 39) for a light chain and synthesized. Then, a DNA fragment having the heavy chain nucleotide sequence (SEQ ID NO: 38) and a DNA fragment having the light chain nucleotide sequence (SEQ ID NO: 39) were digested with EcoRI (NEB, R0101S) and Xhol (NEB, R0146S) before cloning into a pOptiVEC™-TOPO TA Cloning Kit enclosed in an OptiCHO™ Antibody Express Kit (Cat no. 12762-019, Invitrogen), and a pcDNA™3.3-TOPOTA Cloning Kit (Cat no. 8300-01), respectively.

Each of the constructed vectors was amplified using Qiagen Maxiprep kit (Cat no. 12662), and a transient expression was performed using Freestyle™ MAX 293 Expression System (Invitrogen). 293 F cells were used for the expression and cultured in Freestyle™ 293 Expression Medium in a suspension culture manner. At one day before the transient expression, the cells were provided in the concentration of 5x10⁵cells/ml, and after 24 hours, when the cell number reached to 1x10⁶cells/ml, the transient expression was performed. A transfection was performed by a liposomal reagent method using Freestyle™ MAX reagent (Invitrogen), wherein in a 15ml tube, the DNA was provided in the mixture ratio of 1:1 (heavy chain DNA : light chain DNA) and mixed with 2ml of OptiPro™ SFM (Invitrogen) (A), and in another 15ml tube, 100µl (microliter) of Freestyle™ MAX reagent and 2ml of OptiPro™ SFM were mixed (B), followed by mixing (A) and (B) and incubating for 15 minutes. The obtained mixture was slowly mixed with the cells provided one day before the transient expression. After completing the transfection, the cells were incubated in 130 rpm incubator for 5 days under the conditions of 37 °C, 80% humidity, and 8% CO₂.

Afterwards, the cells were incubated in DMEM supplemented with 10% (v/v) FBS for 5 hours at 37 ºC under a 5% CO₂ condition and then in FBS-free DMEM for 48 hours at 37 ºC under a 5% CO₂ condition.

After centrifugation, the supernatant was applied to AKTA prime (GE Healthcare) to purify the antibody. In this regard, 100 mL of the supernatant was loaded at a flow rate of 5 mL/min to AKTA Prime equipped with a Protein A column (GE healthcare, 17-0405-03), followed by elution with an IgG elution buffer (Thermo Scientific, 21004). The buffer was exchanged with PBS to purify a chimeric antibody AbF46 (hereinafter referred to as "chAbF46").

### 1.3. Construction of Humanized Antibody huAbF46 from Chimeric Antibody chAbF46

### 1.3.1. Heavy chain humanization

To design two domains H1-heavy and H3-heavy, human germline genes which share the highest identity/homology with the VH gene of the mouse antibody AbF46 purified in Reference Example 1.2 were analyzed. An Ig BLAST (www.ncbi.nlm.nih.gov/igblast/) result revealed that VH3-71 has an identity/ homology of 83% at the amino acid level. CDR-H1, CDR-H2, and CDR-H3 of the mouse antibody AbF46 were defined according to Kabat numbering. A design was made to introduce the CDR of the mouse antibody AbF46 into the framework of VH3-71. Hereupon, back mutations to the amino acid sequence of the mouse AbF46 were conducted at positions 30 (S→T), 48 (V→L), 73 (D→N), and 78 (T→L). Then, H1 was further mutated at positions 83 (R→K) and 84 (A→T) to finally establish H1-heavy (SEQ ID NO: 40) and H3-heavy (SEQ ID NO: 41).

For use in designing H4-heavy, human antibody frameworks were analyzed by a BLAST search. The result revealed that the VH3 subtype, known to be most stable, is very similar in framework and sequence to the mouse antibody AbF46. CDR-H1, CDR-H2, and CDR-H3 of the mouse antibody AbF46 were defined according to Kabat numbering and introduced into the VH3 subtype to construct H4-heavy (SEQ ID NO: 42).

### 1.3.2. Light chain humanization

To design two domains H1-light (SEQ ID NO: 43) and H2-light (SEQ ID NO: 44), human germline genes which share the highest identity/homology with the VH gene of the mouse antibody AbF46 were analyzed. An Ig BLAST search result revealed that VK4-1 has an identity/homology of 75% at the amino acid level. CDR-L1, CDR-L2, and CDR-L3 of the mouse antibody AbF46 were defined according to Kabat numbering. A design was made to introduce the CDR of the mouse antibody AbF46 into the framework of VK4-1. Hereupon, back mutations to the amino acid sequence of the mouse AbF46 were conducted at positions 36 (Y→H), 46 (L→M), and 49 (V→I). Only one back mutation was conducted at position 49 (Y→I) on H2-light.

To design H3-light (SEQ ID NO: 45), human germline genes which share the highest identity/homology with the VL gene of the mouse antibody AbF46 were analyzed by a search for BLAST. As a result, VK2-40 was selected. VL and VK2-40 of the mouse antibody AbF46 were found to have an identity/homology of 61% at an amino acid level. CDR-L1, CDR-L2, and CDR-L3 of the mouse antibody were defined according to Kabat numbering and introduced into the framework of VK4-1. Back mutations were conducted at positions 36 (Y→H), 46 (L→M), and 49 (Y→I) on H3-light.

For use in designing H4-light (SEQ ID NO: 46), human antibody frameworks were analyzed. A Blast search revealed that the Vk1 subtype, known to be the most stable, is very similar in framework and sequence to the mouse antibody AbF46. CDR-L1, CDR-L2, and CDR-L3 of the mouse antibody AbF46 were defined according to Kabat numbering and introduced into the Vk1 subtype. Hereupon, back mutations were conducted at positions 36 (Y→H), 46 (L→M), and 49 (Y→I) on H4-light.

Thereafter, DNA fragments having the heavy chain nucleotide sequences (H1-heavy: SEQ ID NO: 47, H3-heavy: SEQ ID NO: 48, H4-heavy: SEQ ID NO: 49) and DNA fragments having the light chain nucleotide sequences (H1-light: SEQ ID NO: 50, H2-light: SEQ ID NO: 51, H3-light: SEQ ID NO: 52, H4-light: SEQ ID NO: 53) were digested with EcoRI (NEB, R0101S) and Xhol (NEB, R0146S) before cloning into a pOptiVEC™-TOPO TA Cloning Kit enclosed in an OptiCHO™ Antibody Express Kit (Cat no. 12762-019, Invitrogen) and a pcDNA™3.3-TOPO TA Cloning Kit (Cat no. 8300-01), respectively, so as to construct recombinant vectors for expressing a humanized antibody.

Each of the constructed vectors was amplified using Qiagen Maxiprep kit (Cat no. 12662), and a transient expression was performed using Freestyle™ MAX 293 Expression System (Invitrogen). 293 F cells were used for the expression and cultured in FreeStyle™ 293 Expression Medium in a suspension culture manner. At one day before the transient expression, the cells were provided in the concentration of 5x10⁵cells/ml, and after 24 hours, when the cell number reached to 1x10⁶cells/ml, the transient expression was performed. A transfection was performed by a liposomal reagent method using Freestyle™ MAX reagent (Invitrogen), wherein in a 15ml tube, the DNA was provided in the mixture ratio of 1:1 (heavy chain DNA : light chain DNA) and mixed with 2ml of OptiPro™ SFM (Invitrogen) (A), and in another 15ml tube, 100µl (microliter) of Freestyle™ MAX reagent and 2ml of OptiPro™ SFM were mixed (B), followed by mixing (A) and (B) and incubating for 15 minutes. The obtained mixture was slowly mixed with the cells provided one day before the transient expression. After completing the transfection, the cells were incubated in 130 rpm incubator for 5 days under the conditions of 37 °C, 80% humidity, and 8% CO₂.

After centrifugation, the supernatant was applied to AKTA prime (GE Healthcare) to purify the antibody. In this regard, 100 mL of the supernatant was loaded at a flow rate of 5 mL/min to AKTA Prime equipped with a Protein A column (GE healthcare, 17-0405-03), followed by elution with an IgG elution buffer (Thermo Scientific, 21004). The buffer was exchanged with PBS to purify a humanized antibody AbF46 (hereinafter referred to as "huAbF46"). The humanized antibody huAbF46 used in the following examples included a combination of H4-heavy (SEQ ID NO: 42) and H4-light (SEQ ID NO: 46).

### 1.4. Construction of scFV Library of huAbF46 Antibody

For use in constructing an scFv of the huAbF46 antibody from the heavy and light chain variable regions of the huAbF46 antibody, a gene was designed to have the structure of "VH-linker-VL" for each of the heavy and the light chain variable region, with the linker having the amino acid sequence "GLGGLGGGGSGGGGSGGSSGVGS" (SEQ ID NO: 54). A polynucleotide sequence (SEQ ID NO: 55) encoding the designed scFv of huAbF46 was synthesized in Bioneer and an expression vector for the polynucleotide had the nucleotide sequence of SEQ ID NO: 56.

After expression, the product was found to exhibit specificity to c-Met.

### 1.5. Construction of Library Genes for Affinity Maturation

### 1.5.1. Selection of target CDRs and synthesis of primers

The affinity maturation of huAbF46 was achieved. First, six complementary determining regions (CDRs) were defined according to Kabat numbering. The CDRs are given in Table 3, below.

**TABLE 3**

| CDR | Amino Acid Sequence |
|---|---|
| CDR.H1 | DYYMS (SEQ ID NO: 1) |
| CDR-H2 | FIRNKANGYTTEYSASVKG (SEQ ID NO: 2) |
| CDR-H3 | DNWFAY (SEQ ID NO: 3) |
| CDR-L1 | KSSQSLLASGNQNNYLA (SEQ ID NO: 10) |
| CDR-L2 | WASTRVS (SEQ ID NO: 11) |
| CDR-L3 | QQSYSAPLT (SEQ ID NO: 12) |

For use in the introduction of random sequences into the CDRs of the antibody, primers were designed as follows. Conventionally, N codons were utilized to introduce bases at the same ratio (25% A, 25% G, 25% C, 25% T) into desired sites of mutation. In this experiment, the introduction of random bases into the CDRs of huAbF46 was conducted in such a manner that, of the three nucleotides per codon in the wild-type polynucleotide encoding each CDR, the first and second nucleotides conserved over 85% of the entire sequence while the other three nucleotides were introduced at the same percentage (each 5%) and that the same possibility was imparted to the third nucleotide (33 % G, 33 % C, 33 % T).

### 1.5.2. Construction of a library of huAbF46 antibodies and affinity for c-Met

The construction of antibody gene libraries through the introduction of random sequences was carried out using the primers synthesized in the same manner as in Reference Example 1.5.1. Two PCR products were obtained using a polynucleotide covering the scFV of huAbF46 as a template, and were subjected to overlap extension PCR to give scFv library genes for huAbF46 antibodies in which only desired CDRs were mutated. Libraries targeting each of the six CDRs prepared from the scFV library genes were constructed.

The affinity for c-Met of each library was compared to that of the wildtype. Most libraries were lower in affinity for c-Met, compared to the wild-type. The affinity for c-Met was retained in some mutants.

### 1.6. Selection of Antibody with Improved Affinity from Libraries

After maturation of the affinity of the constructed libraries for c-Met, the nucleotide sequence of scFv from each clone was analyzed. The nucleotide sequences thus obtained are summarized in Table 4 and were converted into IgG forms. Four antibodies which were respectively produced from clones L3-1, L3-2, L3-3, and L3-5 were used in the subsequent experiments.

**TABLE 4**

| Clone | Library constructed | CDR Sequence |
|---|---|---|
| H11-4 | CDR-H1 | PEYYMS (SEQ ID NO: 22) |
| YC151 | CDR-H1 | PDYYMS (SEQ ID NO: 23) |
| YC193 | CDR-H1 | SDYYMS (SEQ ID NO: 24) |
| YC244 | CDR-H2 | RNNANGNT (SEQ ID NO: 25) |
| YC321 | CDR-H2 | RNKVNGYT (SEQ ID NO: 26) |
| YC354 | CDR-H3 | DNWLSY (SEQ ID NO: 27) |
| YC374 | CDR-H3 | DNWLTY (SEQ ID NO: 28) |
| L1-1 | CDR-L1 | KSSHSLLASGNQNNYLA (SEQ ID NO: 29) |
| L1-3 | CDR-L1 | KSSRSLLSSGNHKNYLA (SEQ ID NO: 30) |
| L1-4 | CDR-L1 | KSSKSLLASGNQNNYLA (SEQ ID NO: 31) |
| L1-12 | CDR-L1 | KSSRSLLASGNQNNYLA (SEQ ID NO: 32) |
| L1-22 | CDR-L1 | KSSHSLLASGNQNNYLA (SEQ ID NO: 33) |
| L2-9 | CDR-L2 | WASKRVS (SEQ ID NO: 34) |
| L2-12 | CDR-L2 | WGSTRVS (SEQ ID NO: 35) |
| L2-16 | CDR-L2 | WGSTRVP (SEQ ID NO: 36) |
| L3-1 | CDR-L3 | QQSYSRPYT (SEQ ID NO: 13) |
| L3-2 | CDR-L3 | GQSYSRPLT (SEQ ID NO: 14) |
| L3-3 | CDR-L3 | AQSYSHPFS (SEQ ID NO: 15) |
| L3-5 | CDR-L3 | QQSYSRPFT (SEQ ID NO: 16) |
| L3-32 | CDR-L3 | QQSYSKPFT (SEQ ID NO: 37) |

### 1.7. Conversion of Selected Antibodies into IgG

Respective polynucleotides encoding heavy chains of the four selected antibodies were designed to have the structure of "EcoRI-signal sequence-VH-NheI-CH-XhoI" (SEQ ID NO: 38). The heavy chains of huAbF46 antibodies were used as they were because their amino acids were not changed during affinity maturation. In the case of the hinge region, however, the U6-HC7 hinge (SEQ ID NO: 57) was employed instead of the hinge of human IgG1. Genes were also designed to have the structure of "EcoRI-signal sequence-VL-BsiWI-CL-XhoI" for the light chain. Polypeptides encoding light chain variable regions of the four antibodies which were selected after the affinity maturation were synthesized in Bioneer. Then, a DNA fragment having the heavy chain nucleotide sequence (SEQ ID NO: 38) and DNA fragments having the light chain nucleotide sequences (DNA fragment including L3-1-derived CDR-L3: SEQ ID NO: 58, DNA fragment including L3-2-derived CDR-L3: SEQ ID NO: 59, DNA fragment including L3-3-derived CDR-L3: SEQ ID NO: 60, and DNA fragment including L3-5-derived CDR-L3: SEQ ID NO: 61) were digested with EcoRI (NEB, R0101S) and XhoI (NEB, R0146S) before cloning into a pOptiVEC™-TOPO TA Cloning Kit enclosed in an OptiCHO™ Antibody Express Kit (Cat no. 12762-019, Invitrogen) and a pcDNA™3.3-TOPO TA Cloning Kit (Cat no. 8300-01), respectively, so as to construct recombinant vectors for expressing affinity-matured antibodies.

Each of the constructed vectors was amplified using Qiagen Maxiprep kit (Cat no. 12662), and a transient expression was performed using Freestyle™ MAX 293 Expression System (Invitrogen). 293 F cells were used for the expression and cultured in Freestyle™ 293 Expression Medium in a suspension culture manner. At one day before the transient expression, the cells were provided in the concentration of 5x10⁵cells/ml, and after 24 hours, when the cell number reached to 1x10⁶cells/ml, the transient expression was performed. A transfection was performed by a liposomal reagent method using Freestyle™ MAX reagent (Invitrogen), wherein in a 15ml tube, the DNA was provided in the mixture ratio of 1:1 (heavy chain DNA : light chain DNA) and mixed with 2ml of OptiPro™ SFM (Invitrogen) (A), and in another 15ml tube, 100µl (microliter) of Freestyle™ MAX reagent and 2ml of OptiPro™ SFM were mixed (B), followed by mixing (A) and (B) and incubating for 15 minutes. The obtained mixture was slowly mixed with the cells provided one day before the transient expression. After completing the transfection, the cells were incubated in 130 rpm incubator for 5 days under the conditions of 37 °C, 80% humidity, and 8% CO₂.

After centrifugation, the supernatant was applied to AKTA prime (GE Healthcare) to purify the antibody. In this regard, 100 mL of the supernatant was loaded at a flow rate of 5 mL/min to AKTA Prime equipped with a Protein A column (GE healthcare, 17-0405-03), followed by elution with an IgG elution buffer (Thermo Scientific, 21004). The buffer was exchanged with PBS to purify four affinity-matured antibodies (hereinafter referred to as "huAbF46-H4-A1 (L3-1 origin), huAbF46-H4-A2 (L3-2 origin), huAbF46-H4-A3 (L3-3 origin), and huAbF46-H4-A5 (L3-5 origin)," respectively).

### 1.8. Construction of Constant Region- and/or Hinge Region-Substituted huAbF46-H4-A1

Among the four antibodies selected in Reference Example 1.7, huAbF46-H4-A1 was found to be the highest in affinity for c-Met and the lowest in Akt phosphorylation and c-Met degradation degree. In the antibody, the hinge region, or the constant region and the hinge region, were substituted.

The antibody huAbF46-H4-A1 (U6-HC7) was composed of a heavy chain including the heavy chain variable region of huAbF46-H4-A1, U6-HC7 hinge, and the constant region of human IgG1 constant region, and a light chain including the light chain variable region of huAbF46-H4-A1 and human kappa constant region. The antibody huAbF46-H4-A1 (IgG2 hinge) was composed of a heavy chain including a heavy chain variable region, a human IgG2 hinge region, and a human IgG1 constant region, and a light chain including the light chain variable region of huAbF46-H4-A1 and a human kappa constant region. The antibody huAbF46-H4-A1 (IgG2 Fc) was composed of the heavy chain variable region of huAbF46-H4-A1, a human IgG2 hinge region, and a human IgG2 constant region, and a light chain including the light variable region of huAbF46-H4-A1 and a human kappa constant region. Hereupon, the histidine residue at position 36 on the human kappa constant region of the light chain was changed to tyrosine in all of the three antibodies to increase antibody production.

For use in constructing the three antibodies, a polynucleotide (SEQ ID NO: 63) encoding a polypeptide (SEQ ID NO: 62) composed of the heavy chain variable region of huAbF46-H4-A1, a U6-HC7 hinge region, and a human IgG1 constant region, a polynucleotide (SEQ ID NO: 65) encoding a polypeptide (SEQ ID NO: 64) composed of the heavy chain variable region of huAbF46-H4-A1, a human IgG2 hinge region, and a human IgG1 region, a polynucleotide (SEQ ID NO: 67) encoding a polypeptide (SEQ ID NO: 66) composed of the heavy chain variable region of huAbF46-H4-A1, a human IgG2 region, and a human IgG2 constant region, and a polynucleotide (SEQ ID NO: 69) encoding a polypeptide (SEQ ID NO: 68) composed of the light chain variable region of huAbF46-H4-A1, with a tyrosine residue instead of histidine at position 36, and a human kappa constant region were synthesized in Bioneer. Then, the DNA fragments having heavy chain nucleotide sequences were inserted into a pOptiVEC™-TOPO TA Cloning Kit enclosed in an OptiCHO™ Antibody Express Kit (Cat no. 12762-019, Invitrogen) while DNA fragments having light chain nucleotide sequences were inserted into a pcDNA™3.3-TOPO TA Cloning Kit (Cat no. 8300-01) so as to construct vectors for expressing the antibodies.

Each of the constructed vectors was amplified using Qiagen Maxiprep kit (Cat no. 12662), and a transient expression was performed using Freestyle™ MAX 293 Expression System (Invitrogen). 293 F cells were used for the expression and cultured in FreeStyle™ 293 Expression Medium in a suspension culture manner. At one day before the transient expression, the cells were provided in the concentration of 5x10⁵cells/ml, and after 24 hours, when the cell number reached to 1x10⁶cells/ml, the transient expression was performed. A transfection was performed by a liposomal reagent method using Freestyle™ MAX reagent (Invitrogen), wherein in a 15ml tube, the DNA was provided in the mixture ratio of 1:1 (heavy chain DNA : light chain DNA) and mixed with 2ml of OptiPro™ SFM (Invitrogen) (A), and in another 15ml tube, 100µl (microliter) of Freestyle™ MAX reagent and 2ml of OptiPro™ SFM were mixed (B), followed by mixing (A) and (B) and incubating for 15 minutes. The obtained mixture was slowly mixed with the cells provided one day before the transient expression. After completing the transfection, the cells were incubated in 130 rpm incubator for 5 days under the conditions of 37 °C, 80% humidity, and 8% CO₂.

After centrifugation, the supernatant was applied to AKTA prime (GE Healthcare) to purify the antibody. In this regard, 100 mL of the supernatant was loaded at a flow rate of 5 mL/min to AKTA Prime equipped with a Protein A column (GE healthcare, 17-0405-03), followed by elution with IgG elution buffer (Thermo Scientific, 21004). The buffer was exchanged with PBS to finally purify three antibodies (huAbF46-H4-A1 (U6-HC7), huAbF46-H4-A1 (IgG2 hinge), and huAbF46-H4-A1 (IgG2 Fc)). Among the three antibodies, huAbF46-H4-A1 (IgG2 Fc) was representatively selected for the following examples, and referred as "anti-c-Met antibody".

### Example 1: Preparation of Anti-idiotype Antibody Against Anti-c-Met Antibody

Using a phage display scFv library (construction of a large synthetic human scFv library with six diversified CDRs and high functional diversity. 2009, Mol cells., 27, pp.225-235; A human scFv antibody generation pipeline for proteome research. 2010, J. Biotechnol.,152, pp.159-170), binders recognizing and binding to the anti-c-Met antibody prepared in the above reference example as an antigen were screened to eliminate candidates binding to the Fc site of the antibody.

In particular, the anti-c-Met antibody prepared in the above reference example was immobilized in amounts of about 10 µg (microgram), 2 µg, 0.4 µg, and 0.1 µg, respectively on Dynabeads (Dynal, #143.01) to enrich antibodies that reacted to the anti-c-Met antibody through 1^{st}, 2^{nd}, 3^{rd} and 4^{th} pannings. The surface of Dynabeads was blocked using about 1% (w/v) BSA dissolved in a PBS, and about 1x10¹¹ to 1x10¹² of phage particles derived from the same phage display scFv library as described in the above were added to about 0.5 Mℓ of 1% (w/v) BSA and let stand at room temperature for one hour for blocking. Thereafter, the phages blocked with BSA were added to the Dynabeads on which the anti-c-Met antibodies blocked with BSA were immobilized to bind the anti-c-Met antibodies and the phages by rotation at room temperature for 2 hours. Especially, during the 2^{nd}, 3^{rd} and 4^{th} pannings, in order to eliminate in advance phage particles binding to the Fc portions, prior to binding the phages to the Dynabeads on which the anti-c-Met antibodies were immobilized, the Dynabeads were mixed with the phages blocked with hIgG1 and BSA in an amount corresponding to 1000 times the immobilized anti-c-Met antibodies to react at room temperature for one hour and then, the phages were bound to the Dynabeads on which the anti-c-Met antibodies were immobilized.

After the binding treatment of the phages, the surfaces of the phages were washed 1 to 5 times with 0.1% (v/v) Tween 20 dissolved in a PBS and then, the bound phages were eluted using 100 mM glycine-HCl, pH 2.2 solution. The eluted phages were used to infect *E.coli* XL1-Blue MRF' cells (Agilent, USA) and after amplified, they were obtained to prepare for the next screening step. Such procedures were repeated four times by immobilizing the anti-c-Met antibodies on Dynabeads in amounts of 10 µg (microgram), 2 µg, 0.4 µg, and 0.1 µg, respectively, followed by ELISA (Enzyme-Linked ImmunoSorbent Assay) affinity assay to identify anti-c-Met antibody binding scFv clones which recognize the anti-c-Met antibody.

In order to identify the anti-c-Met antibody binding scFv clones, the anti-c-Met antibody was seeded at 1 µg/ml onto each well of a 96-well plate to perform coating at 4 °C for 16 to 18 hours and then blocked with about 1% (w/v) BSA dissolved in a PBS at room temperature for one hour. Thereafter, the anti-c-Met antibody binding scFv clones which were cultured in advance were seeded at 50 µl onto each well to react at 37°C for 2 hours and then washed three times with 0.1% (v/v) Tween 20 dissolved in a PBS. Then, a 1:3000 dilution of anti-M13-HRP was seeded at 100 µl onto each well to react at 37°C for one hour and then washed three times with 0.1% (v/v) Tween 20 dissolved in a PBS and finally, absorption was measured at 450 nm after color development using TMB(3,3,5,5-tetramethylbenzidine).

17 kinds of binding clones were selected by the ELISA method as above, and in order to convert them into full IgG1 forms, oligomers encoding the heavy chain variable regions and the light chain variable regions of each clone were synthesized through IDT. Thereafter, genes of the 17 heavy chain variable regions and light chain variable regions were amplified through a PCR, and the heavy chain variable regions were inserted into pOptivec into which human IgG1 hinge and human IgG1 constant regions were inserted and the light chain variable regions were inserted into pcDNA 3.3 into which human light chain constant regions were inserted and then, their sequencing was performed by Bionics Inc. Finally, to obtain vectors for expressing antibodies.

Each sequence of the 17 kinds of the selected antibody heavy chain CDR, light chain CDR, heavy chain variable regions, light chain variable regions, heavy chain constant regions, and light chain constant regions was set forth in Tables 5 to 8 below.

The above constructed vectors were each amplified using Qiagen Maxiprep kit (Cat no. 12662), and temporary expression thereof proceeded using Freestyle™ MAX 293 Expression System (Invitrogen). The cells used were 293 F cells, which were cultured in a suspension culture manner using FreeStyle™ 293 Expression Medium as a medium. One day before the temporary expression, the cells were prepared at a concentration of 5x10⁵ cells/ml and after 24 hours, their temporary expression started when the number of the cells reached 1x10⁶ cells/ml. Transfection was performed by a liposomal reagent method using Freestyle™ MAX reagent (Invitrogen). DNA was prepared in a 15-ml tube in a ratio of heavy chain DNA:light chain DNA=1:1 and mixed with 2 ml of OptiPro™ SFM (Invitrogen) (A), and 100µℓ of FreestyleTM MAX reagent and 2 ml of OptiPro™ SFM were mixed in another 15-ml tube (B), and after (A) and (B) were mixed and incubated for 15 min., the mixture solution was then slowly mixed into the cells which were prepared one day before. After the transfection was complete, the cells were cultured in a 37°C, 80% humidity, 8% CO2, 130 rpm incubator for 5 days.

The cultured cells were centrifuged to obtain each 100 ml of supernatants, which were then purified using AKTA Prime (GE healthcare). The culture was flowed at a flow rate of 5 ml/min. onto the AKTA Prime installed with Protein A column (GE healthcare, 17-0405-03) to perform elution using an IgG elution buffer (Thermo Scientific, 21004). The buffer was replaced by a PBS buffer to finally obtain 17 kinds of antibodies.

Sequences of the purified 17 kinds of antibody heavy chain CDR, light chain CDR, heavy chain variable regions, light chain variable regions, heavy chain constant regions, and light chain constant regions were set forth in Tables 5 to 10 below.

**Table 5**

| Heavy Chain CDR | | | |
|---|---|---|---|
| Antibody | CDR-H1 | CDR-H2 | CDR-H3 |
| EW01 | DYYMS (SEQ ID NO: 115) | GIYSSSSNIYYADSVKG (SEQ ID NO: 125) | |
| EW02 | NYAMS (SEQ ID NO: 116) | SISSSGGNTYYADSVKG (SEQ ID NO: 126) | KYHSVFDY (SEQ ID NO: 140) |
| EW03 | DYDMS (SEQ ID NO:117) | LISYGGSNTYYADSVKG (SEQ ID NO: 127) | |
| EW06 | GYDMS (SEQ ID NO: 118) | GISHGDGNIYYADSVKG (SEQ ID NO: 128) | |
| EW09 | DYDMS (SEQ ID NO: 1 17) | SISYGGGSIYYADSVKG (SEQ ID NO: 129) | RDAAYFDY (SEQ ID NO: 143) |
| EW10 | GYDMS (SEQ ID NO: 118) | GISYNGGSKYYADSVKG (SEQ ID NO: 130) | |
| EW16 | DYYMS (SEQ ID NO: 115) | AISHSSGNTYYADSVKG (SEQ ID NO: 131) | |
| EW26 | NYAMS (SEQ ID NO: 116) | AIYPGGGNTYYADSVKG (SEQ ID NO: 132) | |
| EW28 | DYAMS (SEQ ID NO: 119) | AISSGDGNTYYADSVKG (SEQ ID NO: 133) | |
| EW34 | DYAMS (SEQ ID NO: 119) | SIYPDDGNTYYADSVKG (SEQ ID NO: 134) | |
| EW37 | NYSMS (SEQ ID NO: 120) | SISSSGGNTYYADSVKG (SEQ ID NO: 126) | RVHLYFDY (SEQ ID NO: 149) |
| HAL 7-1 | SYAMH (SEQ ID NO: 121) | VISYDGSNKYYADSVKG (SEQ ID NO: 135) | |
| HAL 7-2 | SYAIS (SEQ ID NO: 122) | GIIPIFGTANYAQKFQG (SEQ ID NO: 138) | RDRNSYYEEPMYYFDY (SEQ ID NO: 151) |
| HAL 7-5 | SYAIS (SEQ ID NO: 122) | GIIPIFGTANYAQKFQG (SEQ ID NO: 138) | RDRNSYYEEPMYYFDY (SEQ ID NO: 151) |
| HAL 7-7 | SYGMH (SEQ ID NO: 123) | VISYDGSNKYYADSVKG (SEQ ID NO: 135) | RDLVADDYGDYGTVDY (SEQ ID NO: 152) |
| HAL 7-12 | SYAMS (SEQ ID NO: 124) | AISGSGGSTYYADSVEG (SEQ ID NO: 136) | KERLEEPGFFDY (SEQ ID NO: 153) |
| HAL 8-7 | SYAMS (SEQ ID NO: 124) | AISGSGGSTYYADSVKG (SEQ ID NO: 137) | |

**Table 6**

| Light Chain CDR | | | |
|---|---|---|---|
| Antibody | CDR-L1 | CDR-L2 | CDR-L3 |
| EW01 | SGSSSNIGNNSVY(SEQ ID NO: 155) | SDSQRPS(SEQ ID NO: 172) | GTWDYSLNG(SEQ ID NO: 188) |
| EW02 | SGSSSNIGNNYVY(SEQ ID NO: 156) | ANNQRPS(SEQ ID NO: 173) | GAWDDSLSG(SEQ ID NO: 189) |
| EW03 | SGSSSNIGNNDVT(SEQ ID NO: 157) | SDSNRPS(SEQ ID NO: 174) | GTWDSSLSA(SEQ ID NO: 190) |
| EW06 | TGSSSNIGSNNVT (SEQ ID NO: 158 | SNSHRPS (SEQ ID NO: 175) | GTWDDSLNG (SEQ ID NO: 191) |
| EW09 | SGSSSNIGNNSVN (SEQ ID NO: 159) | ANNNRPS (SEQ ID NO: 176) | GAWDASLNG (SEQ ID NO: 192) |
| EW10 | TGSSSNIGSNYVS (SEQ ID NO: 160) | SDSNRPS (SEQ ID NO: 177) | ATWDASLSA (SEQ ID NO: 193) |
| EW16 | TGSSSNIGNNDVY (SEQ ID NO: 161) | SDSNRPS (SEQ ID NO: 178) | GTWDDSLNG (SEQ ID NO: 191) |
| EW26 | TGSSSNIGSNSVS (SEQ ID NO: 162) | DDSNRPS (SEQ ID NO: 178) | ASWDYSLNA (SEQ ID NO: 194) |
| EW28 | SGSSSNIGSNDVY (SEQ ID NO: 163) | SDNNRPS (SEQ ID NO: 179) | GAWDDSLSG (SEQ ID NO: 189) |
| EW34 | TGSSSNIGSNNVN (SEQ ID NO: 164) | ADSQRPS (SEQ ID NO: 180) | GSWDSSLSG (SEQ ID NO: 195) |
| EW37 | SGSSSNIGSNSVN (SEQ ID NO: 165) | SDSHRPS (SEQ ID NO: 181) | GSWDDSLSG (SEQ ID NO: 196) |
| HAL 7-1 | TGSSSNIGAAYEVH (SEQ ID NO: 166) | DTSNRPS (SEQ ID NO: 182) | AAWDDSLNG (SEQ ID NO: 197) |
| HAL 7-2 | SGDKLGDRYVF (SEQ ID NO: 167) | DDSDRPS (SEQ ID NO: 183) | QVWDSVNDH (SEQ ID NO: 198) |
| HAL 7-5 | SGSGSNIGSNAVN (SEQ ID NO: 168) | SNNQRPS (SEQ ID NO: 184) | AAWDDSLNG (SEQ ID NO: 197) |
| HAL 7-7 | GGNNIATKGVH (SEQ ID NO: 169) | DDSGRPS (SEQ ID NO: 185) | QLWDGRSDQ (SEQ ID NO: 199) |
| HAL 7-12 | TGTSSDVGGYNYVS (SEQ ID NO: 170) | EVSNRPS (SEQ ID NO: 186) | SSYTTDNA (SEQ ID NO: 200) |
| HAL 8-7 | KSSQSLLNSGNQKNDLA (SEQ ID NO: 171) | GASTRES (SEQ ID NO: 187) | QNDHSYP (SEQ ID NO: 201) |

**Table 7**

| Heavy Chain Variable Region | | |
|---|---|---|
| Antibody | Amino acid sequence | Coding DNA sequence |
| EW01 | | |
| | | |
| EW02 | | |
| EW03 | | |
| EW06 | | |
| EW09 | | |
| EW10 | | |
| | | |
| EW16 | | |
| EW26 | | |
| EW28 | | |
| EW34 | | |
| EW37 | | |
| | | |
| HAL7-1 | | |
| HAL 7-2 | | |
| HAL 7-5 | | |
| HAL 7-7 | | |
| | | |
| HAL 7-12 | | |
| HAL 8-7 | | |

**Table 8**

| Light Chain Variable Region | | |
|---|---|---|
| Antibody | Amino acid sequence | Coding DNA sequence |
| EW01 | | |
| EW02 | | |
| EW03 | | |
| | | |
| EW06 | | |
| EW09 | | |
| EW10 | | |
| EW16 | | |
| EW26 | | |
| EW28 | | |
| | | |
| EW34 | | |
| EW37 | | |
| HAL7-1 | | |
| HAL 7-2 | | |
| HAL 7-5 | | |
| | NO: 249) | |
| HAL 7-7 | | |
| HAL 7-12 | | |
| HAL 8-7 | | |

**Table 9**

| Heavy Chain Constant Region | | |
|---|---|---|
| Antibody | Amino acid sequence | Coding DNA sequence |
| EW01, EW02, EW03, EW06, EW09, EW10, EW16, EW26, EW28, EW34, EW37, HAL 7-1, HAL 7-2, HAL 7-5, HAL 7-7, HAL 7-12, HAL 8-7 | | |
| | | |

**Table 10**

| Light Chain Constant Region | | |
|---|---|---|
| Antibody | Amino acid sequence | Coding DNA sequence |
| EW01, EW02, EW03, EW06, EW09, EW10, EW16, EW26, EW28, EW34, EW37, HAL 7-1, HAL 7-2, HAL 7-5, HAL 7-7, HAL 7-12, | | |
| HAL 8-7 | | |

### Example 2: Binding Affinity of Anti-Idiotype Antibody Against Anti-c-Met Antibody

The antibodies prepared in Example 1 above were produced in 293 F cells (Invitrogen), purified and quantified to adjust their concentration, and then used for the following tests. The quantification of the purified antibodies was performed using a Nano-drop machine, and numerals reflecting A280/A260 absorption values were used. The quantification results were confirmed again through SDA-PAGE procedures. MaxiSorp™ flat-bottom plates (Nunc) were treated with the anti-c-Met antibody prepared in reference example 1 at a concentration of 1 µg/ml, and then reacted with a blocking solution (3% BSA, 0.05% Tween 20) at room temperature for one hour. After blocking, the plates were washed with a PBST (0.05% Tween20 in PBS) and then treated with the anti-idiotype antibodies prepared in example 1 at 10-fold serial dilution concentrations (10 µg/ml, 1 µg/ml, 0.1 µg/ml, and 0.01 µg/ml) starting from 10 µg/ml, respectively to react at room temperature for one hour. After the reaction was complete, the plates were washed again with a microplate washer (Biotek ELx405, PBST).

A biotinylated anti-c-Met antibody was prepared by binding the anti-c-Met antibody prepared in reference example 1 with biotin, the plates washed as described above were treated with the biotinylated anti-c-Met antibody at a concentration of 200 ng/ml to react at room temperature for one hour. The plates were washed again with a PBST (0.05% Tween20 in PBS) to eliminate unbound biotinylated anti-c-Met antibodies, treated with HRP (horse radish peroxidase) conjugated avidin (BioLegend) which specifically binds to biotin to react at room temperature for one hour, and then washed with a PBST (0.05% Tween20 in PBS). For color development, the plates were treated with TMB (3,3,5,5-tetramethylbenzidine) which is a HRP substrate, and absorption was measured at 450 nm.

In order to identify idiotope site-specific binding affinity, the same procedures as above were carried out using plates coated with human IgG (chromPure human IgG, Jackson ImmunoResearch) in an amount of 1 µg/ml instead of the anti-c-Met antibody.

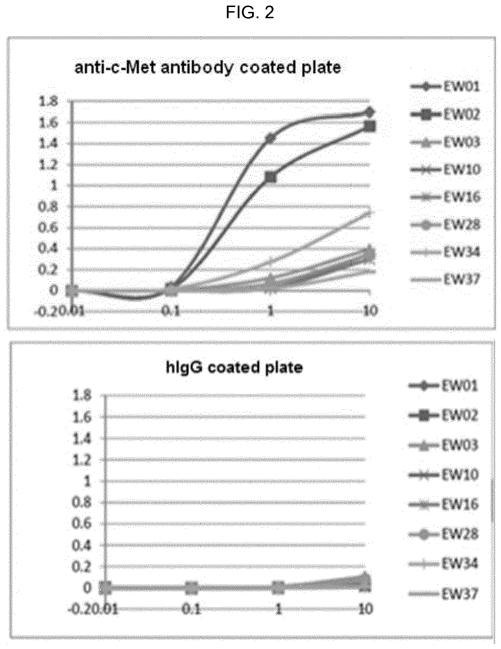

The results obtained are shown in FIG. 2. The Y-axis in FIG. 2 indicates a binding affinity between the anti-c-Met antibody and the anti-idiotype antibodies prepared in example 1 at 450 nm (upper), and a binding affinity between hIgG and the anti-idiotype antibodies prepared in example 1 (bottom). As seen in FIG. 2, the anti-idiotype antibodies prepared in example 1 showed higher binding affinity to the anti-c-Met antibody than hIgG. This demonstrates that the anti-idiotype antibodies prepared in example 1 bind specifically to the idiotope site of the anti-c-Met antibody.

In order to view the antigen-antibody binding affinity of an EW02 antibody, which has relatively high affinity to the anti-c-Met antibody on ELISA among the anti-idiotype antibodies, Kd value was investigated using Biacore equipment (GE healthcare) and shown in Table 11, as follows.

**Table 11**

| Antibody | K_{D} (nM) | kₐ (1/Ms) | k_{d} (1/s) |
|---|---|---|---|
| EW02 | 38.4 | 8.5×10⁴ | 3.2×10⁻³ |

The results indicate the affinity of the EW02 antibody against the anti-c-Met antibody was measured to be 38.4 nM.

### Example 3: Effects on Binding Affinity of Anti-c-Met Antibody and c-Met by Anti-Idiotype Antibody

The anti-c-Met antibody prepared in Reference Example 1 was bound to biotin to prepare a biotinylated anti-c-Met antibody. 200 ng/ml of the biotinylated anti-c-Met antibody was reacted with its antigen, c-Met protein (358-MT/CF, RND systems) at concentrations (2,000 ng/ml, 200 ng/ml, 20 ng/ml and 0 ng/ml) at room temperature for one hour and then it was treated onto plates coated with the anti-idiotype antibodies prepared in the above example 1 in an amount of 1 µg/ml each to react at room temperature for one hour. Thereafter, the plates were washed with PBST (0.05% Tween20 in PBS) to eliminate unspecific binders and then treated with Streptavidin-HRP (BioLegend) to further react at room temperature for one hour. After the reaction was complete, the plates were further washed with a washing solution (PBST (0.05% Tween20 in PBS)), treated with a TMB substrate for color development and then, absorption was measured at 450 nm. For comparison, the same procedures as above were carried out using plates coated with 1 µg/ml of human IgG1 or 1 µg/ml of c-Met protein instead of the anti-idiotype antibodies prepared in example 1.

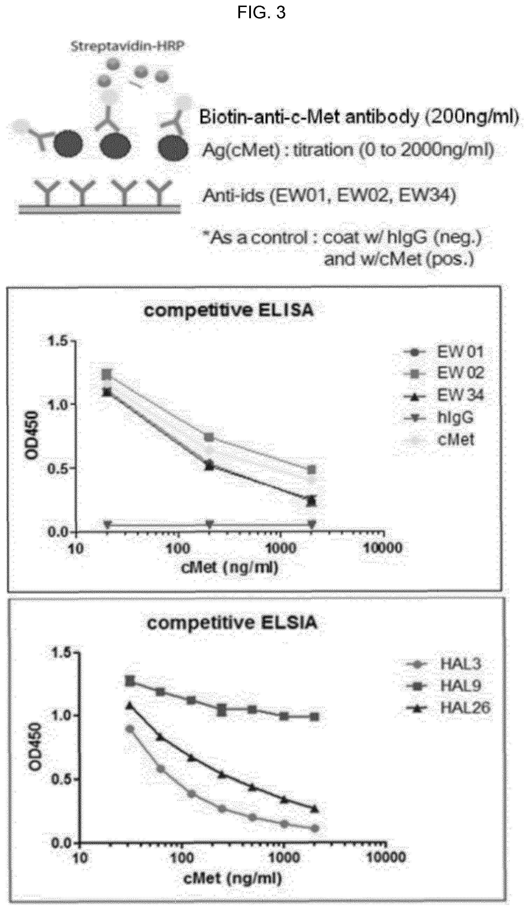

The results are shown in FIG. 3. FIG. 3 indicates that as the concentration of c-Met used in pre incubation increased, the binding between the anti-idiotype antibodies and the anti-c-Met antibody decreased in a c-Met concentration-dependent manner. Such a concentration-dependent reduction is similar to the scenario where c-Met was used instead of the anti-idiotype antibodies. Such concentration dependent reduction did not occur when human IgG was used. The results demonstrate that the anti-idiotype antibodies compete with c-Met to bind to the anti-c-Met antibody.

### Example 4: Detection of Anti-c-Met Antibody Using Anti-Idiotype Antibody in Monkey Serum

The EW01 antibody, which showed the most excellent binding affinity of the anti-idiotype antibodies against the anti-c-Met antibody prepared in the above example 1 (see FIG. 2), was selected for additional testing. The binding affinity of the EW01 antibody to the anti-c-Met antibody in a monkey serum was tested. This test illustrates the application of the anti-idiotype antibodies to the detection of an anti-c-Met antibody within a serum.

MaxiSorp™ flat-bottom plates (Nunc) were treated with the EW01 antibody at a concentration of 0.25 µg/ml for coating and then, they were reacted with a blocking solution (3%(w/v) BSA in PBST) at room temperature for one hour. A 10% (v/v) serum solution (in PBS) was prepared using the serum of cynomolgus monkey and the anti-c-Met antibody prepared in reference example 1 was added thereto from 1 µg/ml to 3-fold serial dilution concentrations (from 1 µg/ml to 8-point serial dilution), respectively to prepare anti-c-Met antibody samples. The above prepared plates were treated with the thus prepared anti-c-Met antibody samples to further react at room temperature for one hour. After the reaction was complete, the plates were washed again with a washing solution (PBST (0.05% Tween20 in PBS)), and 0.25 µg/ml of the EW01 antibody which was biotinylated for detection purpose was added thereto to react at room temperature for one hour. After the reaction of the detection antibody was over, the same washing procedures as above were carried out, and a HRP-streptavidin solution (BioLegend) was added thereto to react at room temperature for one hour. After all the reactions were over, the plates were finally washed and treated with a TMB substrate for color development, and absorption was measured at 450 nm and analyzed.

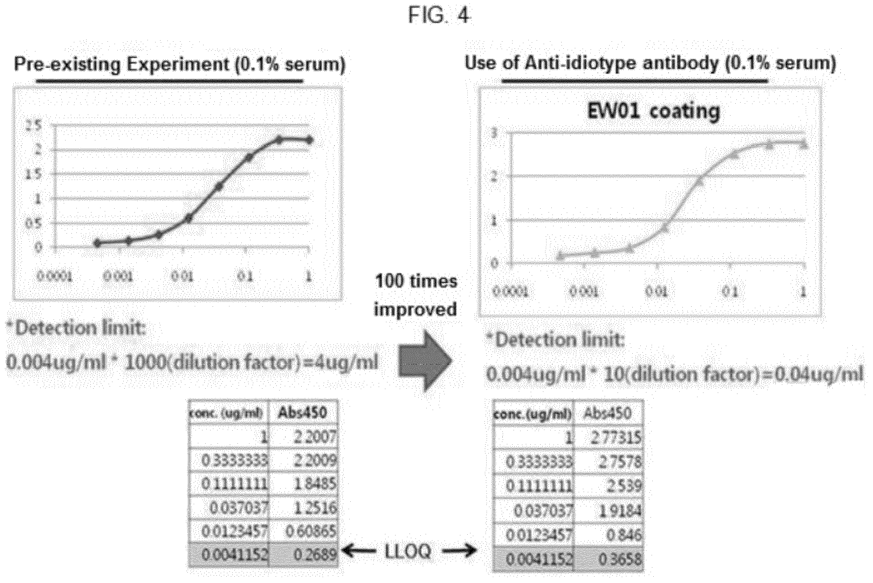

The results are shown in FIG. 4. The X-axis in FIG. 4 indicates the concentrations of the anti-c-Met antibody, and Y- axis indicates absorption values at 450 nm. The left graph shows results measured by reacting plates treated with antigen c-Met protein with an anti-c-Met antibody-containing monkey serum diluted to 0.1% (v/v) in order to measure the anti-c-Met antibody in a monkey serum according to the previous experiment method prior to using anti-idiotype antibodies. On the right, plates treated with the anti-idiotype antibodies were used in order to detect the anti-c-Met antibody in a monkey serum according to the test method improved after the anti-idiotype antibodies were developed. Even when the monkey serum was diluted to 10% (v/v) or so, results similar to those of the previous method were still obtained, which suggests that the detection limit is increased by 100 times.

### Example 5: Effects on Intracellular Efficacy of Anti-c-Met Antibody by Anti-Idiotype Antibody Against c-Met Antibody

An EBC1 cell line (ATCC) was prepared in an amount of 1X10⁴cell/well, which was then treated with 1 µg/ml of the anti-c-Met antibody prepared in reference example 1 and treated with the anti-idiotype antibody prepared in example 1 so that the ratios of the anti-c-Met antibody and the anti-idiotype antibody in weight became 1:2, 1:1, 2:1, and 4:1 (weight of anti-idiotype antibody: weight of anti-c-Met antibody) to culture at 37°C for 72 hours. For comparison, the anti-c-Met antibody was treated alone or only 1 µg/ml of hIgG was treated without the anti-c-Met antibody.

After the culture, cell proliferation was analyzed using CellTiter-Glo (promega) according to the manufacturer's instructions. Particularly, the cultured cells were treated with a Cell Titer-Glo solution in an amount of 100 µl/well and reacted at room temperature for 30 min in the state of light being shielded. The plates where color development by CellTiter-Glo was complete were analyzed for apoptosis by measuring luminescence.

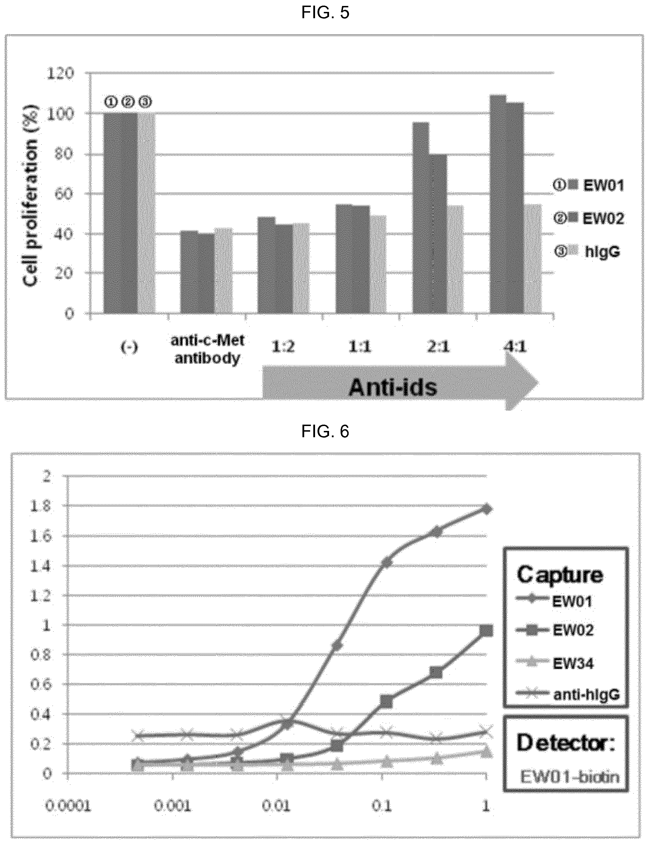

The results are shown in FIG. 5. The X-axis in FIG. 5 indicates the weight ratios of the anti-idiotype antibody and the anti-c-Met antibody (weight of anti-idiotype antibody: weight of anti-c-Met antibody). When compared to the very left no-treatment group, cell proliferation when treated with the anti-c-Met antibody was reduced by 60% or so, and the cell proliferation inhibitory efficacy of the anti-c-Met antibody disappeared by further reaction with the anti-idiotype antibody. As the ratios of the anti-idiotype antibody increased from left to right, the efficacy of the anti-c-Met antibody disappeared in proportion to those ratios. When the hIgG antibody was used instead of the anti-idiotype antibody for comparison, there was no reduction in efficacy.

### Example 6: Detection of Anti-c-Met Antibody Using Anti-Idiotype Antibody in Human Serum

In order to see whether the method for detecting an anti-c-Met antibody in a monkey serum is applicable to a human serum, the human serum was diluted to 10% (v/v) and the anti-c-Met antibody prepared in reference example 1 was prepared from a concentration of 1 µg/ml to 3-fold serial dilutions. The above prepared human serums containing the c-Met antibody were reacted to plates treated with 0.25 µg/ml of each anti-idiotype antibody (capture) prepared in example 1 at room temperature for one hour. The plates were washed with a washing solution (0.05% Tween20 in PBS) in order to eliminate unreacted antibodies and then treated with a biotinylated form of EW01 (detector) which showed the best binding affinity out of the anti-idiotype antibodies at a concentration of 0.25 µg/ml to react at room temperature for one hour. The plates where the reaction was over were washed with a washing solution in the same manner and then reacted with Streptavidin-HRP(BioLegend) which shows biotin-specific binding at room temperature for one hour in the state of light being shielded. Finally, the plates were washed with a washing solution (PBST (0.05% Tween20 in PBS)) to eliminate unreacted HRP and then, they were subject to color development using a TMB substrate to measure absorption at 450 nm. The same procedures as above were carried out using plates treated with 0.25 µg/ml of hIgG instead of the anti-idiotype antibody for comparison.

The results are shown in FIG. 6. The X-axis in FIG. 6 indicates the concentrations of the anti-c-Met antibody and Y- axis indicates absorption values at 450 nm. From the results of FIG. 6, it was confirmed that the anti-idiotype antibodies of the present invention are applicable to not only a monkey serum but also a human serum.

### Example 7: Quantification of anti-drug antibody (ADA) in anti-c-Met antibody treated monkey serum

The presence or absence and the amount of ADA against anti-c-Met antibody in anti-c-Met antibody treated monkey serum (Equitech) were determined by using an anti-idiotype antibody. Assuming that anti-idiotype antibodies from example 1 are examples of ADAs against anti-c-Met antibody, 100µg/mL of each anti-idiotype antibody was pooled in normal monkey serum (Equitech) replacing ADA against anti-c-Met antibody.

MaxiSorpTM flat-bottom plate (Nunc) was coated with 1µ/ml of the anti-c-Met antibody prepared in Reference Example 1 and blocked with blocking solution (1%BSA in PBST) at room temperature for 1 hour. As a standard sample, anti-idiotype antibody EW01 prepared in Example 1 was provided by 3-fold serial dilution starting from 10µg/ml. As a test sample, anti-idiotype antibodies treated monkey serum was prepared by treating 4 types of anti-idiotype antibodies of Example 1 at the total concentration of 400µg/mL, wherein each of the anti-idiotype antibodies was treated at the concentration of 100µg/mL. The monkey serum sample was diluted 1/10, 1/50, or 1/100 times by volume using PBST.

The standard sample (anti-idiotype antibody EW01) and the test sample (anti idiotype antibody treated monkey serum) were respectively added to the blocked plate and reacted for 1 hour. The non-reacted materials were removed with washing solution (PBST(0.05% Tween20 in PBS)). To make a detection, 0.5µg/ml of biotinylated anti-c-Met antibody was added to the plate, reacted for 1 hours, and then washed with washing solution(PBST(0.05% Tween20 in PBS)) to remove the non-reacted materials.

The washed plate was reacted with Streptavidin-HRP (BioLegend), which specifically binds to biotin, at room temperature for 1 hour with blocking light. Finally, the plate was washed with washing solution (PBST(0.05% Tween20 in PBS)) to remove the non-reacted materials. TMB substrate was used for a coloring reaction and the absorbance was measured at 450nm using SpectraMax.

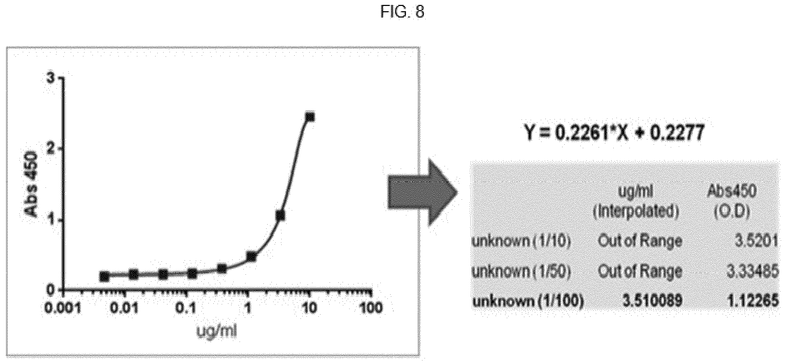

The absorbance measured from the standard sample according to the concentrations of the anti-idiotype antibody EW01 was shown in FIG. 8. The absorbance measured form the test sample (monkey serum of 1/100 dilution) was 1.12 (the absorbance of the test samples of 1/10 and 1/50 dilutions was out of the standard sample's range in this experimentation). The concentration of ADA in the test sample can be calculated from absorbance value 1.12 by applying the formula shown in FIG. 8 The formula was obtained from softmax program (5-PL) in SpectraMax machine. The calculated value was 3.5µg/ml, indicating that considering the dilution fold, the concentration of the ADA present in the test sample is 350µg/ml. This value was similar with initial amount of the anti-idiotype antibodies which were initially added into the test sample.

As described above, the amount as well as the presence/absence of ADA in a test sample can be determined by obtaining a standard curve of absorbance from a standard sample, establishing a formula, and then applying the absorbance value measured from the test sample to the formula.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention.

## Claims

1. An anti-idiotype antibody or antigen-binding fragment thereof, wherein the anti-idiotype antibody or antigen-binding fragment thereof specifically binds to an anti-c-Met antibody or an antibody fragment of the anti-c-Met antibody with an affinity of 50 nM or less, and wherein the anti-c-Met antibody or the antibody fragment of the anti-c-Met antibody comprises:
a CDR-H1 comprising SEQ ID NO: 1;
a CDR-H2 comprising SEQ ID NO: 2;
a CDR-H3 comprising SEQ ID NO: 3;
a CDR-L1 comprising SEQ ID NO: 10;
a CDR-L2 comprising SEQ ID NO: 11; and
a CDR-L3 comprising SEQ ID NO: 13; and
wherein the anti-idiotype antibody or antigen-binding fragment thereof comprises:
(i) a CDR-H1 of SEQ ID NO: 115, a CDR-H2 of SEQ ID NO: 125, a CDR-H3 of SEQ ID NO: 139, a CDR-L1 of SEQ ID NO: 155, a CDR-L2 of SEQ ID NO: 172, and a CDR-L3 of SEQ ID NO: 188; or
(ii) a CDR-H1 of SEQ ID NO: 116, a CDR-H2 of SEQ ID NO: 126, a CDR-H3 of SEQ ID NO: 140, a CDR-L1 of SEQ ID NO: 156, a CDR-L2 of SEQ ID NO: 173, and a CDR-L3 of SEQ ID NO: 189.

2. The anti-idiotype antibody or antigen-binding fragment thereof according to claim 1, wherein the anti-c-Met antibody or the antibody fragment of the anti-c-Met antibody comprises a heavy chain comprising the amino acid sequence from the 18^{th} to 460^{th} positions of SEQ ID NO: 66 and a light chain comprising the amino acid sequence from the 21^{st} to 240^{th} positions of SEQ ID NO: 68.

3. The anti-idiotype antibody or antigen-binding fragment thereof according to claim 1 or 2, wherein the anti-idiotype antibody or antigen-binding fragment thereof comprises:
a heavy chain variable region comprising SEQ ID NO: 202 and a light chain variable region comprising SEQ ID NO: 236; or
a heavy chain variable region comprising SEQ ID NO: 203 and a light chain variable region comprising SEQ ID NO: 237.

4. The anti-idiotype antibody or antigen-binding fragment thereof according to claim 1 or 2, wherein the anti-idiotype antibody is a mouse-human chimeric antibody, a humanized antibody, or a human antibody.

5. The anti-idiotype antibody or antibody fragment thereof according to any one of claims 1 to 4, wherein the anti-idiotype antibody or antibody fragment is an antigen-binding fragment selected from the group consisting of scFv, (scFv)₂, Fab, Fab', and F(ab')₂.

6. The anti-idiotype antibody or antibody fragment thereof according to any one of claims 1 to 5 for use in detecting the anti-c-Met antibody or for use in analyzing an anti-drug antibody against the anti-c-Met antibody.

7. A method for detecting an anti-c-Met antibody, wherein the anti-c-Met antibody comprises:
a CDR-H1 comprising SEQ ID NO: 1;
a CDR-H2 comprising SEQ ID NO: 2;
a CDR-H3 comprising SEQ ID NO: 3;
a CDR-L1 comprising SEQ ID NO: 10;
a CDR-L2 comprising SEQ ID NO: 11; and
a CDR-L3 comprising SEQ ID NO: 13,
wherein the method comprises the steps of:
contacting a biological sample with the anti-idiotype antibody or antigen-binding fragment thereof according to any one of claims 1 to 5; and
determining the presence or absence of an antigen-antibody reaction.

8. A polynucleotide comprising the following nucleotide sequences:
(i) SEQ ID NO: 219 and SEQ ID NO: 253; or
(ii) SEQ ID NO: 220 and SEQ ID NO: 254.

## Patentansprüche

1. Anti-Idiotyp-Antikörper oder Antigen-bindendes Fragment davon, wobei der Anti-Idiotyp-Antikörper oder das Antigen-bindende Fragment davon spezifisch an einen Anti-c-Met-Antikörper oder ein Antikörperfragment des Anti-c-Met-Antikörpers mit einer Affinität von 50 nM oder weniger bindet, und wobei der Anti-c-Met-Antikörper oder das Antikörperfragment des Anti-c-Met-Antikörpers:
eine CDR-H1 umfassend SEQ ID NR: 1;
eine CDR-H2 umfassend SEQ ID NR: 2;
eine CDR-H3 umfassend SEQ ID NR: 3;
eine CDR-L1 umfassend SEQ ID NR: 10;
eine CDR-L2 umfassend SEQ ID NR: 11; und
eine CDR-L3 umfassend SEQ ID NR: 13
umfasst; und
wobei der Anti-Idiotyp-Antikörper oder das Antigen-bindende Fragment davon:
(i) eine CDR-H1 von SEQ ID NR: 115, eine CDR-H2 von SEQ ID NR: 125, eine CDR-H3 von SEQ ID NR: 139, eine CDR-L1 von SEQ ID NR: 155, eine CDR-L2 von SEQ ID NR: 172, und eine CDR-L3 von SEQ ID NR: 188 umfasst; oder
(ii) eine CDR-H1 von SEQ ID NR: 116, eine CDR-H2 von SEQ ID NR: 126, eine CDR-H3 von SEQ ID NR: 140, eine CDR-L1 von SEQ ID NR: 156, eine CDR-L2 von SEQ ID NR: 173, und eine CDR-L3 von SEQ ID NR: 189 umfasst.

2. Anti-Idiotyp-Antikörper oder Antigen-bindendes Fragment davon nach Anspruch 1, wobei der Anti-c-Met-Antikörper oder das Antikörperfragment des Anti-c-Met-Antikörpers eine schwere Kette umfassend die Aminosäuresequenz von der 18ten bis zur 460ten Position von SEQ ID NR: 66 und eine leichte Kette umfassend die Aminosäuresequenz von der 21ten bis zur 240ten Position von SEQ ID NR: 68 umfasst.

3. Anti-Idiotyp-Antikörper oder Antigen-bindendes Fragment davon nach Anspruch 1 oder 2, wobei der Anti-Idiotyp-Antikörper oder das Antigen-bindende Fragment davon:
eine variable Region der schweren Kette umfassend SEQ ID NR: 202 und eine variable Region der leichten Kette umfassend SEQ ID NR: 236 umfasst; oder
eine variable Region der schweren Kette umfassend SEQ ID NR: 203 und eine variable Region der leichten Kette umfassend SEQ ID NR: 237 umfasst.

4. Anti-Idiotyp-Antikörper oder Antigen-bindendes Fragment davon nach Anspruch 1 oder 2, wobei der Anti-Idiotyp-Antikörper ein chimärer Maus-Mensch-Antikörper, ein humanisierter Antikörper oder ein menschlicher Antikörper ist.

5. Anti-Idiotyp-Antikörper oder Antigen-bindendes Fragment davon nach einem der Ansprüche 1 bis 4, wobei der Anti-Idiotyp-Antikörper oder das Antigen-bindende Fragment ein Antigen-bindendes Fragment ausgewählt aus der Gruppe bestehend aus scFv, (scFv)₂, Fab, Fab' und F(ab')₂ ist.

6. Anti-Idiotyp-Antikörper oder Antigen-bindendes Fragment davon nach einem der Ansprüche 1 bis 5 zur Verwendung in der Detektion des Anti-c-Met-Antikörpers oder zur Verwendung in der Analyse eines Anti-Drug-Antikörpers gegen den Anti-c-Met-Antikörper.

7. Verfahren zur Detektion eines Anti-c-Met-Antikörpers, wobei der Anti-c-Met-Antikörper:
eine CDR-H1 umfassend SEQ ID NR: 1;
eine CDR-H2 umfassend SEQ ID NR: 2;
eine CDR-H3 umfassend SEQ ID NR: 3;
eine CDR-L1 umfassend SEQ ID NR: 10;
eine CDR-L2 umfassend SEQ ID NR: 11; and
eine CDR-L3 umfassend SEQ ID NR: 13
umfasst,
wobei das Verfahren die Schritte:
Kontaktieren einer biologischen Probe mit dem Anti-Idiotyp-Antikörper oder Antigenbindendem Fragment davon nach einem der Ansprüche 1 bis 5; und
Bestimmung der Anwesenheit oder Abwesenheit einer Antigen-Antikörper-Reaktion umfasst.

8. Polynukleotid umfassend die folgenden Nukleotidsequenzen:
(i) SEQ ID NR: 219 und SEQ ID NR: 253; oder
(ii) SEQ ID NR: 220 und SEQ ID NR: 254.

## Revendications

1. Anticorps anti-idiotypique ou fragment de celui-ci de liaison à un antigène, où l'anticorps anti-idiotypique ou le fragment de celui-ci de liaison à un antigène se lient spécifiquement à un anticorps anti-cMet ou à un fragment d'anticorps de l'anticorps anti-cMet avec une affinité de 50 nM ou moins, et où l'anticorps anti-cMet ou le fragment d'anticorps de l'anticorps anti-cMet comprennent :
une CDR-H1 comprenant la SEQ ID NO : 1 ;
une CDR-H2 comprenant la SEQ ID NO : 2 ;
une CDR-H3 comprenant la SEQ ID NO : 3 ;
une CDR-L1 comprenant la SEQ ID NO : 10 ;
une CDR-L2 comprenant la SEQ ID NO : 11 ; et
une CDR-L3 comprenant la SEQ ID NO : 13 ; et
l'anticorps anti-idiotypique ou le fragment de celui-ci de liaison à un antigène comprenant :
(i) une CDR-H1 selon la SEQ ID NO : 115, une CDR-H2 selon la SEQ ID NO : 125, une CDR-H3 selon la SEQ ID NO : 139, une CDR-L1 selon la SEQ ID NO : 155, une CDR-L2 selon la SEQ ID NO : 172 et une CDR-L3 selon la SEQ ID NO : 188 ; ou
(ii) une CDR-H1 selon la SEQ ID NO : 116, une CDR-H2 selon la SEQ ID NO : 126, une CDR-H3 selon la SEQ ID NO : 140, une CDR-L1 selon la SEQ ID NO : 156, une CDR-L2 selon la SEQ ID NO : 173 et une CDR-L3 selon la SEQ ID NO : 189.

2. Anticorps anti-idiotypique ou fragment de celui-ci de liaison à un antigène selon la revendication 1, où l'anticorps anti-cMet ou le fragment d'anticorps de l'anticorps anti-cMet comprennent une chaîne lourde comprenant la séquence d'acides aminés de la 18^{e} à la 460^{e} position de la SEQ ID NO : 66 et une chaîne légère comprenant la séquence d'acides aminés de la 21^{e} à la 240^{e} position de la SEQ ID NO : 68.

3. Anticorps anti-idiotypique ou fragment de celui-ci de liaison à un antigène selon la revendication 1 ou 2, l'anticorps anti-idiotypique ou le fragment de celui-ci de liaison à un antigène comprenant :
une région variable de chaîne lourde comprenant la SEQ ID NO : 202 et une région variable de chaîne légère comprenant la SEQ ID NO : 236 ; ou
une région variable de chaîne lourde comprenant la SEQ ID NO : 203 et une région variable de chaîne légère comprenant la SEQ ID NO : 237.

4. Anticorps anti-idiotypique ou fragment de celui-ci de liaison à un antigène selon la revendication 1 ou 2, l'anticorps anti-idiotypique étant un anticorps chimérique humain-souris, un anticorps humanisé, ou un anticorps humain.

5. Anticorps anti-idiotypique ou fragment de celui-ci de liaison à un antigène selon l'une quelconque des revendications 1 à 4, l'anticorps anti-idiotypique ou le fragment de liaison à un antigène étant un fragment d'anticorps choisi dans le groupe constitué par scFv, (scFv)₂, Fab, Fab' et F(ab')₂.

6. Anticorps anti-idiotypique ou fragment de celui-ci de liaison à un antigène selon l'une quelconque des revendications 1 à 5 destinés à être utilisés pour détecter l'anticorps anti-cMet ou destinés à être utilisés pour analyser un anticorps anti-médicament dirigé contre l'anticorps anti-cMet.

7. Procédé de détection d'un anticorps anti-cMet, l'anticorps anti-cMet comprenant :
une CDR-H1 comprenant la SEQ ID NO : 1 ;
une CDR-H2 comprenant la SEQ ID NO : 2 ;
une CDR-H3 comprenant la SEQ ID NO : 3 ;
une CDR-L1 comprenant la SEQ ID NO : 10 ;
une CDR-L2 comprenant la SEQ ID NO : 11 ; et
une CDR-L3 comprenant la SEQ ID NO : 13,
le procédé comprenant les étapes consistant :
à mettre en contact un échantillon biologique avec l'anticorps anti-idiotypique ou le fragment de celui-ci de liaison à un antigène selon l'une quelconque des revendications 1 à 5 ; et
à déterminer la présence ou l'absence d'une réaction antigène-anticorps.

8. Polynucléotide comprenant les séquences de polynucléotides suivantes :
(i) SEQ ID NO : 219 et SEQ ID NO : 253 ; ou
(ii) SEQ ID NO : 220 et SEQ ID NO : 254.
